(19) **Europäisches Patentamt / European Patent Office / Office européen des brevets**

(11) **EP 4 121 007 B1**

(12) **EUROPEAN PATENT SPECIFICATION**

(45) Date of publication and mention of the grant of the patent:
**02.07.2025 Bulletin 2025/27**

(21) Application number: **21722538.2**

(22) Date of filing: **19.03.2021**

(51) International Patent Classification (IPC):
**A61K 9/00** *(2006.01)*    **A61K 39/00** *(2006.01)*
**A61K 47/12** *(2006.01)*    **A61K 47/18** *(2017.01)*
**A61K 47/26** *(2006.01)*

(52) Cooperative Patent Classification (CPC):
**A61K 9/0019; A61K 39/39591; A61K 47/183;
C07K 16/18;** A61K 47/12; A61K 47/26

(86) International application number:
**PCT/IB2021/000155**

(87) International publication number:
**WO 2021/186245 (23.09.2021 Gazette 2021/38)**

(54) **HIGH CONCENTRATION ANTI-AMYLOID BETA PEPTIDE PROTOFIBRIL ANTIBODY FORMULATIONS AND METHODS OF USE THEREOF**

HOCHKONZENTRIERTE ANTI-AMYLOID-BETA-PROTOFIBRILLEN ANTIKÖRPERFORMULIERUNGEN UND VERFAHREN ZU IHRER VERWENDUNG

FORMULATIONS À CONCENTRATION ÉLEVÉE D'ANTICORPS DIRIGÉS CONTRE LES PROTOFIBRILLES DE PEPTIDE BÊTA-AMYLOÏDE ET LEURS MÉTHODES D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.03.2020 US 202062992746 P
19.05.2020 US 202063027263 P**

(43) Date of publication of application:
**25.01.2023 Bulletin 2023/04**

(60) Divisional application:
**23179487.6 / 4 252 777**

(73) Proprietor: **Eisai R&D Management Co., Ltd.
Tokyo 112-8088 (JP)**

(72) Inventors:
• **YOSHIDA, Nobuo
Tokyo 112--8088 (JP)**
• **SAKURAMOTO, Naomi
Kakamigahara-shi, Gifu 501--6195 (JP)**

• **SAKAGUCHI, Takahisa
Kakamigahara-shi, Gifu 501--6195 (JP)**
• **KITO, Hirokazu
Kakamigahara-shi, Gifu 501--6195 (JP)**
• **OZAWA, Takahiro
Kakamigahara-shi, Gifu 501--6195 (JP)**
• **JOSHI, Anjali
Cary, North Carolina 27519 (US)**
• **HSU, Yung Yueh
Carlisle, Massachusetts 01741 (US)**
• **SOUILLAC, Pierre
Winchester, Massachusetts 01890 (US)**

(74) Representative: **Hoffmann Eitle
Patent- und Rechtsanwälte PartmbB
Arabellastraße 30
81925 München (DE)**

(56) References cited:
**WO-A2-2020/023530**

EP 4 121 007 B1

- **ANN L DAUGHERTY AND RANDALL J MRSNY ED - STEVEN J SHIRE ET AL: "Formulation and Delivery Issues for Monoclonal Antibody Therapeutics", 1 January 2010, CURRENT TRENDS IN MONOCLONAL ANTIBODY DEVELOPMENT AND MANUFACTURING, SPRINGER, US, PAGE(S) 103 - 129, ISBN: 978-0-387-76642-3, XP009133774**
- **WANG W ET AL: "ANTIBODY STRUCTURE, INSTABILITY, AND FORMULATION", JOURNAL OF PHARMACEUTICAL SCIENCES, AMERICAN CHEMICAL SOCIETY AND AMERICAN PHARMACEUTICAL ASSOCIATION, US, vol. 96, no. 1, 1 January 2007 (2007-01-01), pages 1 - 26, XP009084505, ISSN: 0022-3549, DOI: 10.1002/ JPS.20727**

Remarks:
The complete document including Reference Table(s) and the Sequence Listing(s) can be downloaded from the EPO website

**Description**

The present application claims the benefit of priority to U.S. Provisional Application No. 62/992,746 filed March 20, 2020 and U.S. Provisional Application No. 63/027,263 filed May 19, 2020

Alzheimer's disease (AD) is a progressive, neurodegenerative disorder of unknown etiology and the most common form of dementia among older people. In 2006, there were 26.6 million cases of AD in the world (range: 11.4-59.4 million) (Brookmeyer, R., et al., Forecasting the global burden of Alzheimer's Disease. Alzheimer Dement. 2007; 3:186-91), while there were more than 5 million people in the United States reportedly living with AD (2010 Alzheimer's disease facts and figures. Alzheimer Dement. 2010;6:158-94). By the year 2050, the worldwide prevalence of AD is predicted to grow to 106.8 million (range: 47.2 - 221.2 million), while in the United States alone the prevalence is estimated to be 11 to 16 million. (Brookmeyer, *supra,* and 2010 Alzheimer's disease facts and figures, *supra).*

The disease generally involves a global decline of cognitive function that progresses slowly and leaves end-stage subjects bedridden. AD subjects typically survive for only 3 to 10 years after symptom onset, although extremes of 2 and 20 years are known. (Hebert, L.E., etal., Alzheimer disease in the U.S. population: prevalence estimates using the 2000 census. Arch Neurol. 2003; 60:1119-1122.) AD is the seventh leading cause of all deaths in the United States and the fifth leading cause of death in Americans older than the age of 65 years, despite the fact that mortality due to AD is greatly underestimated because death certificates rarely attribute the cause of death to AD. (2010 Alzheimer's disease facts and figures, *supra.*)

Histologically, the disease is characterized by neuritic plaques, found primarily in the association cortex, limbic system and basal ganglia. The major constituent of these plaques is amyloid beta peptide (Aβ). Aβ exists in various conformational states - monomers, oligomers, protofibrils, and insoluble fibrils. Details of the mechanistic relationship between onset of Alzheimer's disease and Aβ production is unknown. However, some anti-Aβ antibodies are undergoing clinical study now as potential therapeutic agents for Alzheimer's disease.

Anti-Aβ antibodies and other proteins may be administered to subjects via intravenous, subcutaneous, intramuscular, and other means. The dosage and/or dosage form of an antibody can present many challenges to developing a suitable pharmaceutical formation. For instance, at high antibody concentrations, the stability of the antibody can be problematic due to the formation of protein-protein aggregates or fragmentation. Generally, aggregation increases with increasing antibody concentrations. Moreover, high concentration antibody formulations require high concentrations of stabilizers and other excipients in order to achieve long-term protein stability and shelf-life. High concentration antibody formulations are also often viscous, potentially complicating the manufacture and administration of the pharmaceutical formulation.

Provided herein are pharmaceutical formulations comprising 80-300 mg/ml of an isolated anti-Aβ protofibril antibody or fragment thereof that binds to Aβ protofibril, wherein the antibody is BAN2401 (also known as lecanemab). The pharmaceutical formulations provided herein have been found to be advantageous. For example, despite high concentrations of the anti-Aβ protofibril antibody (e.g., 100 mg/mL or 200 mg/mL), the protein-protein aggregation rate is unexpectedly low and comparable to aggregation rates typically seen with much lower antibody concentrations (e.g., 10 mg/mL). In some embodiments, the presently disclosed pharmaceutical formulations exhibit a lower initial rate of aggregates than formulations having significantly lower concentrations of anti-Aβ protofibril antibody (e.g., ~0.3% initial level of aggregates for 100 mg/mL anti-Aβ protofibril antibody vs. ~0.8% initial level of aggregates for 10 mg/mL anti-Aβ protofibril antibody). In some embodiments, the presently disclosed pharmaceutical formulations exhibit a lower generation rate of sub-visible particles than formulations having significantly lower concentrations of the anti-Aβ protofibril antibody (e.g., 10.6 particles/mL for 100 mg/mL anti-Aβ protofibril antibody vs. 12.6 particles/mL for 10 mg/mL anti-Aβ protofibril antibody). In some embodiments, the presently disclosed pharmaceutical formulations exhibit a decreased aggregation rate, decreased initial aggregation level, decreased protein fragmentation rate, and/or decrease in sub-visible particle formation as compared to formulations having significantly lower concentrations of the anti-Aβ protofibril antibody. Low aggregation rate, initial aggregation level, protein fragmentation rate, and/or sub-visible particle generation may allow for increased stability and/or longer product shelf life. Moreover, the excipients in the presently disclosed pharmaceutical formulations can be present in lower quantities than in currently marketed intravenous products. In some embodiments, the pH and osmolality of the presently disclosed pharmaceutical formulations are acceptable for intravenous administration after dilution in intravenous fluids.

The assignment of amino acids to each domain is, generally, in accordance with the definitions of SEQUENCES OF PROTEINS OF IMMUNOLOGICAL INTEREST (Kabat et al., 5th ed., U.S. Department of Health and Human Services, NIH Publication No. 91- 3242. 1991, hereafter referred to as "Kabat report").

The anti-Aβ protofibril antibody according to the invention is BAN2401, also known as lecanemab. BAN2401 is a humanized IgG1 monoclonal version of mAb158, which is a murine monoclonal antibody raised to target protofibrils and disclosed in WO 2007/108756 and Journal of Alzheimer's Disease 43: 575-588 (2015). BAN2401 is an anti-Aβ protofibril antibody, demonstrating low affinity for Aβ monomer while binding with high selectivity to soluble Aβ aggregate species. For example, BAN2401 has been reported demonstrates an approximately 1000-fold and 5-fold to 10-fold higher selectivity for soluble Aβ protofibrils than for Aβ monomers or Aβ-insoluble fibrils, respectively.

BAN2401 comprises (i) a heavy chain variable domain comprising the amino acid sequence of SEQ ID NO:1 and (ii) a light chain variable domain comprising the amino acid sequence of SEQ ID NO:2. The full length sequences of heavy chain and light chain of BAN2401 are set forth in SEQ ID NOs: 11 and 12 and is described in WO 2007/108756 and in Journal of Alzheimer's Disease 43:575-588 (2015).

Other non-limiting examples of antibodies for use as the at least one anti-Aβ protofibril antibody in the present disclosure include those disclosed in WO 2002/003911, WO 2005/123775, WO 2007/108756, WO 2011/001366, WO 2011/104696, and WO 2016/005466.

WO 2020/023530 refers to methods of treatement and prevention of Alzheimer's disease.

In some embodiments, the isolated anti-Aβ protofibril antibody is present in a concentration ranging from 85 mg/mL to 275 mg/mL. In some embodiments, the isolated anti-Aβ protofibril antibody is present in a concentration ranging from 90 mg/mL to 250 mg/mL. In some embodiments, the isolated anti-Aβ protofibril antibody is present in a concentration ranging from 95 mg/ml. to 225 mg/mL. In some embodiments, the isolated anti-Aβ protofibril antibody is present in a concentration ranging from 100 mg/mL to 200 mg/mL. In some embodiments, the isolated antibody is present in a concentration of 80 mg/mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL, or 300 mg/mL. In some embodiments, the isolated antibody is present in a concentration of 100 mg/mL. In some embodiments, the isolated antibody is present in a concentration of 200 mg/mL. In some embodiments, the isolated antibody is present in a concentration of 250 mg/mL. In some embodiments, the isolated antibody is present in a concentration of 300 mg/mL. The pharmaceutical formulation of the invention comprises a pharmaceutically acceptable buffer. In some embodiments, the buffer is a citrate buffer. In some embodiments, the buffer is a histidine buffer. In some embodiments, the at least one additional component in the pharmaceutical formulation is chosen from emulsifiers. In some embodiments, the at least one additional component in the pharmaceutical formulation is chosen from citric acid (or citric acid monohydrate), sodium chloride and histidine (and/or histidine hydrochloride).

The concentration of arginine (and/or arginine hydrochloride) in the pharmaceutical formulation ranges from about 100 mM to about 400 mM. In some embodiments, the concentration of arginine (and/or arginine hydrochloride) in the pharmaceutical formulation ranges from about 110 mM to about 380 mM. In some embodiments, the concentration of arginine (and/or arginine hydrochloride) in the pharmaceutical formulation ranges from about 120 mM to about 360 mM. In some embodiments, the concentration of arginine (and/or arginine hydrochloride) in the pharmaceutical formulation ranges from about 125 mM to about 350 mM. In some embodiments, the concentration of arginine (and/or arginine hydrochloride) in the pharmaceutical formulation is 125 mM. In some embodiments, the concentration of arginine (and/or arginine hydrochloride) in the pharmaceutical formulation is 200 mM. In some embodiments, the concentration of arginine (and/or arginine hydrochloride) in the pharmaceutical formulation is 350 mM.

In some embodiments, the pharmaceutical formulation comprises histidine. In some embodiments, the concentration of histidine in the pharmaceutical formulation ranges from about 10 mM to about 100 mM. In some embodiments, the concentration of histidine in the pharmaceutical formulation ranges from 10 mM to 100 mM, 12 mM to 80 mM, 14 mM to 60 mM, 15 mM to 55 mM, 15 mM to 35 mM, or 15 mM to 25 mM. In some embodiments, the concentration of histidine is 25 mM. In some embodiments, the concentration of histidine is 50 mM.

The concentration of polysorbate 80 in the pharmaceutical formulation ranges from about 0.01 to 0. 1% w/v, 0.01 to 0.08% w/v, 0.02 to 0.08% w/v, 0.03 to 0.07% w/v, or 0.04 to 0.06% w/v. In some embodiments, the polysorbate 80 is present in the pharmaceutical formulation in a concentration of 0.0 1% w/v, 0.02% w/v, 0.03% w/v, 0.04% w/v, 0.05% w/v, 0.06% w/v, 0.07% w/v, or 0.08% w/v. In some embodiments, the polysorbate 80 is present in the pharmaceutical formulation in a concentration of 0.02% w/v. In some embodiments, the polysorbate 80 is present in the pharmaceutical formulation in a concentration of 0.05% w/v.

In some embodiments, the pharmaceutical formulation comprises citric acid monohydrate. In some embodiments, the concentration of citric acid monohydrate in the pharmaceutical formulation ranges from about 10 mM to 100 mM. In some embodiments, the concentration of citric acid monohydrate in the pharmaceutical formulation ranges from 10 mM to 100 mM, 10 mM to 90 mM, 15 mM to 85 mM, 20 mM to 80 mM, 25 mM to 75 mM, 30 mM to 70 mM, 30 mM to 60 mM, or 30 mM to 50 mM. In some embodiments, the concentration of citric acid monohydrate in the pharmaceutical formulation is 50 mM.

The disclosure provides a pharmaceutical formulation having a pH in the range of 4.5 to 5.5. In some embodiments, the pH is 4.5, 4.6, 4.7, 4.8, 4.9, 5.0, 5.1, 5.2, 5.3, 5.4 or 5.5. In some embodiments, the pH is 5.0.

In some embodiments, the pharmaceutical formulations disclosed herein may be in the form of a solution and/or any other suitable liquid formulation deemed appropriate by one of ordinary skill in the art. The route of administration of the compositions of the present disclosure may be intravenous or subcutaneous. In some embodiments, the pharmaceutical formulation is formulated as a sterile, non-pyrogenic liquid for intravenous administration. In some embodiments, the pharmaceutical formulation is formulated as a sterile, non-pyrogenic liquid for subcutaneous administration. In some embodiments, the pharmaceutical composition is a saline solution.

In some embodiments, the pharmaceutical formulation comprises 100 mg/mL of BAN2401, 50 mM citric acid monohydrate, 110 mM arginine. 240 mM arginine hydrochloride, and 0.05% (w/v) polysorbate 80, and has a pH of 5.0 ± 0.4.

In some embodiments, the pharmaceutical formulation comprises 100 mg/mL or 200 mg/mL of BAN2401, 25 mM of histidine and histidine hydrochloride, 200 mM arginine hydrochloride, and 0.05% (w/v) polysorbate 80, and has a pH of 5.0 ± 0.4.

In some embodiments, the pharmaceutical formulation comprises 200 mg/mL of BAN2401, 50 mM histidine and histidine hydrochloride, 125 mM arginine hydrochloride, and 0.02% (w/v) polysorbate 80, and has a pH of 5.0 ± 0.4.

In some embodiments, the pharmaceutical formulation comprises 200 mg/mLof BAN2401, 50 mM citric acid (and/or citric acid monohydrate), 125 mM arginine (and/or arginine hydrochloride), and 0.02% (w/v) polysorbate 80, and has a pH of 5.0 ± 0.4.

BAN2401 and methods comprising the use of BAN2401 are disclosed in U.S. Provisional Application No. 62/749,614 and PCT International Application No. PCT/US2019/043067.

### Brief Description of the Drawings

FIG. 1 depicts percentage of aggregation in formulations of BAN2401 at 10 mg/mL, 100 mg/mL, and 200 mg/mL at time points 0, 1, 2, and 3 months at 5°C.

FIG. 2 depicts percentage of fragmentation in formulations of BAN2401 at 10 mg/mL, 100 mg/mL, and 200 mg/mL at time points 0, 1, 2, and 3 months at 5 °C.

FIG. 3 depicts percentage of monomers in formulations of BAN2401at 10 mg/mL, 100 mg/mL, and 200 mg/mL at time points 0, 1, 2, and 3 months at 5 °C.

FIG. 4 depicts percentage of aggregation in formulations of BAN2401 at 10 mg/mL, 100 mg/mL, and 200 mg/mL at time points 0, 1, 2, and 3 months at 25 °C.

FIG. 5 depicts percentage of fragmentation in formulations of BAN2401at 10 mg/mL, 100 mg/mL, and 200 mg/mL at time points 0, 1, 2, and 3 months at 25 °C.

FIG. 6 depicts percentage of monomers in formulations of BAN2401 at 10 mg/mL, 100 mg/mL, and 200 mg/mL at time points 0, 1, 2, and 3 months at 25 °C.

FIG. 7 depicts percentage of aggregation in formulations of BAN2401 at pH values of 4.5, 5.0, 5.5, 6.0, and 6.5 at time points 0, 1, 2, and 3 months at 5 °C.

FIG. 8 depicts percentage of fragmentation in formulations of BAN2401 at pH values of 4.5, 5.0, 5.5, 6.0, and 6.5 at time points 0, 1, 2, and 3 months at 5 °C.

FIG. 9 depicts percentage of monomers in formulations of BAN2401 at pH values of 4.5, 5.0, 5.5, 6.0, and 6.5 at time points 0, 1, 2, and 3 months at 5 °C.

FIG. 10 depicts percentage of aggregation in formulations of BAN240I at pH values of 4.5, 5.0, 5.5, 6.0, and 6.5 at time points 0, 1, 2, and 3 months at 25 °C.

FIG. 11 depicts percentage of fragmentation in formulations of BAN2401at pH values of 4.5, 5.0, 5.5, 6.0, and 6.5 at time points 0, 1, 2, and 3 months at 25 °C.

FIG. 12 depicts percentage of monomers in formulations of BAN2401 at pH values of 4.5, 5.0, 5.5, 6.0, and 6.5 at time points 0, 1, 2, and 3 months at 25 °C.

FIG. 13 depicts percentage of aggregation in formulations of BAN2401at 10 mg/mL, 100 mg/mL, and 200 mg/mL at time points 0, 1, and 2 months at 25 °C.

FIG. 14 depicts percentage of fragmentation in formulations of BAN2401 at 10 mg/mL, 100 mg/mL, and 200 mg/mL at time points 0, 1, and 2 months at 25 °C.

FIG. 15 depicts percentage of monomers in formulations of BAN2401 at 10 mg/mL, 100 mg/mL, and 200 mg/mL at time points 0, 1, and 2 months at 25 °C.

FIG. 16 depicts percentage of aggregation of monomers in formulations of BAN2401 as a function of arginine concentration.

FIG. 17 depicts a flowchart for the process of manufacturing a BAN2401 10 mg/mL injection.

FIG. 18 depicts a flowchart for the process of manufacturing a BAN2401 100 mg/mL injection.

FIG. 19 depicts the pH values of formulations of BAN2401 at pH values at time points 0 and 12 months at 5 °C.

FIG. 20 depicts the pH values of formulations of BAN2401 at pH values at time points 0, 1, and 3 months at 25 °C.

FIG. 21 depicts the pH values of formulations of BAN2401 at pH values after one month at 5 °C and with agitation at 25 °C.

FIG. 22 depicts the absorbance at 405 nm of formulations of BAN2401 at time points 0, 1, 3, 6, 9, and 12 months at 5 °C.

FIG. 23 depicts the absorbance at 405 nm of formulations of BAN2401 at time points 0, 1, and 3 months at 25 °C.

FIG. 24 depicts the absorbance at 405 nm of formulations of BAN2401 at one month at 5 °C and with agitation at 25 °C.

FIG. 25 depicts percentage of aggregation in formulations of BAN2401 at time points 0, 1, 3, 6, 9, and 12 months at 5 °C.

FIG. 26 depicts percentage of fragmentation in formulations of BAN2401 at time points 0, 1, 3, 6, 9, and 12 months at 5 °C.

FIG. 27 depicts percentage of monomers in formulations of BAN2401 at time points 0, 1, 3, 6, 9, and 12 months at 5 °C.

FIG. 28 depicts percentage of aggregation in formulations of BAN2401 at time points 0, 1, and 3 months at 25 °C.

FIG. 29 depicts percentage of fragmentation in formulations of BAN2401 at time points 0, 1, and 3 months at 25 °C.

FIG. 30 depicts percentage of monomers in formulations of BAN2401 at time points 0, 1, and 3 months at 25 °C.

FIG. 31 depicts percentage of aggregation in formulations of BAN2401 at one month at 5 °C and with agitation at 25 °C.

FIG. 32 depicts percentage of fragmentation in formulations of BAN2401 at one month at 5 °C and with agitation at 25 °C.

FIG. 33 depicts percentage of monomers in formulations of BAN2401 at one month at 5 °C and with agitation at 25 °C.

*Definitions*

The following are definitions of terms used in the present application.

As used herein, the singular terms "a," "an," and "the" include the plural reference unless the context clearly indicates otherwise.

The phrase "and/or," as used herein, means "either or both" of the elements so conjoined, i.e., elements that are conjunctively present in some cases and disjunctively present in other cases. Thus, as a non-limiting example, "A and/or B," when used in conjunction with open-ended language such as "comprising" can refer, in some embodiments, to A only (optionally including elements other than B); in other embodiments, to B only (optionally including elements other than A); in yet other embodiments, to both A and B (optionally including other elements); etc.

As used herein, "at least one" means one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

When a number is recited, either alone or as part of a numerical range, it should be understood that the numerical value can vary above and below the stated value by a variance that is reasonable for the value described, as recognized by one of skill in the art.

As used herein, a "fragment" of an antibody comprises a portion of the antibody, for example comprising an antigen-binding or a variable region thereof. Non-limiting examples of fragments include Fab fragments, Fab' fragments, F(ab')2 fragments, Fv fragments, diabodies, linear antibodies, and single-chain antibody molecules.

As used herein, "fragmentation" or "fragment formation" refers to degradation of an antibody or fragment thereof when it is in or added to a formulation. A fragment generated by the fragmentation or the fragment formation may or may not be capable of binding to the antigen to which the antibody or fragment thereof binds.

As used herein "histidine buffer" may comprise histidine, histidine hydrochloride, or a combination thereof, wherein histidine hydrochloride may be histidine hydrochloride monohydrate.

As used herein "citrate buffer" may comprise citric acid, its salts, or a combination thereof, wherein citric acid may be citric acid monohydrate or anhydrous citric acid.

*Non-limiting embodiments of the disclosure:*

Certain embodiments of the present disclosure relate to aqueous pharmaceutical formulations.

**Concentration of** Antibody

For any of the aqueous pharmaceutical formulations described herein, the antibody may be present in the following concentrations.

In some embodiments of the pharmaceutical formulation, the isolated anti-Aβ protofibril antibody is present in a concentration of 100 mg/ml.

In some embodiments of the pharmaceutical formulation, the isolated anti-Aβ protofibril antibody is present in a concentration of 200 mg/mL.

In some embodiments of the pharmaceutical formulation, the isolated anti-Aβ protofibril antibody is present in a concentration ranging from 80 mg/mL to 300 mg/mL. In some embodiments of the pharmaceutical formulation, the isolated anti-Aβ protofibril antibody is present in a concentration ranging from 80 mg/mL to 240 mg/mL.

In some embodiments of the pharmaceutical formulation, the isolated anti-Aβ protofibril antibody is present in a concentration ranging from 100 mg/mL to 200 mg/mL.

In some embodiments of the pharmaceutical formulation, the isolated anti-Aβ protofibril antibody is present in a concentration ranging from 80 mg/mL to 300 mg/mL, 85 mg/mL to 275 mg/mL, 90 mg/mL to 250 mg/mL, 95 mg/mL to 225 mg/mL, 100 to 200 mg/mL. In some embodiments of the pharmaceutical formulation, the isolated anti-Aβ protofibril antibody is present in a concentration ranging from 90 mg/mL to 220 mg/mL, 100 mg/mL to 210 mg/mL, or 110 mg/mL to 200 mg/mL.

In some embodiments of the pharmaceutical formulation, the isolated anti-Aβ protofibril antibody is present in a concentration of 80 mg/mL, 90 mg/mL, 100 mg/mL, 110 mg/mL, 120 mg/mL, 180 mg/mL, 190 mg/mL, 200 mg/mL, 210 mg/mL, 220 mg/mL, 230 mg/mL, 240 mg/mL, 250 mg/mL, 260 mg/mL, 270 mg/mL, 280 mg/mL, 290 mg/mL, or 300 mg/mL.

In some embodiments of the pharmaceutical formulation, the isolated anti-Aβ protofibril antibody is present in a concentration of 100 mg/ml.

In some embodiments of the pharmaceutical formulation, the isolated anti-Aβ protofibril antibody is present in a concentration of 200 mg/mL.

Arginine

For any of the aqueous pharmaceutical formulations described herein, the formulation comprises arginine. The arginine is arginine, arginine hydrochloride, or a combination thereof.

The concentration of arginine ranges from about 100 mM to about 400 mM.

In some embodiments of the pharmaceutical formulation, the concentration of arginine ranges from 110 mM to 380 mM, 120 mM to 360 mM, 125 mM to 350 mM, 100 mM to 200 mM, 125 mM to 200 mM or 150 mM to 200 mM. In some embodiments of the pharmaceutical formulation, the concentration of arginine ranges from 150 mM to 250 mM, 160 mM to 240 mM, 170 mM to 230 mM, 180 mM to 220 mM, 190 mM to 210 mM arginine, arginine hydrochloride, or a combination thereof.

In some embodiments of the pharmaceutical formulation, the concentration of arginine is 125 mM.

In some embodiments of the pharmaceutical formulation, the concentration of arginine is 200 mM.

In some embodiments of the pharmaceutical formulation, the concentration of arginine ranges from 200 mM to 400 mM, 210 mM to 390 mM, 220 mM to 380 mM, 230 mM to 370 mM, 240 mM to 360 mM, 240 mM to 350 mM or 250 mM to 350 mM.

In some embodiments of the pharmaceutical formulation, the concentration of arginine is 350 mM.

Polysorbate 80 (PS80)

For any of the aqueous pharmaceutical formulations described herein, the formulation comprises polysorbate 80 0.01% w/v to 0.1% w/v.

In some embodiments of the pharmaceutical formulation, the concentration of polysorbate 80 ranges from about 0.01% w/v to 0.08% w/v, 0.02% w/v to 0.08% w/v, 0.03% w/v to 0.07% w/v, or 0.04% w/v to 0.06% w/v.

In some embodiments of the pharmaceutical formulation, the polysorbate 80 is present in a concentration of 0.01% w/v, 0.02% w/v, 0.03% w/v, 0.04% w/v, 0.05% w/v, 0.06% w/v, 0.07% w/v, or 0.08% w/v.

In some embodiments of the pharmaceutical formulation, the polysorbate 80 is present in a concentration of 0.02% w/v.

In some embodiments of the pharmaceutical formulation, the polysorbate 80 is present in a concentration of 0.05% w/v.

Buffer

For any of the aqueous pharmaceutical formulations described herein, the formulation comprises a pharmaceutically acceptable buffer as follows.

In some embodiments of the pharmaceutical formulation, the pharmaceutically acceptable buffer is citrate buffer.

In some embodiments of the pharmaceutical formulation, the citrate buffer is present in a concentration of about 10 mM to about 100 mM.

In some embodiments of the pharmaceutical formulation, the concentration of the citrate buffer ranges from 10 mM to 100 mM, 10 mM to 90 mM, 15 mM to 85 mM, 20 mM to 80 mM, 25 mM to 75 mM, 30 mM to 70 mM, 30 mM to 60 mM, or 30 mM to 50 mM.

In some embodiments of the pharmaceutical formulation, the citrate buffer is present in a concentration of 50 mM.

In some embodiments of the pharmaceutical formulation, the pharmaceutically acceptable buffer is a histidine buffer.

In some embodiments of the pharmaceutical formulation, the histidine buffer is present in a concentration of about 10 mM to about 100 mM.

In some embodiments of the pharmaceutical formulation, the concentration of the histidine buffer ranges from 10 mM to 100 mM, 12 mM to 80 mM, 14 mM to 60 mM, or 15 mM to 55 mM, 15 mM to 35 mM, or 15 mM to 25 mM.

In some embodiments of the pharmaceutical formulation, the histidine buffer is present in a concentration of 25 mM.

In some embodiments of the pharmaceutical formulation, the histidine buffer is present in a concentration of 50 mM.

In some embodiments of the pharmaceutical formulation, the histidine buffer comprises histidine and histidine hydrochloride monohydrate. In some embodiments of the pharmaceutical formulation, the histidine buffer comprises histidine and histidine hydrochloride monohydrate, wherein the histidine is in a concentration of about 0.1 to 0.3 mg/mL. and the histidine hydrochloride monohydrate is in a concentration of about 4 to 6 mg/mL. In some embodiments of the pharmaceutical formulation, the histidine buffer comprises histidine and histidine hydrochloride monohydrate, wherein the histidine is in a concentration of about 0.26 mg/mL, and the histidine hydrochloride monohydrate is in a concentration of about 4.89 mg/mL. In some embodiments of the pharmaceutical formulation, the histidine buffer comprises histidine and histidine hydrochloride monohydrate, wherein the histidine comprises 0.2-0.3 mg/mL histidine and 4.4 to 4.9 mg/mL histidine hydrochloride, optionally where the histidine hydrochloride is monohydrate.

**pH**

For any of the aqueous pharmaceutical formulations described herein, the formulation has a pH ranging from 4.5 to 5.5.

In some embodiments of the pharmaceutical formulation, the pH is 4.5, 4.6, 4.7, 4.8, 4.9, 5.0, 5.1, 5.2, 5.3, 5.4 or 5.5.

In some embodiments of the pharmaceutical formulation, the pH is 5.0.

In some embodiments of the pharmaceutical formulation, the pharmaceutical formulation is suitable for intravenous injection.

In some embodiments of the pharmaceutical formulation, the pharmaceutical formulation is suitable for subcutaneous injection.

In some embodiments of the aqueous pharmaceutical formulation, the pharmaceutical formulation comprises methionine.

In some embodiments, disclosed is an aqueous pharmaceutical formulation comprising:

    (a) 80 mg/mL to 240 mg/mL BAN2401,
    (b) 140 mM to 260 mM arginine hydrochloride,
    (c) 0.01% w/v to 0.1% w/v polysorbate 80, and
    (d) 15 mM to 35 mM histidine buffer,

wherein the pharmaceutical formulation has a pH ranging from 4.5 to 5.5.

In some embodiments, disclosed herein is an aqueous pharmaceutical formulation comprising:

    (a) 80 mg/mL to 120 mg/mL BAN2401,
    (b) 240 mM to 360 mM arginine,
    (c) 0.03% w/v to 0.08% w/v polysorbate 80, and
    (d) 30 mM to 70 mM citrate buffer,

wherein the pharmaceutical formulation has a pH ranging from 4.5 to 5.5, and wherein arginine is arginine, arginine hydrochloride, or a combination thereof.

In some embodiments, disclosed herein is an aqueous pharmaceutical formulation comprising:

    (a) 100 mg/mL BAN2401,
    (b) 200 mM arginine hydrochloride,
    (c) 0.05% w/v polysorbate 80, and

(d) 25 mM histidine buffer,

wherein the pharmaceutical formulation has a pH ranging from 4.5 to 5.5.

In some embodiments, disclosed herein is an aqueous pharmaceutical formulation comprising:

(a) 200 mg/mL BAN2401,
(b) 200 mM arginine hydrochloride,
(c) 0.05% w/v polysorbate 80, and
(d) 25 mM histidine buffer,

wherein the pharmaceutical formulation has a pH ranging from 4.5 to 5.5.

In some embodiments, disclosed herein is an aqueous pharmaceutical formulation comprising:

(a) 100 mg/mL BAN2401,
(b) 350 mM arginine,
(c) 0.05% w/v polysorbate 80, and
(d) 50 mM citrate buffer,

wherein the pharmaceutical formulation has a pH ranging from 4.5 to 5.5, and wherein arginine is arginine, arginine hydrochloride, or a combination thereof,

In some embodiments, disclosed herein is an aqueous pharmaceutical formulation comprising:

(a) 150 mg/mL to 250 mg/mL BAN2401,
(b) 100 mM to 150 mM arginine hydrochloride,
(c) 0.01% w/v to 0.05% w/v polysorbate 80, and
(d) 35 mM to 65 mM histidine buffer,

wherein the pharmaceutical formulation has a pH ranging from 4.5 to 5.5.

In some embodiments, the aqueous pharmaceutical formulation has pH 5.0.

## EXAMPLES

### Example 1: Protein Concentration Study

Samples with three protein concentrations (10, 100, 200 mg/mL) were prepared and examined in the concentration study. The 10 mg/mL material was BAN2401 purified drug substance (PDS) and the 100 and 200 mg/mL materials were produced from BAN2401 PDS using Amicon Ultra-15 spin filters. The samples were all in the same formulation buffer (25 mM sodium citrate, 125 mM sodium chloride, pH 5.7), except that each had a different PS80 level. The PS80 percentages of the 10, 100, 200 mg/mL samples were 0.02%, 0.16%, and 0.32%, respectively. All samples were 0.2 gm filtered and aliquoted into sterile polypropylene (PP) tubes for stability testing. (Note: The PS80 removal process was not available at the time that the concentration study was conducted.)

Sample stability was evaluated at two temperatures (5 & 25 °C) for 3 months using native HPLC-SEC, pH, and DLS. Additional characterization assays, such as PS80 verification, were performed on the T = 0 samples. Details of the sample testing are shown in Table 1.

**Table 1. Testing Schedule (Concentration Study)**

| Time Point | T = 0 | T = 1 mo | T = 2 mo | T = 3 mo |
|---|---|---|---|---|
| Temperature | N/A | 5 °C | 5 °C | 5 °C |
| | | 25 °C | 25 °C | 25 °C |
| Number of Samples | 3 | 6 | 6 | 6 |
| Testing | Native SEC | Native SEC | Native SEC | Native SEC |

| | pH DLS | pH DLS | pH DLS | pH DLS |
|---|---|---|---|---|

pH was measured using a pH meter with a microprobe. Prior to measurements, a calibration was performed using pH 4.0 and 7.0 standards.

Aggregation and physical degradation of the concentrated BAN2401 was assessed by performing native HPLC-SEC on stability samples stored at 5 and 25 °C.

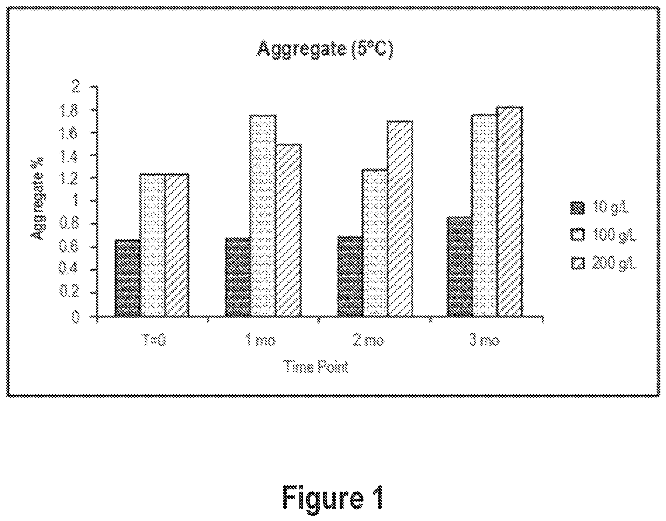

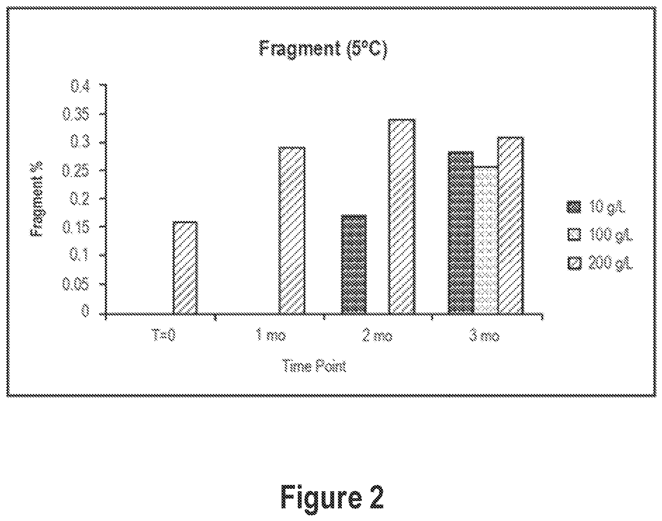

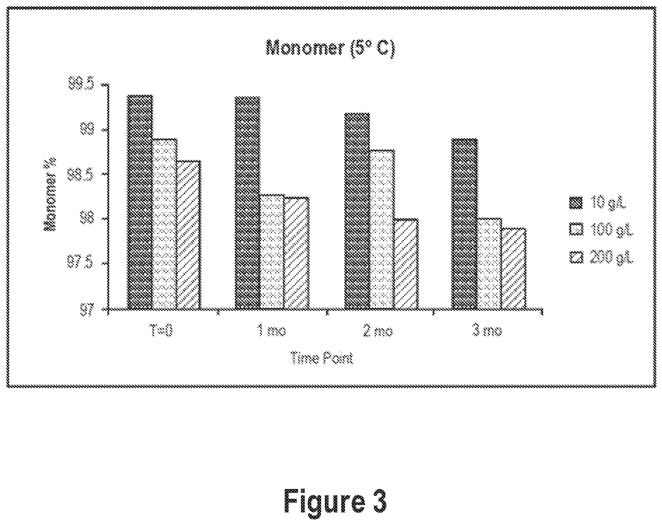

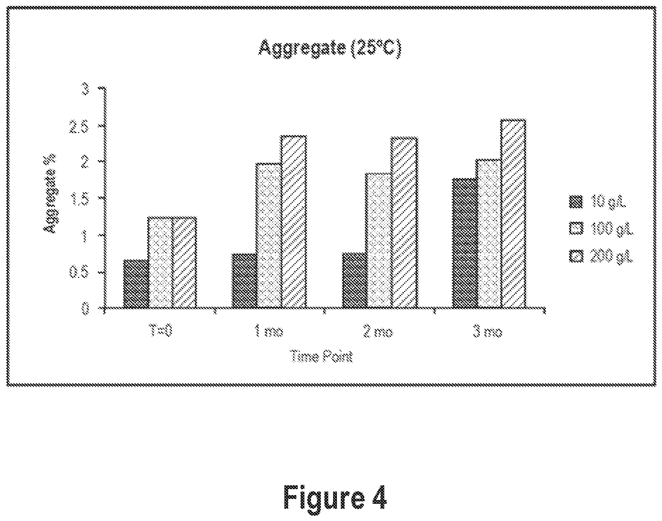

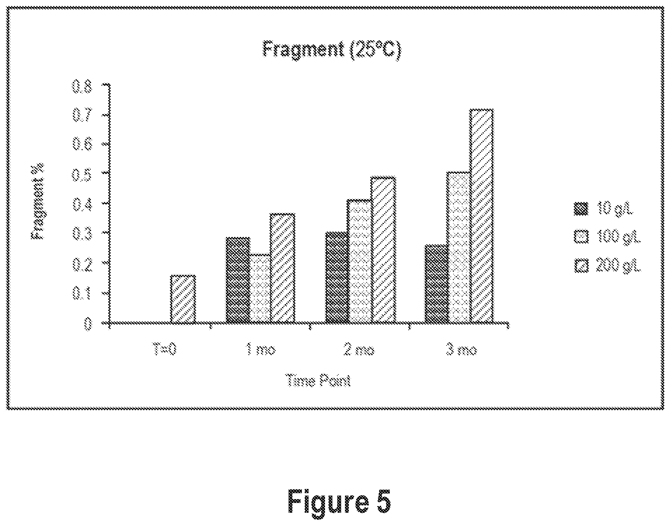

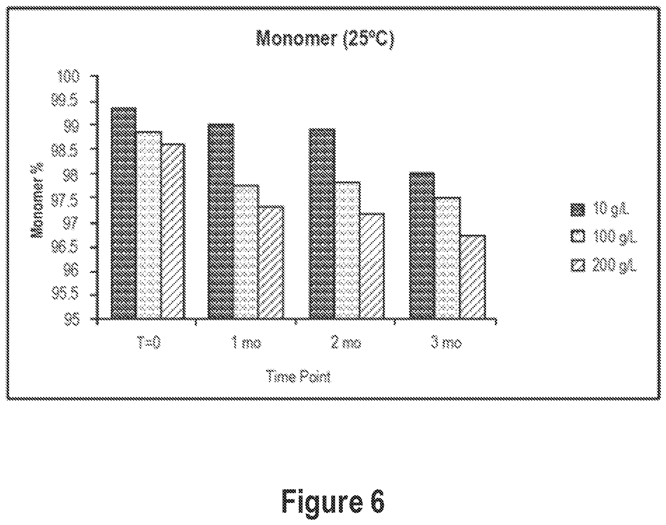

HPLC-SEC (Native) analysis was performed utilizing a TSK G3000 SWXL column with 0.2 M sodium phosphate, pH 7.0 mobile phase at a flow rate of 1.0 mL/min. The sample injection volumes were 15, 1.5, or 0.8 pi, for protein concentrations of 10, 100, or 200 mg/mL, respectively. This ensures approximately 150 pg of protein is injected onto the column across the study. Results of relative peak areas (%) for monomer, aggregate and fragment are reported in Figures 1-6.

At both temperatures (5 and 25 °C), higher aggregate percentages and aggregation rates were seen with increased protein concentrations. For the 100 and 200 mg/mL samples, the starting aggregate percentages were nearly twice as much as that of the 10 mg/mL sample. This indicated the concentration process alone caused protein to aggregate. Additional aggregation occurred during the storage at 5 and 25 °C. The average aggregation rate over 3 months at 5 °C: was 0.17% per month for 100 mg/mL, and 0.20% per month for 200 mg/mL. For comparison, the 10 mg/mL sample exhibited only a 0.07% per month aggregation rate under the same condition. In summary, it appeared that BAN2401 at 100 mg/mL or greater was not physically stable in the formulation.

Protein concentration was evaluated by UV by measuring the absorbance at 280 and 320 mu on a Beckman DU-800 spectrophotometer. Samples were diluted 500-fold and were prepared in duplicate. Protein concentration was calculated using an extinction coefficient, $\varepsilon$, of 1.32 using the following formula:

$$\text{Concentration} = (A280\text{-}A320)/\varepsilon \times \text{Dilution Factor}$$

Protein concentration was measured using a UV-Vis spectrophotometer. The results are listed in Table 2. Overall, the concentrations remained unchanged throughout the 3 months at both temperatures.

**Table 2. Protein Concentration Results (Concentration Study)**

| Sample ID | Protein Concentration (mg/ml) | | | | | | |
|---|---|---|---|---|---|---|---|
| | T = 0 | T= 1 mo | | T=2 mo | | T=3 mo | |
| | | 5 °C | 25 °C | 5 °C | 25 °C | 5 °C | 25 °C |
| 10 mg/ml | 10.6 | 9.93 | 9.93 | 9.9 | 10.0 | 10.11 | 10.15 |
| 100 mg/ml | 110.3 | 110.1 | 114.1 | 103.4 | 104.2 | 106.6 | 106.4 |
| 200 mg/ml | 210.8 | 197.2 | 224.5 | 207.4 | 205.4 | 205.3 | 205.8 |

Example 2: **pH** Screening Study

The stability of BAN2401 at 200 mg/mL was evaluated at five different pH values. To prepare the samples, PS80-removed BAN2401 PDS was concentrated and diafiltered into a buffer containing 50 mM sodium citrate, 100 mM sodium chloride, pH 4.5. The final concentration adjustment was performed via a dilution to achieve a concentration of 200 mg/mL protein and 0.02% PS80. The resulting material was divided into 5 aliquots; four of the aliquots were titrated with a 10 N sodium hydroxide to produce samples at different pH (*i.e.,* pH 5.0, 5.5, 6.0, and 6.5). The resulting samples were 0.2-$\mu$m filtered and sub-aliquoted into sterile polypropylene (PP) tubes for stability evaluation.

Stability of the samples was evaluated at two temperatures (5 & 25 °C) using native HPLC-SEC, pH, and DLS over 3 months. Additionally, an agitation study was conducted at the I-month time point, where a subset of the 5 °C samples was agitated horizontally at 250 RPM for 3 days in a 25 °C incubator. Details of the sample testing are shown in Table 3.

**Table 3. Testing Schedule (pH Study)**

| Time Point | T = 0 | T = 1 month | T = 2 months | T = 3 months |
|---|---|---|---|---|
| Temperature | n/a | 5 °C | 5 °C | 5 °C |
| | | 25 °C | 25 °C | 25 °C |
| Agitation | n/a | Agitation | Not required | Not required |
| Number of Samples | 5 | 15 | 10 | 10 |
| Testing | Native SEC<br><br>pH<br><br>DLS<br><br>DSC | Native SEC<br><br>pH<br><br>DLS | Native SEC<br><br>pH<br><br>DLS | Native SEC<br><br>pH<br><br>DLS |

SEC-HPLC analysis was performed utilizing a TSK G3000 SWXL column with 0.2 M sodium phosphate, pH 7.0 mobile phase at a flow rate of 1.0 mL/min. The sample injection volumes were 15, 1.5, or 0.8 $\mu$L for protein concentrations of 10, 100, or 200 mg/mL, respectively. This ensures approximately 150 $\mu$g of protein is injected onto the column across the study. Results of relative peak areas (%) for monomer, aggregate and fragment were reported.

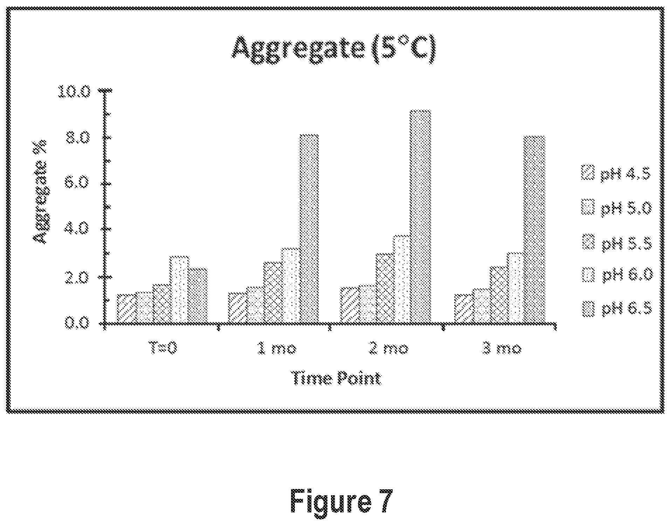

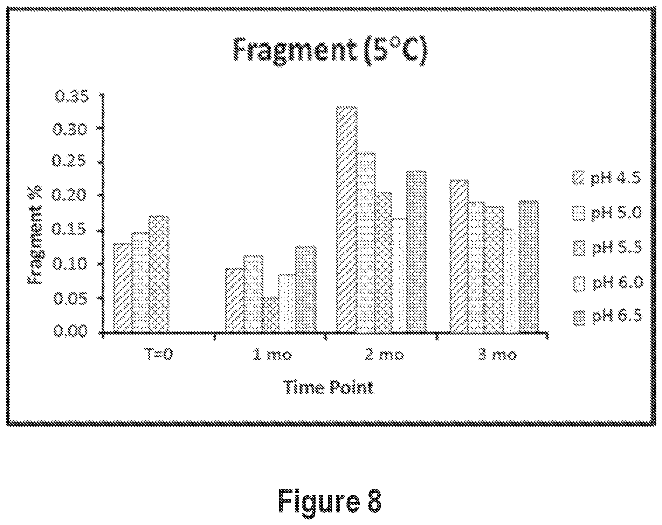

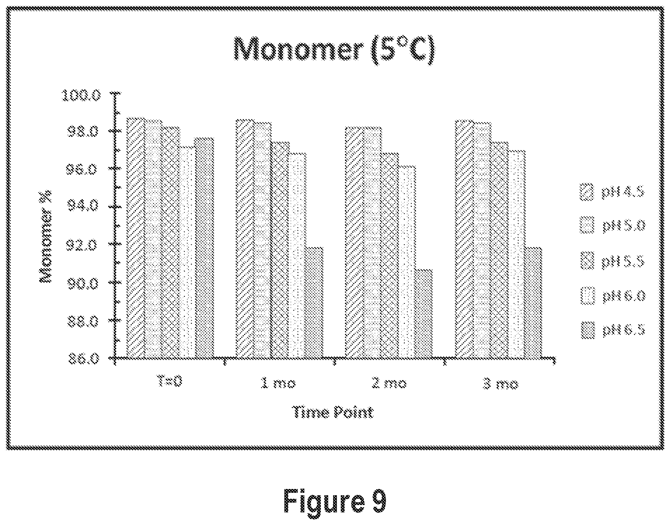

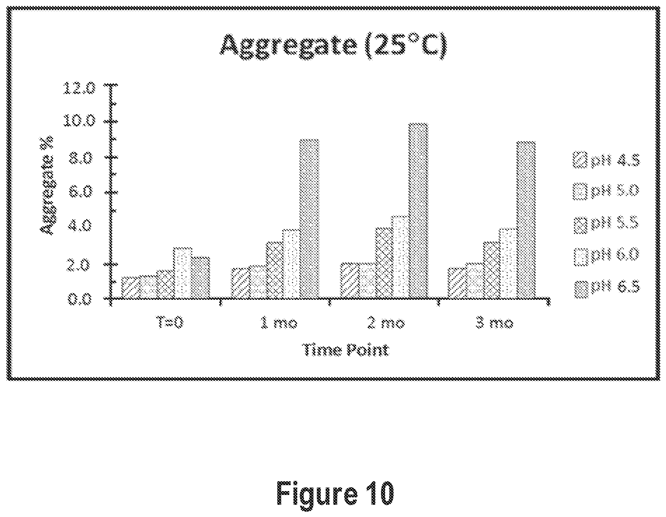

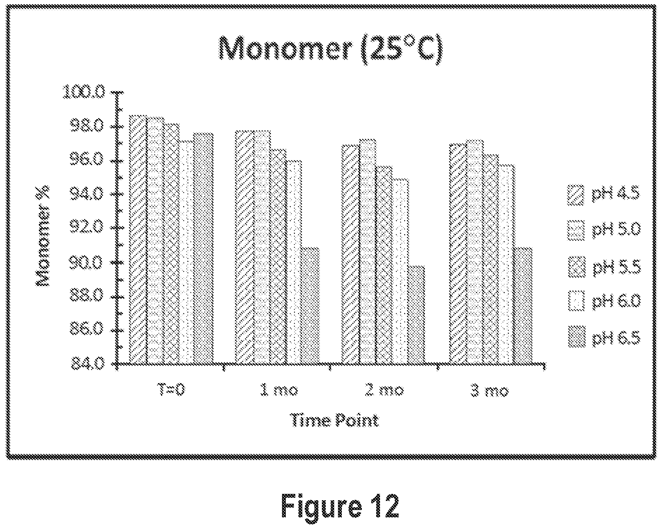

Aggregation and physical degradation of the concentrated BAN2401 was assessed by performing native HPLC-SEC on stability samples stored at 5 and 25 °C. The results are shown in Figures 7-12. BAN2401 appeared to be more stable at lower pH at both temperatures (5 and 25 °C). Formulations with pH 4.5 and 5.0 started with low aggregate percentages and exhibited a slower increase in aggregate formation, at a rate of -0.07% per month at 5 °C. In addition to aggregation, fragmentation of the protein was also analyzed. The results suggested that fragmentation of BAN2401 was more pronounced in formulations of BAN2401 at lower pH. Based on these considerations, formulations of BAN2401 at pH 5.0 were considered the most stable (*i.e.*, exhibited less aggregation and/or physical degradation of BAN2401 relative to other formulations at different pH values).

**Example 3: Excipient Screening Study**

Twelve excipients were screened in this study. To prepare the samples with BAN2401 at 200 mg/mL, PS80-removed BAN2401 was concentrated via a TFF step, followed by a diafiltration step with the base buffer (50 mM citrate, 0.02% PS80, pH 6.0). The concentrated material was aliquoted into twenty-four fractions. Each fraction was spiked with a stock solution containing a specific excipient. For the majority of the excipients, two concentrations were examined, except sodium chloride and ascorbic acid for which samples with three and one concentration level respectively were prepared. A list of the excipients used and their concentrations is shown in Table 4.

## Table 4. List of Excipients and Corresponding Concentrations

| Excipient | Concentration (Formulation #) |
|---|---|
| None | Control sample (F0) contained the protein in the base buffer without any excipient added. |
| Sucrose | 1% (F1), 6% (F2) |
| Trehalose | 0.5% (F3), 3.5% (F4) |
| Mannitol | 0.5% (F5), 1.8% (F6) |
| Proline | 50 Mm (F9), 400 Mm (F10) |
| Glycine | 50 Mm (F11), 400 Mm (F12) |
| Arginine | 25 Mm (F13), 160 mM (F14) |

| NaCl | 50 mM (F16), 150 mM (F17), 500 mM (F18) |
|---|---|
| PS80 | 0.05% v/v (F19), 0.1% v/v (F20) |
| PEG400 | 1% (F21), 6% (F22) |
| Dextran, LMW | 1% (F23), 5% (F24) |
| Ascorbic Acid | 50 Mm (F15) |
| Sorbitol | 1% (F7), 6% (F8) |

All samples, including the control (i.e., the sample in the base buffer without excipient), were 0.2-gm filtered and then filled into BD syringes aseptically using a hand stoppering tool. Control samples were also filled in glass vials. Vials and the BD syringes were placed at 5 and 25 °C. Sample stability was evaluated for 2 months using native HPLC-SEC and pH.

The samples of the excipient screening study were prepared in a suboptimal buffer (50 mM citrate, 0.02% PS80, pH 6.0) condition in order to amplify positive effects from excipients in preventing aggregate formation. The stability profile (aggregation) of each formulation over 2 months at 25 °C was assessed by performing native HPLC-SEC.

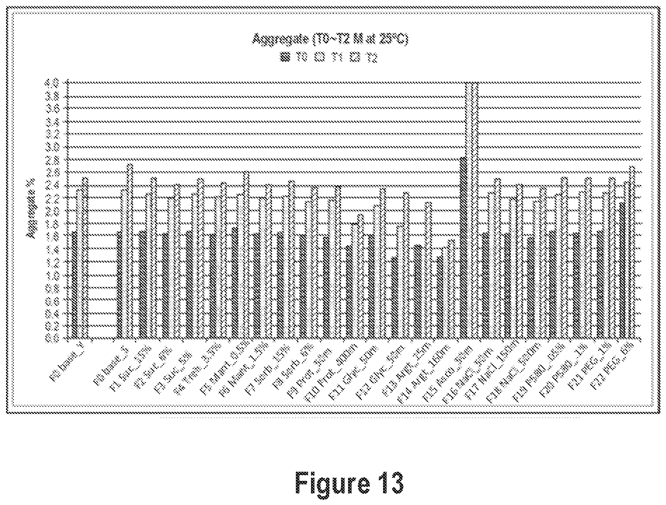

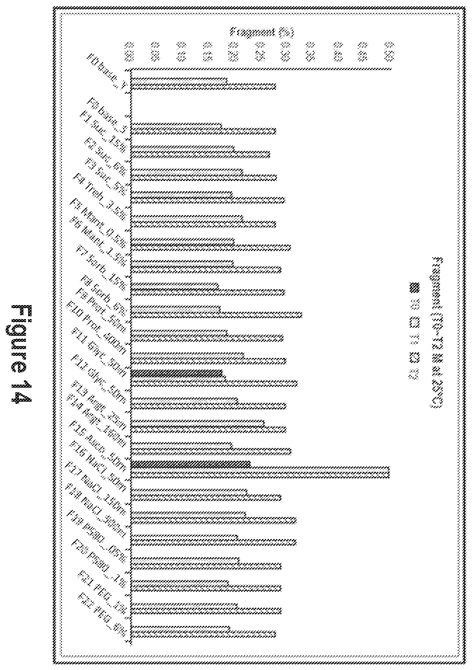

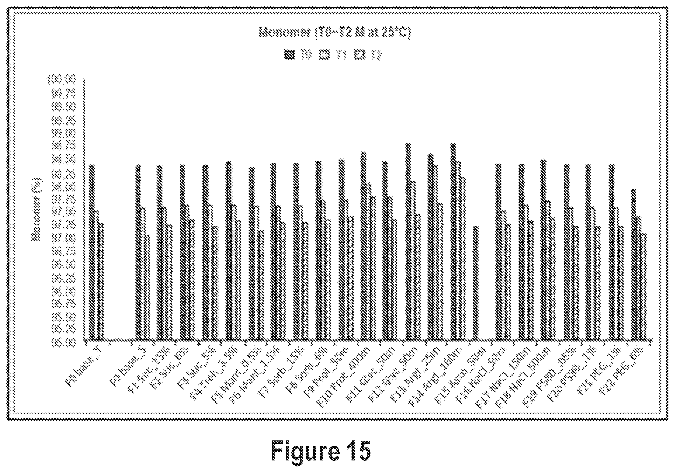

As shown in Figures 13-15, the formulation containing 160 mM arginine (F14) produced the lowest aggregation rate among the formulations. As a comparison, the 160 mM arginine formulation exhibited only 1.4% aggregates at 25 °C after 2 months while the control (i.e., the formulation in the base buffer without excipient - F0) showed 2.4% aggregates at the same time under the same condition.

Additionally, formulations with arginine had the lowest starting aggregate percentages, indicating arginine was capable of suppressing aggregate formation during the protein concentration step. The fragmentation was slightly higher with the arginine formulation (F14) as compared to the control (F0). However, the difference could have been within the assay variability.

The second-best excipient in this study was the formulation with 400 mM proline (F10), although its effect in controlling aggregation was not as effective as the arginine formulation (F14).

The formulations with sodium chloride (F16-F18) showed no effect on the stability as compared to the control sample. The same observation was made for the formulations with PS80 (F19 and F20): no stability effect was observed by varying the PS80 content. The formulation with the ascorbic acid (F15) showed a dramatic effect an increasing aggregate and fragment percentages from the beginning of the study.

The pH of the two arginine formulations (F13 & F14) was measured to verify that the presence of arginine in the formulations had not caused the pH to drift over time. As shown in Table 4, no change in pH was observed. The osmolality of the two formulations was also measured. The samples were stored at -20 °C before they were thawed at the same time for measurements.

**Table 5. pH and Osmolality of Arginine Formulations (F13 & F14)**

| Arginine Formulation (F13 and F14; time point) | pH | mOsm/kg |
|---|---|---|
| F13, T=0 | no sample | no sample |
| F14, T=0 | 5.86 | no sample |
| F13, T=1 month | 5.97 | 231 |
| F14, T=1 month | 5.88 | 477 |
| F13, T=2 months | 5.97 | 230 |
| F14, T=2 months | 5.89 | 459 |

**Example 4: Arginine Concentration Study**

The relation between concentration of arginine and aggregates in the formulation was studied by Design Expert® 7.0. The factors chosen for study, and the levels explored for each factor are shown in Table 6 below.

**Table 6. Factors Investigated in Example 4**

| Factor | Factor Range | Factor Levels |
|---|---|---|
| BAN2401 | 30 to 120 mg/mL | 30, 53, 75, 98, 120 mg/mL |
| pH | 4.5 to 6.0 | 4.5, 4.9, 5.2, 5.6, and 6.0 |
| Arginine | 0 to 450 mM | 0, 225, 338, and 450 mM |
| Polysorbate 80 | 0 to 0.1% | 0, 0.025, 0.05, 0.075, and 0.1% |

A sample randomization was generated by D-optimal design in Design Expert® 7.0. The obtained sample set contained 25 data points including 4 replicates, as shown in Table 7. Each formulation was prepared and evaluated.

## Table 7. Formulations prepared for Example 4

| Entry | BAN2401 (mg/mL) | pH | Arginine (mM) | Polysorbate 80 (%) |
|---|---|---|---|---|
| 1 | 30 | 5.2 | 0 | 0.000 |
| 2 | 30 | 6.0 | 0 | 0.050 |
| 3 | 30 | 4.5 | 0 | 0.100 |
| 4 | 30 | 4.5 | 0 | 0.100 |
| 5 | 30 | 6.0 | 225 | 0.000 |
| 6 | 30 | 4.5 | 450 | 0.000 |
| 7 | 30 | 4.5 | 450 | 0.000 |
| 8 | 30 | 6.0 | 450 | 0.100 |

| | | | | |
|---|---|---|---|---|
| 9 | 30 | 6.0 | 450 | 0.100 |
| 10 | 53 | 5.6 | 225 | 0.025 |
| 11 | 53 | 4.9 | 338 | 0.075 |
| 12 | 75 | 4.5 | 0 | 0.000 |
| 13 | 75 | 4.5 | 0 | 0.000 |
| 14 | 75 | 6.0 | 0 | 0.100 |
| 15 | 75 | 5.2 | 225 | 0.000 |
| 16 | 75 | 6.0 | 450 | 0.000 |
| 17 | 75 | 6.0 | 450 | 0.000 |
| 18 | 98 | 4.5 | 450 | 0.100 |
| 19 | 98 | 5.6 | 225 | 0.025 |
| 20 | 120 | 4.9 | 338 | 0.075 |
| 21 | 120 | 6.0 | 0 | 0.000 |
| 22 | 120 | 4.5 | 0 | 0.100 |
| 23 | 120 | 5.2 | 225 | 0.050 |
| 24 | 120 | 4.5 | 450 | 0.000 |
| 25 | 120 | 6.0 | 450 | 0.100 |

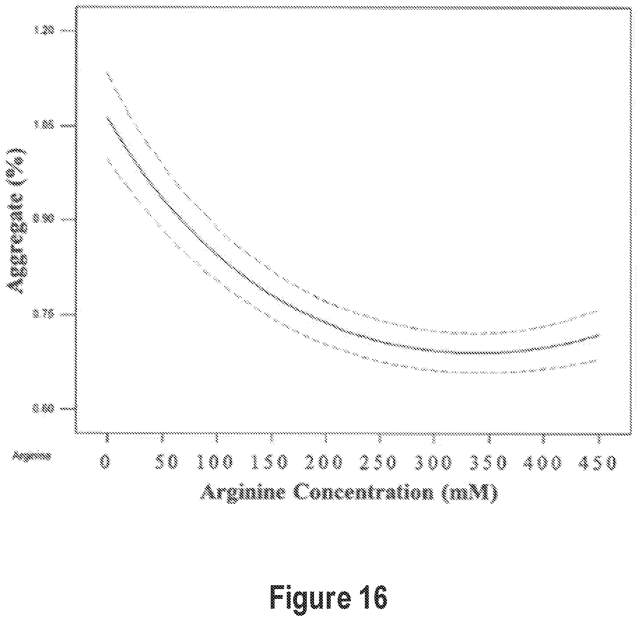

The relation between arginine concentration and aggregate levels in 100 mg/mL BAN2401 formulations at pH 5.0 containing 0.02% polysorbate 80 was estimated by Design Expert® 7.0. The result of the estimation was shown in Figure 16.

**Example 5: PS80 Study**

To evaluate the changes in drug product quality under stressed conditions, agitation and freeze-thaw studies were performed using a formulation buffer (pH 5.0, 350 mM arginine, 50 mM citric acid). In this experiment, formulations with varying polysorbate 80 concentration were investigated to confirm the effect of polysorbate 80 on sub-visible particle generation under such stressed conditions. The formulations investigated and applied stress conditions are shown in Table 8.

**Table 8. Formulations Studied for Agitation and Freeze-Thaw Stress Studies**

| Formulation | Concentrations of Polysorbate 80 | Stress Condition |
|---|---|---|
| 100 mg/mL BAN2401 50 mM citrate buffer 350 mM Arginine pH 5.0 | • 0% <br> • 0.02% <br> • 0.05% <br> • 0.1% | • Shaking at 250 rpm for 3 days at 5 °C <br> • Shaking at 250 rpm for 3 days at ambient temperature <br> • Three freeze-thaw cycles (-20 °C to ambient temperature) |

The samples were prepared in the same manner as described in Example 4. A 10% PS80 solution was added to achieve the target PS80 concentration in the formulations and the final protein concentration adjustment was performed via dilution with the formulation buffer. The formulations were passed through 0.2 gm filters and filled into 2 mL vials with a 1.3 mL fill volume. The samples were placed on an orbital shaker in a horizontal orientation (vial laying on its side) which was then placed in a refrigerator or on the lab bench. The samples were shaken at 250 rpm for 3 days. Other samples were frozen by placing in a -20°C chamber for 2 hours, then removed and left at room temperature for 2 hours to thaw. The freeze-thaw cycle was repeated three times. Samples from the agitation and freeze-thaw studies were evaluated for visual appearance, aggregate and fragment levels by SEC-HPLC, and sub-visible particles by Micro Flow Imaging (MFI).

The results of the agitation and freeze-thaw studies on BAN2401 formulations with varying levels of PS80 are summarized below.

### SEC-HPLC analysis

SEC-HPLC analysis was performed utilizing a TSK G3000 SWXL column with 0.2 M sodium phosphate, pH 7.0 mobile phase at a flow rate of 1.0 mL/min. The sample injection volumes were 5.0 $\mu$L or 2.5 $\mu$L for protein concentrations of 50 or 100 mg/mL, respectively. This ensures approximately 250 $\mu$g of protein is injected onto the column across the study. Results of relative peak areas (%) for monomer, aggregate and fragment were reported.

In the level of aggregates in pH 5 formulations stored at 25 °C for 3 months with varying polysorbate 80 concentrations, there was no significant difference in aggregate levels when the PS80 concentration was increased from 0% to 0.06%, at a formulation pH of 5.0. It is concluded that PS80 does not affect the formation of aggregates, i.e. dimer and trimer as measured by SEC-HPLC, during storage of BAN2401 formulations at 25 °C.

In the effect of polysorbate 80 concentration on fragment levels in formulations at pH 5, polysorbate 80 concentration does not affect fragment levels in BAN2401.

In the appearance of formulations after shaking at 250 rpm for 3 days at ambient temperature, the sample without PS80 was cloudy with precipitated protein, while the other samples (0.02%, 0.05% and 0.1% polysorbate 80) were visibly clear, particle free solutions. All samples that were shaken at 250 rpm for 3 days at 5 °C were clear and free of precipitate. All samples subjected to freeze/thaw cycles were also clear and free of precipitate.

The aggregate and fragment levels in BAN2401 formulations subjected to agitation and freeze-thaw are shown in Table 9. The stress condition that caused the greatest instability was agitation under ambient conditions. Under these conditions, the sample without PS80 showed the greatest formation of High Molecular Weight species, and the greatest loss in monomer. This effect was nullified as the PS80 concentration increased.

**Table 9. Results of Aggregates and Fragment Levels for the Agitation and Freeze-Thaw Studies**

| Stress Condition | Polysorbate 80 (%) | HPLC purity (% Area) | | | |
|---|---|---|---|---|---|
| | | HMW1[a] | HMW2[a] | Monomer | LMW[b] |
| 5 °C (control) | 0 | ~ | 0.4 | 99.4 | 0.2 |
| | 0.02 | - | 0.5 | 99.3 | 0.2 |
| | 0.05 | ~ | 0.5 | 99.4 | 0.2 |
| | 0.1 | ~ | 0.5 | 99.4 | 0.2 |
| Shaking at 5 °C | 0 | 0.5 | 0.6 | 98.7 | 0.2 |
| | 0.02 | ~ | 0.5 | 99.3 | 0.2 |
| | 0.05 | - | 0.5 | 99.3 | 0.2 |
| | 0.1 | - | 0.5 | 99.3 | 0.2 |
| Shaking at ambient temperature | 0[c] | 1.5 | 1.2 | 97.1 | 0.2 |
| | 0.02 | 4.0 | 0.8 | 95.1 | 0.2 |
| | 0.05 | 0.1 | 0.5 | 99.2 | 0.2 |
| | 0.1 | ~ | 0.5 | 99.3 | 0.2 |
| Three freeze/thaw cycles | 0 | - | 0.4 | 99.4 | 0.2 |
| | 0.02 | - | 0.5 | 99.4 | 0.2 |
| | 0.05 | ~ | 0.4 | 99.4 | 0.2 |
| | 0.1 | ~ | 0.5 | 99.4 | 0.2 |

[a] High Molecular Weight Species; [b] Low Molecular Weight Species; and [c] Sample was cloudy

*Sub-visible particle analysis*

Sub-visible particle analysis was performed using a Micro Flow Imaging (DPA4100 Flow Microscope and BP-4100-FC-400-UN flow cell). Total sample volume was 0.9 mL and the results were reported for 2.25-100 μm, 5-100 μm, 10-100 μm range, and 25-100 μm range.

An increase in sub-visible particles (2 to 10 pm) was observed in the samples without PS80 that were subjected to shaking and freeze-thaw. The generation of sub-visible particles was increasingly suppressed as the PS80 concentration in the formulation was increased. This effect was observed in all the size ranges studied. There was no significant change in sub-visible particles in the formulations containing 0.05% and 0.1% polysorbate 80. Therefore, a PS80 concentration of 0.05% was chosen as optimal for the formulation.

The data would suggest that PS80 has no effect on the formation of BAN2401 aggregates (dimers and trimers) and fragments as measured by SEC-HPLC. Therefore, the PS80 concentration was selected based on the results of the agitation and freeze-thaw studies. A PS80 concentration of 0.05% was chosen to prevent potential precipitation during shipping and to minimize formation of sub-visible particles.

### Example 6: Preparation of intravenous (IV) formulations of BAN2401

### A. BAN2401 10 mg/mL Formulation

A BAN2401 10 mg/mL formulation for intravenous injection ("10 mg/mL Injection") was manufactured by a conventional cGMP aseptic process for preparation of a sterile aqueous formulation. BAN2401 10 mg/mL Injection was produced from the corresponding BAN2401 drug substances formulation below without addition of any excipients and dilution. An exemplary IV formulation containing 10 mg/mL BAN2401 is shown in Table 10.

**Table 10. Comparative 10 mg/mL IV formulation comprising BAN2401.**

| component | composition |
|---|---|
| BAN2401 | 10 mg/mL |
| Sodium citrate/Citric acid buffer | 25 mM |
| Sodium Chloride | 125 mM |

| | |
|---|---|
| Polysorbate 80 | 0.02 % (w/v) |
| Water for Injection | QS |
| pH | 5.7 |

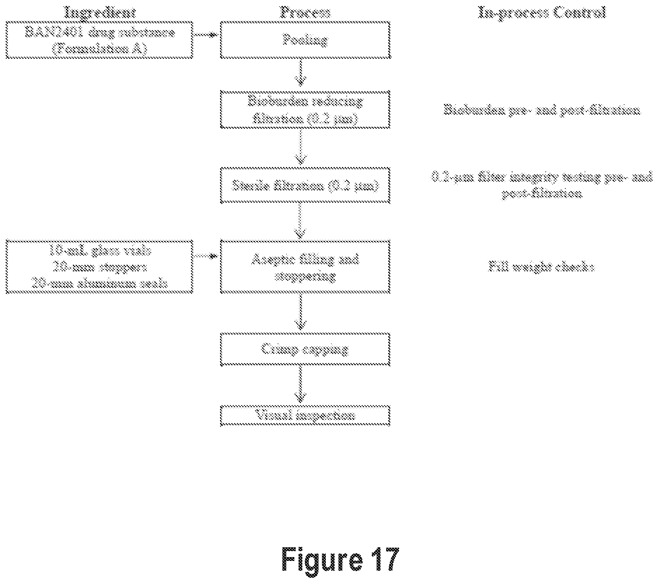

The filtered BAN2401 drug substance solution was aseptically filled into vials as illustrated in Figure 17 (referred to in Figure 17 as "Formulation A"). The pooled drug substance underwent a bioburden reducing filtration step through a 0.2-pm filter. The final sterile filtration was performed through two 0.2-pm filters in series, and pre-and post-filtration filter integrity tests were conducted. The sterile bulk drug product was filled aseptically into vials. During the filling operation, filling accuracy was confirmed by measuring the vial fill weight. Filled vials were stoppered and then sealed with an aluminum overseal. After crimp capping, the product was stored at 5± 3 °C.

The vials were analyzed using Method 1 (Light Obscuration) according to USP 788. Results are shown in Tables 11 and 12.

**Table 11. Particle Size Distribution (particles/container) for 10 mg/mL Injection Vials**

| Lot No. | A1 | A2 | A3 | | A4 | A5 | | A6 | A7 | A8 | A9 | | A10 | A11 | A12 |
|---|---|---|---|---|---|---|---|---|---|---|---|---|---|---|---|
| 25 µm | 1 | 1 | 1 | 0 | 1 | 1 | 1 | 1 | 1 | 1 | 5 | 5 | 2 | 1 | 1 |
| 10 µm | 126 | 145 | 469 | 241 | 469 | 225 | 107 | 617 | 249 | 315 | 215 | 273 | 231 | 191 | 125 |
| 5 µm | ~ | ~ | ~ | 2922 | - | ~ | ~ | - | - | ~ | ~ | ~ | ~ | ~ | ~ |
| 2 µm | - | - | - | 16636 | ~ | ~ | ~ | ~ | ~ | - | - | - | - | - | - |

# EP 4 121 007 B1

## Table 12. Particle Size Distribution (particles/mL) for 10 mg/mL Injection Vials

| Lot No. | A1 | A2 | A3 | | A4 | A5 | | A6 | A7 | A8 | A9 | | A10 | A11 | A12 |
|---|---|---|---|---|---|---|---|---|---|---|---|---|---|---|---|
| 25 µm | 0.1 | 0.1 | 0.1 | 0 | 0.1 | 0.1 | 0.1 | 0.1 | 0.1 | 0.1 | 0.5 | 0.5 | 0.2 | 0.1 | 0.1 |
| 10 µm | 12.6 | 14.5 | 46.9 | 24.1 | 46.9 | 22.5 | 10.7 | 61.7 | 24.9 | 31.5 | 21.5 | 27.3 | 23.1 | 19.1 | 12.5 |
| 5 µm | - | - | - | 292.2 | - | - | | - | - | - | - | | - | - | - |
| 2 µm | - | - | - | 1664 | - | - | | - | - | - | - | | - | - | - |

## Example 7: Preparation of Intravenous (IV) and Subcutaneous (SC) Formulations of BAN2401

### A. BAN2401 100 mg/mL Formulations

A BAN2401 100 mg/mL formulation ("100 mg/mL Injection") was manufactured by a conventional cGMP aseptic process for preparation of a sterile aqueous formulation. BAN2401 100 mg/mL was produced from the corresponding BAN2401 drug substances without addition of any excipients and dilution (Table 13).

## Table 13. Exemplary 100 mg/mL IV formulation comprising BAN2401.

| component | composition |
|---|---|
| BAN2401 | 100 mg/mL |
| Citric acid buffer | 50 mM |
| Arginine | 110 mM |
| Arginine Hydrochloride | 240 mM |
| Polysorbate 80 | 0.05 % (w/v) |
| Water for Injection | QS |
| pH | 5.0 |

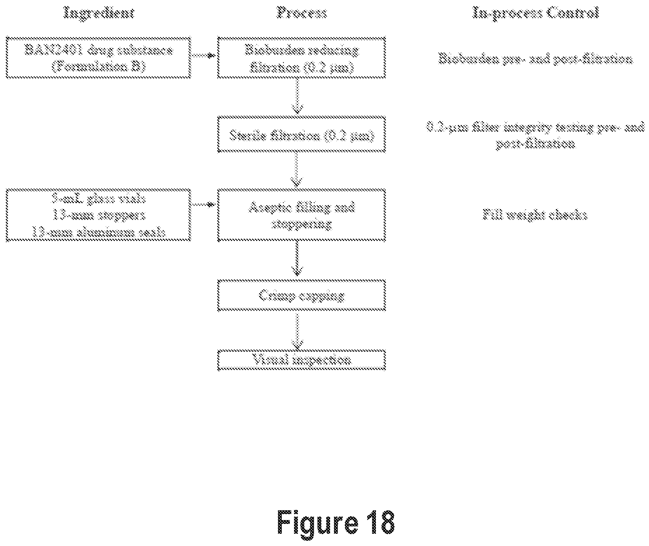

The filtered BAN2401 drug substance (100 mg/mL Injection) solution was aseptically filled into vials as illustrated in Figure 18 (referred to in Figure 18 as "Formulation B"). The drug substance underwent a bioburden reducing filtration step through a 0.2-pm filter. The final sterile filtration was performed through two 0.2-pm filters in series, and pre-and post-filtration filter integrity tests were conducted. The sterile bulk drug product was filled aseptically into vials. During the filling operation, filling accuracy was confirmed by measuring the vial fill weight. Filled vials were stoppered and then sealed with an aluminum overseal. After crimp capping, the product was stored at 5± 3 °C.

The vials were analyzed using Method 1 (Light Obscuration) according to USP 788. Results are shown in Tables 14 and 15.

## Table 14. Particle Size Distribution (particles/vial) for 100 mg/mL Injection Vials

| Lot No. | B1 | B2 | B3 | B4 |
|---|---|---|---|---|

| 25 µm | 0 | 0 | 1 | 0 |
|---|---|---|---|---|
| 10 µm | 53 | 8 | 9 | 9 |
| 5 µm | 631 | 72 | 51 | 60 |
| 2 µm | 1954 | 321 | 249 | 267 |

**Table 15. Particle Size Distribution (particles/mL) for 100 mg/mL Injection Vials**

| Lot No. | B1 | B2 | B3 | B4 |
|---|---|---|---|---|
| 25 µm | 0 | 0 | 0.2 | 0 |
| 10 µm | 10.6 | 1.6 | 1.8 | 1.8 |
| 5 µm | 126.2 | 14.4 | 10.2 | 12 |
| 2 µm | 390.8 | 64.2 | 49.8 | 53.4 |

Another BAN2401 100 mg/mL Injection was manufactured. The following materials can be used in a second exemplary formulation containing 100 mg/mL BAN2401, as shown in Table 16.

**Table 16. Another Exemplary 100 mg/mL IV Formulation comprising BAN2401.**

| component | composition |
|---|---|
| BAN2401 | 100 mg/mL |
| Histidine | Total 25 mM |
| Histidine HCl | |
| Arginine HCl | 200 mM |
| Polysorbate 80 | 0.05%(w/v) |
| Water for infection | QS |
| pH | 5.0 ±0.4 |

**B. BAN2401 200 mg/mL Formulation**

The following materials can be used in an exemplary SC formulation containing 200 mg/mL BAN2401, as shown in Table 17. The stability of BAN2401 in these formulations (FSC1. FSC2 and FSC3) were evaluated in conjunction with an evaluation of the material stability in three container closures:

i) Becton-Dickenson Hypak Biotech glass pre-filled syringe (PFS) and stopper
ii) West Crystal Zenith plastic PFS and stopper
iii) West 5 mL glass vial and stopper

**Table 17. Exemplary 200 mg/mL SC formulations comprising BAN2401.**

| component | composition | | | | |
|---|---|---|---|---|---|
| Sample ID | FSC1a | FSC1b | FSC2 | FSC3 | FSC4 |
| BAN2401 | 200 mg/mL | 200 mg/mL | 200 mg/mL | 200 mg/mL | 200 mg/mL |
| Histidine | Total 50 | Total 50 | Total 50 | -- | Total 25 |
| Histidine HCl | mM [1] | mM [1] | mM [1] | | mM [1] |
| Citric Acid | -- | -- | -- | 50 mM | -- |
| Arginine HCl | 125 mM | 125 mM | 125 mM | 125 mM | 200 mM |
| Polysorbate 80 | 0.02%(w/v) | 0.02%(w/v) | 0.02%(w/v) | 0.02%(w/v) | 0.05%(w/v) |
| Methionine | -- | 5 mM | -- | -- | -- |
| Water for infection | QS | QS | QS | QS | QS |
| pH | 5.0 ±0.4 | 5.0 ±0.4 | 5.5 ±0.4 | 5.0 ±0.4 | 5.0 ±0.4 |

[1] Total concentration as Histidine

BAN2401 at a target protein concentration of 200 mg/mL was prepared via TFF as summarized below. A separate TFF operation was performed to prepare BAN2401 material in each formulation buffer, except for FSC1a and FSC1b. For two of the formulations, one TFF operation was performed, and the resulting concentrated material was split into two half-lots. A small quantity of sterile filtered material in each final formulation buffer was not filled at time zero, but was stored frozen at -20°C to be filled into the appropriate container closures for syringe testing.

**(a) BAN2401 Preparation**

The process of protein concentration/diafiltration via TFF can be subdivided into 3 stages:

1. Concentration of the material to 100-150 mg/mL
2. Diafiltration (5X) against formulation buffer
3. Concentration to >200 mg/mL

The concentration/diafiltration step was performed using a Pall Centramate LV system installed with 0.02 $m^2$ of membrane area. The BAN2401 material (pulled from GMP lot manufacture prior to PS80 addition) was charged into the TFF system and a 10-15 fold concentration (stage 1) was performed. The material was then diafiltered against up to 5 diavolumes of the formulation buffer (stage 2), with pH and conductivity checks of the permeate being done to monitor diafiltration. After diafiltration, the material was further concentrated to the target protein concentration of 210 to 250 mg/mL (stage 3). The retentate was collected and samples were taken for protein concentration determination.

In preparing this formulation, the target protein concentration of 210 to 250 mg/mL was not reached due to high pressure in the TFF system. Therefore, the target protein concentration was achieved by using Millipore centrifi.lgal filter units (30,000 MWCO). To perform this concentration step, filter units were equilibrated with the BAN2401 formulation buffer, followed by centrifugation of the BAN2401 material at 3600 RPM (~3000 $x$ g) for 30 minutes intervals at 20 °C. until the protein concentration in the retentate was expected to be greater than 200 mg/mL. The retentate was recovered from the filter units and pooled. After thorough mixing, the pooled retentate was sampled for protein concentration measurements.

After the protein was concentrated, a sample was taken from the pool and diluted 500-fold with the appropriate formulation buffer. The absorbance of the diluted sample at 280 nm and 320 nm was measured against the buffer blank. The final protein concentration adjustment was performed via dilution with the appropriate formulation buffer. Lastly, 10% PS80 solution was added to the BAN2401 to achieve 0.02% PS80 in the final solution, and the protein solution was thoroughly mixed via end-over-end rotation.

Final BAN2401 formulated material was filtered using 0.2 $\mu$m syringe filters, and subsequently filled into vials or PFS. This step was performed aseptically in a biosafety cabinet. The resulting vials or PFS were placed in a freezer at -20°C. Vials were stored inverted, and PFS were stored horizontally in order to simulate worst case conditions.

**C. Stability Experiments on Exemplary 200 mg/mL SC formulations comprising BAN2401.**

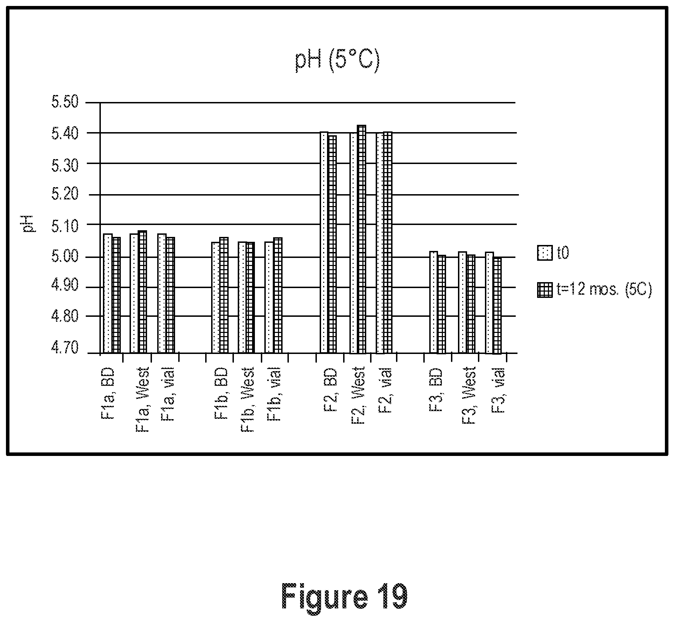

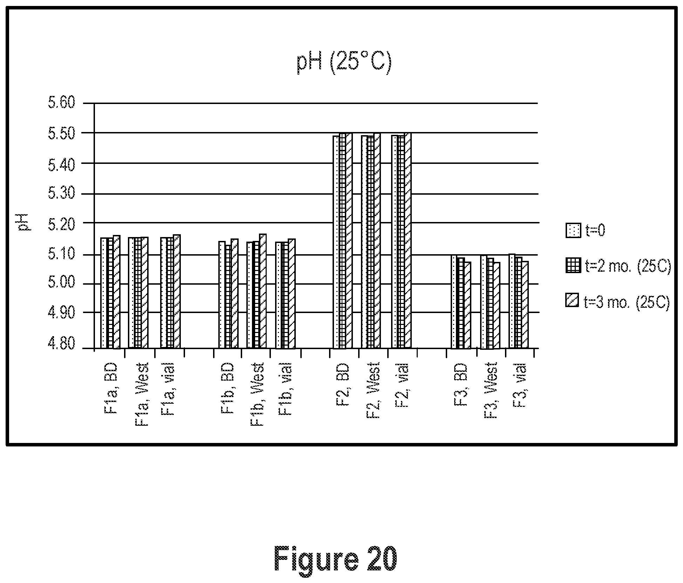

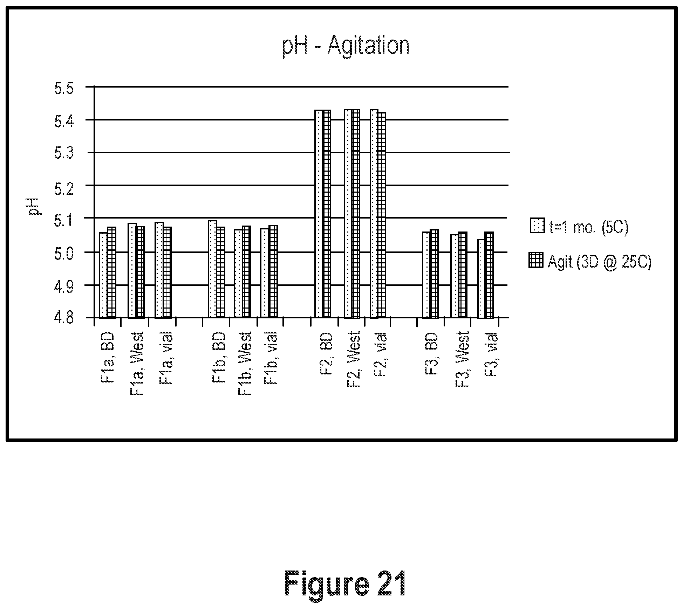

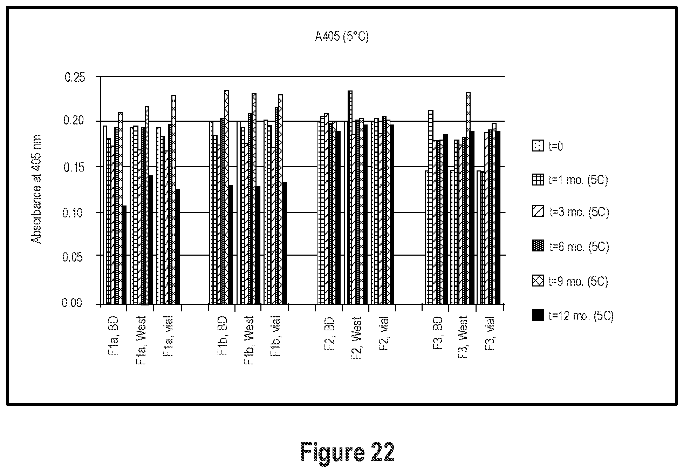

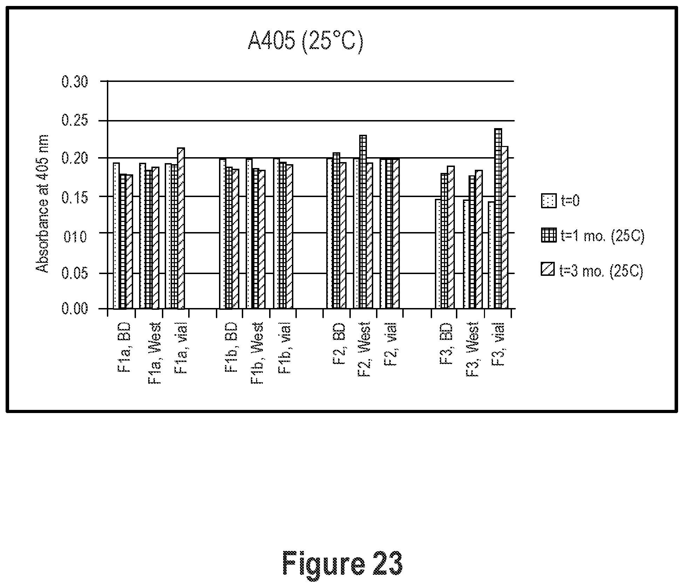

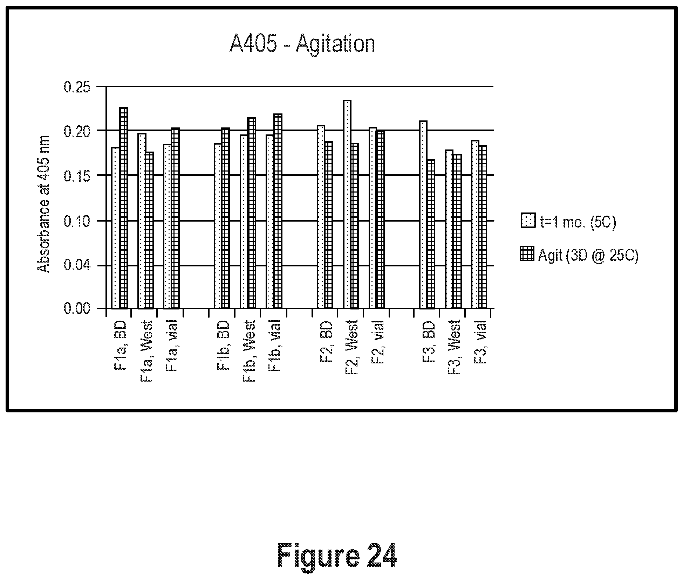

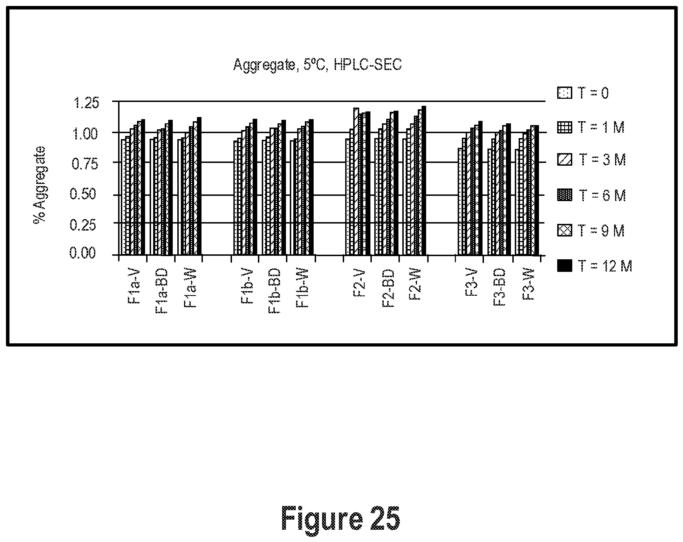

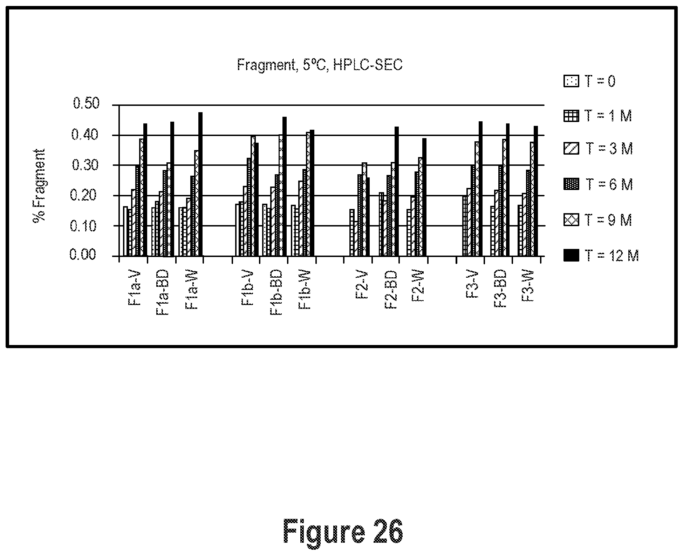

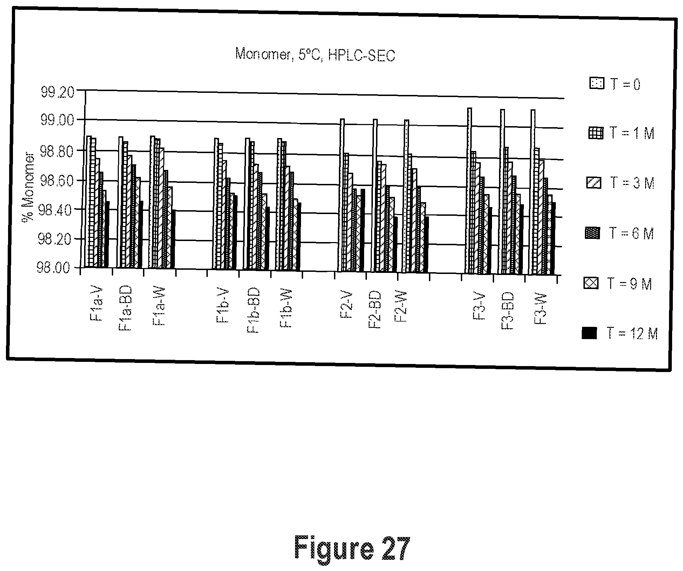

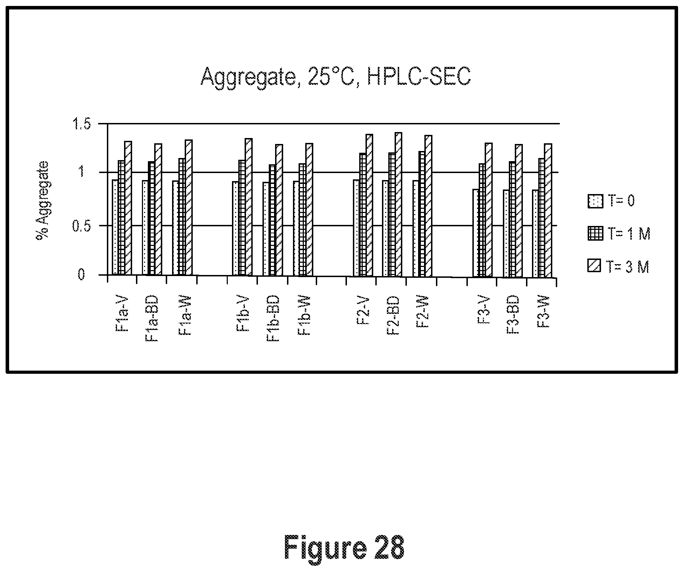

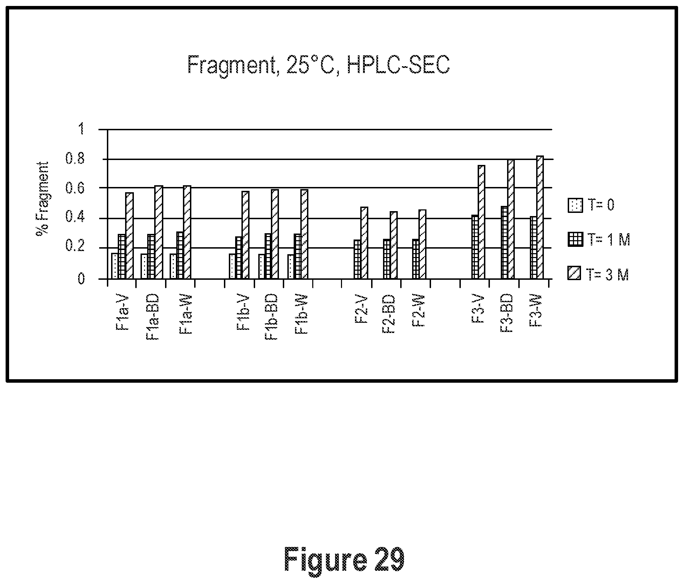

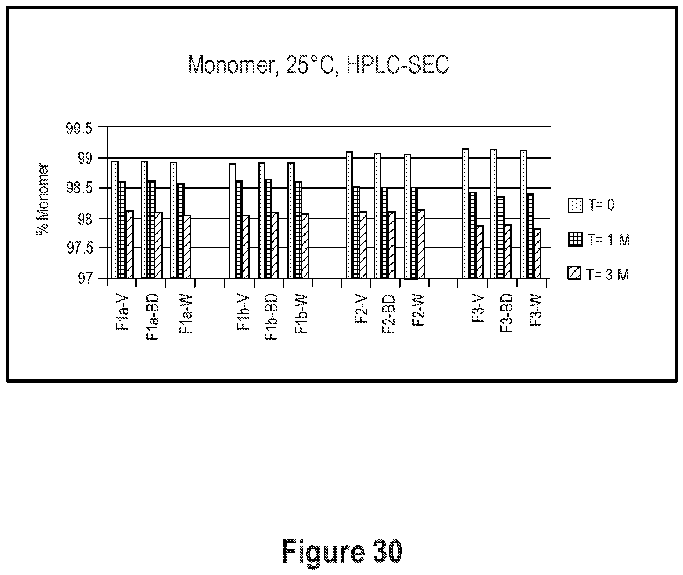

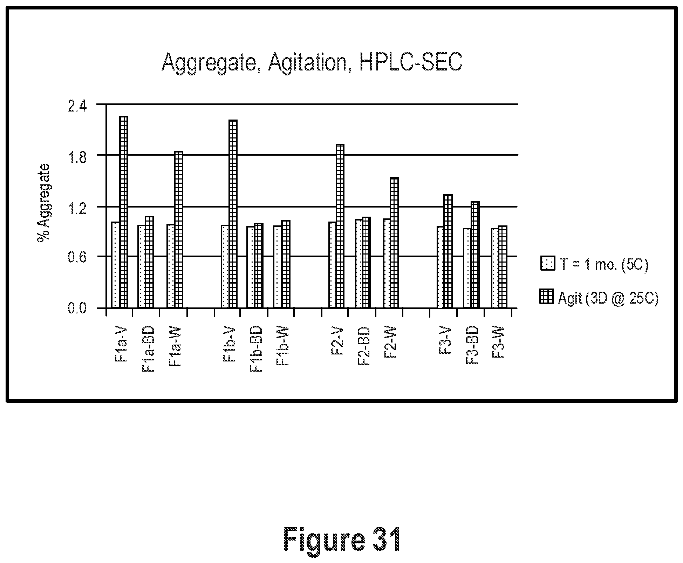

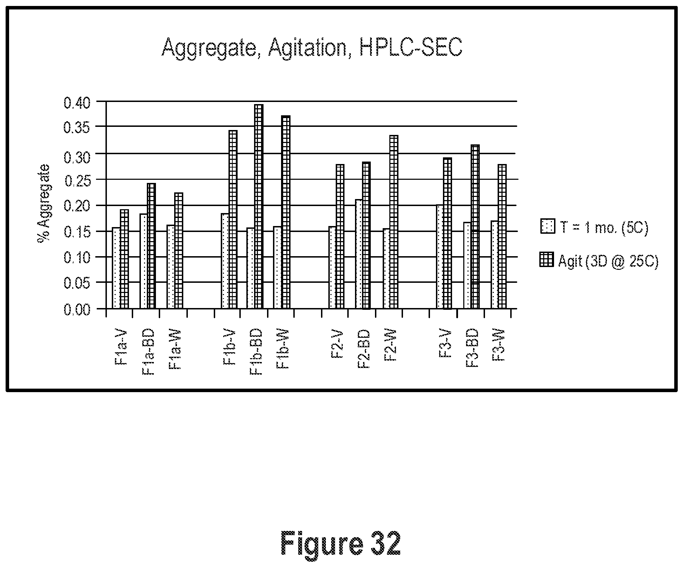

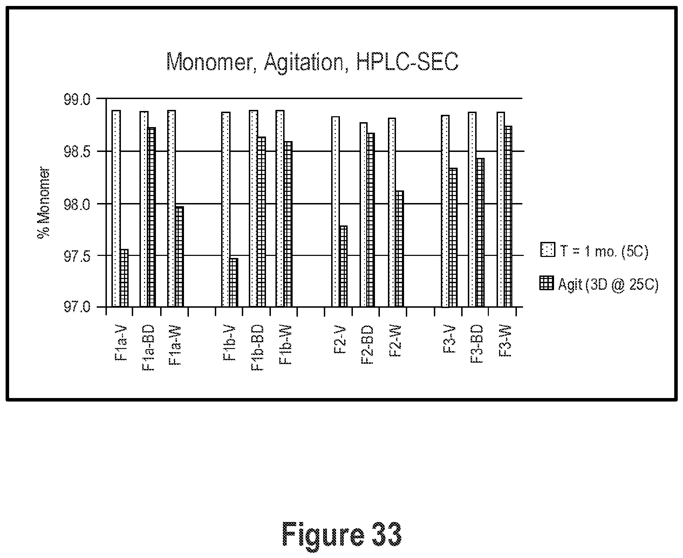

Sample stability was evaluated at 5°C (3M, 6M, 9M, 12M) and 25°C (1M, 3M) using assay for pH (Figures 19-21), absorbance at 405 nm (Figures 22-24) (to detect increases in yellowing), Size-exclusion Chromatography (SEC) (Figures 25-33), and 5°C thereafter. See Table 17 for the exemplary formulations FSC1-FSC4; note that in Figures 19- 33, "F1a" refers to FSC1a, "F1b" refers to FSC1b, "F2" refers to FSC2, and "F3" refers to FSC3. Additional characterization assays, including protein concentration, Differential Scanning Calorimetry (DSC), PS80, and osmolality were performed on t=0 samples. Subvisible particle testing were performed on t=0 and t=6 month samples. A 3-day agitation study was performed on samples stored for 1 month at 5°C. Samples for the agitation study were transferred from 5°C storage after 1 month to 25°C with agitation for 3 days at 250 rpm.

Test results showed that 200 mg/ml BAN2401 in all FSC1a-FSC3 formulations had similar levels of aggregates and fragments after storage at 2-8°C for 12 months. Stability for BAN2401 at 200 mg/mL, in the three FSC1a-FSC3 formulations was evaluated. Overall, the stability of BAN2401 in each tested formulation appeared similar, and maintained greater than 98.2% monomer after storage at 5°C for 12 months regardless of the container closure tested. In addition, after storage at 5°C for 12 months, the pH remained stable, there was no appreciable increase in yellowing by A405.

**(a) Protein Concentration (T=0 Samples)**

Protein concentration was measured using a UV-Vis spectrophotometer. The results are listed in Table 18.

### Table 18. Protein Concentration Results

| Sample ID | Protein Concentration (mg/ml) |
|-----------|-------------------------------|
| FSC1a | 206 |
| FSC1b | 204 |
| FSC2 | 201 |
| FSC3 | 189 |

Protein concentration was evaluated by measuring the absorbance at 280 and 320 nm on a Beckman DU-800 spectrophotometer using a 1cm quartz cuvette. Samples were diluted 500-fold and were prepared in triplicate. Protein concentration was calculated using an extinction coefficient, $\varepsilon$. of 1.32 using the following formula:

$$\text{Concentration} = (A280\text{-}A320)/ \varepsilon \ \text{x} \ \text{Dilution Factor}$$

**(b) Polysorbate 80 (T=0 Samples)**

The PS80 content of samples was measured via quantitation of oleic acid. The results are shown in Table 19.

### Table 19. Polysorbate 80 Results

| Sample ID | Polysorbate 80 (%) |
|-----------|--------------------|
| FSC1a | 0.016 |
| FSC1b | 0.022 |
| FSC2 | 0.019 |
| FSC3 | 0.019 |

The measurement was performed by quantitation of oleic acid, a hydrolysis product of PS80. Using base hydrolysis, PS80 releases oleic acid at a 1:1 molar ratio. The oleic acid can then be separated from other PS80 hydrolysis products and matrices using reversed phase HPLC. The oleic acid was monitored without derivatization using the absorbance at 195 nm. [J. Chromatography B, 878 (2010) 1865-1870]. Experimentally, samples were mixed with sodium hydroxide to release oleic acid, which was subsequently extracted with acetonitrile. The extract was diluted with a potassium phosphate solution, and a sample volume of 100 $\mu$L was injected into a Waters Symmetry C18 column. The separation was done using an isocratic elution containing 80% organic phase A (acetonitrile) and 20% aqueous phase B (20 mM Potassium Phosphate Monobasic, pH 2.8). The PS80 concentration in the sample was calculated from the peak area using a standard curve.

**(c) Osmolality (T=0 Samples)**

The osmolality of samples was determined using a freezing point osmometer. The results are shown in Table 20.

### Table 20. Osmolality Results

| Sample ID | Osmolality (mOsmol/kg $H_2O$) |
|---|---|
| FSC1a | 294 |
| FSC1b | 309 |
| FSC2 | 297 |
| FSC3 | 327 |

Osmolality was measured using a Precision Systems Osmette III freezing point osmometer. Samples were diluted 3-fold with WFI, and diluted sample volumes of 10 $\mu$L were withdrawn and loaded to the instrument using the osmometer pipette. The resulting osmolality measurements were corrected for sample dilution.

**(d) Subvisible Particles**

Subvisible particle analysis was performed using a Fluid Technologies FlowCarn instrument. The T=O results are listed in Table 21. There appears to be an increase in the total particle concentration and number of particles greater than 10$\mu$m over time when material is stored at 5°C. The degree of the increased change was small in the following order, FSC I a < FSC1b < FSC2 < FSC3.

## Table 21. Subvisible Particle Results

| Sample ID | Particles/ mL Total | Particles/ mL $\geq 10\mu m$ | Particles/ mL $\geq 25\mu m$ |
|---|---|---|---|
| FSC1a | $1.2 \times 10^4$ | 9 | 0 |
| FSC1b | $2.6 \times 10^4$ | 0 | 0 |
| FSC2 | $2.8 \times 10^4$ | 18 | 0 |
| FSC3 | $3.9 \times 10^4$ | 63 | 0 |

Subvisible particle analysis was performed using a Fluid Imaging Technologies FlowCam with a 20X objective and a 50 um flow cell. Before running BAN2401 samples, the flow cell was flushed with dI $H_2O$ and a measurement was performed on the dI $H_2O$ to ensure the flow cell was clean. If the total number of panicles counted per 0.2 mL of di $H_2O$ was $\leqq 2$, the flow cell was considered ready for use. Samples were equilibrated to room temperature, then diluted 20-fold with deionized water. Duplicate diluted samples were analyzed using a sample volume of 0.2 mL and a sample flowrate of 0.02 mL/min. The autoimage rate was 14, giving an efficiency of 19.6% and a run time of 10 min. The resulting paiiicle concentrations were corrected for sample dilution.

### (e) pH

The pH of each formulation was monitored throughout the stability testing. As shown in Figures 19-21, no apparent change was observed for the samples in any of the storage conditions tested.

### (f) HPLC-SEC

High Performance Liquid Chromatography Size Exclusion Chromatography (HPLC-SEC) analysis was performed utilizing a TSK 03000 SWXL column with 0.2 M sodium phosphate, pH 7.0 mobile phase at a flow rate of 1.0 mL/min. The sample injection volume was 0.8 $\mu L$ for a protein concentration of 200 mg/mL. This ensures approximately 150 $\mu g$ of protein is injected onto the column across the study. Results of relative peak areas (%) for monomer, aggregate and fragment were reported, as shown in Figures 25-33.

Physical degradation of the BAN2401 was assessed by performing HPLC-SEC on the stability samples stored at 5 and 25 °C. After storage at 5°C for 12 months, the percent of aggregate was similar for the 3 formulations at approximately 1.1%. The percent fragment generated for all tested formulations was in the range of 0.4-0.5%. The monomer content after 12 months storage at 5°C was in the range of 98.4-98.6% for all tested formulations and container closures. At the current rates of degradation, it is possible that BAN2401 in all tested formulations could have greater than 97% monomer after up to 24 months storage at 5°C.

After storage at 25°C up to 3 months, fragment generation after 3 months was in the range of 0.4-0.8%. Monomer content was slightly greater than 98% for all formulations, except for FSC3, which had slightly less than 98% monomer after 3 months at 25°C.

### Example 8: Selection of Protein, Arginine and Polysorbate 80 Concentrations

### A. Sample Preparation

Each candidate formulation (F1-F12) was prepared as follows. Drug substance (DS) process intermediate-was concentrated and equilibrated with the corresponding formulation buffers (Table 22) by centrifugal filter units. After the concentration and equilibration, PS80 dissolved in the formulation buffer was added to achieve pre-determined concentrations, and protein concentrations were adjusted to the final concentration. Each candidate formulation was filled into vials with fill volume of 0.5 mL. Candidate formulations (F1-F12) are shown in Table 22. Formulation F0 was evaluated as a control.

**Table 22  Formulation (F0) and candidate formulations F1–F12**

| Form No. | BAN2401 | Buffer | | Stabilizer (Arginine) | | Polysorbate 80 | | pH | Fill volume |
|---|---|---|---|---|---|---|---|---|---|
| | mg/mL | mmol/L | mg/mL | mmol/L | mg/mL | (w/v)% | mg/mL | | mL |
| F0 | 100 | Citrate 50 | 9.6 | 350 | 61.0 | 0.05 | 0.5 | 5.0 | 5/0.5[b] |
| F1 | 200 | Histidine 25 | 3.9 | 150 | 26.1 | 0.05 | 0.5 | 5.0 | 0.5 |
| F2[a] | 200 | Histidine 25 | 3.9 | 200 | 34.8 | 0.05 | 0.5 | 5.0 | 0.5 |
| F3 | 200 | Histidine 25 | 3.9 | 250 | 43.6 | 0.05 | 0.5 | 5.0 | 0.5 |
| F4 | 200 | Histidine 25 | 3.9 | 300 | 52.3 | 0.05 | 0.5 | 5.0 | 0.5 |
| F5 | 200 | Histidine 25 | 3.9 | 350 | 61.0 | 0.05 | 0.5 | 5.0 | 0.5 |
| F6 | 250 | Histidine 25 | 3.9 | 200 | 34.8 | 0.05 | 0.5 | 5.0 | 0.5 |
| F7 | 300 | Histidine 25 | 3.9 | 200 | 34.8 | 0.05 | 0.5 | 5.0 | 0.5 |
| F8 | 200 | Histidine 25 | 3.9 | 200 | 34.8 | 0 | 0 | 5.0 | 0.5 |
| F9 | 200 | Histidine 25 | 3.9 | 200 | 34.8 | 0.02 | 0.2 | 5.0 | 0.5 |
| F10[a] | 200 | Histidine 25 | 3.9 | 200 | 34.8 | 0.05 | 0.5 | 5.0 | 0.5 |
| F11 | 200 | Histidine 25 | 3.9 | 200 | 34.8 | 0.08 | 0.8 | 5.0 | 0.5 |
| F12 | 200 | Histidine 25 | 3.9 | 200 | 34.8 | 0.10 | 1.0 | 5.0 | 0.5 |

a: F2 and F10 are the same formulations, prepared for different studies.
b: In the selection of polysorbate 80 concentration, drug solution was withdrawn from drug product and was filled with 0.5 mL to align the fill volume and the container closure system.

**B. Stability protocol**

Stability protocols are shown in Table 23, Table 24, and Table 25. Once removed from storage, the vials were stored at 5 °C until testing. Storage conditions were as follows:

- Long-term condition: stored at 5 °C±3°C upright
- Accelerated condition: Stored at 25 °C±2 °C, 60%RH±5%RH upright
- Stressed condition (Freeze-thaw): Frozen at -30°C and thawed at room temperature, upright
- Stressed condition (Agitation): 5 °C, laying down, horizontal, at 250 rpm on a reciprocating shaker with shaking amplitude of 50 mm

**Table 23  Stability Protocol (Long-term and Accelerated Condition)**

| Test item | Test Point | | | |
|---|---|---|---|---|
| | Initial | 5 °C±3°C Upright | 25 °C±2°C, 60%RH±5%RH Upright | |
| | | 3M | 1M | 3M |
| Appearance | F0-F7 | F0-F7 | F0-F7 | F0-F7 |
| pH | F0-F7 | F0-F7 | F0-F7 | F0-F7 |
| Size exclusion HPLC | F0-F7 | F0-F7 | F0-F7 | F0-F7 |
| Ion exchange HPLC | F0-F7 | F0-F7 | N/A | F0-F7 |
| Polysorbate 80 | F0-F7 | F0-F7 | F0-F7 | F0-F7 |

N/A: Not applicable.

**Table 24 Stability Protocol (Stressed Condition: F0-F7)**

| Test item | Test Point | |
|---|---|---|
| | Initial | Freeze-thaw (~30°C ⇔ room temperature) |
| | | 3 cycles |
| Appearance | F0-F7 | F6-F7 |
| pH | F0-F7 | F0-F7 |
| Size exclusion HPLC | F0-F7 | F0-F7 |
| Ion exchange HPLC | F0-F7 | F0-F7 |
| Polysorbate 80 | F0-F7 | N/A |
| Protein concentration | F0-F7 | N/A |
| Viscosity | F0-F7 | N/A |
| Osmolality | F0-F7 | N/A |

N/A: Not applicable.

**Table 25 Stability Protocol (Stressed Condition: F0, F8-F12)**

| Test item | Test Point | | |
|---|---|---|---|
| | Initial | Freeze-thaw (~30°C ⇔ room temperature) | Agitation (5 °C, lay down, horizontal, 250 rpm) |
| | | 3 cycles | 3 days |
| Appearance | F0, F8-F12 | F0, F8-F12 | F0, F8-F12 |
| pH | F0, F8-F12 | F0, F8-F12 | F0, F8-F12 |
| Protein concentration | F0, F8-F12 | F0, F8-F12 | F0, F8-F12 |
| Foreign insoluble matter (Visible particles) | F0, F8-F12 | F0, F8-F12 | F0, F8-F12 |
| Size exclusion HPLC (SEC) | F0, F8-F12 | F0, F8-F12 | F0, F8-F12 |
| Polysorbate 80 (PS80) | F0, F9-F12 | F0, F9-F12 | F0, F9-F12 |
| Micro-flow imaging (MFI) | F0, F8-F12 | F0, F8-F12 | F0, F8-F12 |

N/A: Not applicable.

Since the viscosity of solution is known to increase exponentially at highly concentrated protein solutions, protein concentration of each candidate formulation (F1-F5) were adjusted to 200 ± 10 mg/mL with formulation buffer containing 0.05 (w/v)% of PS80 for the analysis. Other formulations were not diluted before the measurement.

**C. Results and Discussion**

**(a) Selection of arginine concentration**

To select the target arginine concentration in the formulation, physical properties were evaluated and freeze-thaw, long-term, and accelerated stability studies were conducted for candidate formulations with different arginine concentration (150 to 350 mmol/L, F1 to F5) and compared to formulation F0 (see, Table 22). Physical properties and the results of freeze-thaw study were shown in Table 26. The results of long-term and accelerated stability study were shown in Table 27 and Table 28.

**Table 26: Results of Physical Properties and Freeze-thaw Stability Study for BAN2401 Drug Product (F0-F5)**

| Test item | Acceptance Criterion | F0 (Control) | | F1 Arg 150mM | | F2 Arg 200mM | | F3 Arg 250mM | | F4 Arg 300mM | | F5 Arg 350mM | |
|---|---|---|---|---|---|---|---|---|---|---|---|---|---|
| | | Initial | Freeze-thaw (3 cycle) | Initial | Freeze-thaw (3 cycle) | Initial | Freeze-thaw (3 cycle) | Initial | Freeze-thaw (3 cycle) | Initial | Freeze-thaw (3 cycle) | Initial | Freeze-thaw (3 cycle) |
| Appearance | Report result | CSYL | NS | CSYL | NS | CSYL | NS | CSYL | NS | CSYL | NS | CSYL | NS |
| pH | Report result | 5.00 | 5.01 | 4.89 | 4.90 | 4.90 | 4.90 | 4.90 | 4.91 | 4.90 | 4.90 | 4.93 | 4.92 |
| Size exclusion HPLC | Report result | | | | | | | | | | | | |
| | Monomer | 98.8% | 98.8% | 98.7% | 98.7% | 98.7% | 98.7% | 98.8% | 98.7% | 98.8% | 98.8% | 98.8% | 98.8% |
| | Aggregate | 0.9% | 0.9% | 1.0% | 1.0% | 1.0% | 1.0% | 1.0% | 1.0% | 1.0% | 1.0% | 1.0% | 1.0% |
| | Fragment | 0.3% | 0.3% | 0.3% | 0.3% | 0.3% | 0.3% | 0.3% | 0.3% | 0.3% | 0.3% | 0.2% | 0.2% |
| Ion exchange HPLC | Report result | | | | | | | | | | | | |
| | Main peak | 67.2% | 67.2% | 67.1% | 70.6% | 70.4% | 70.7% | 67.6% | 67.9% | 70.1% | 67.3% | 70.7% | 70.5% |
| | Acidic peak | 27.3% | 27.1% | 24.5% | 23.3% | 23.2% | 23.1% | 24.1% | 24.2% | 23.5% | 24.6% | 23.1% | 23.5% |
| | Basic peak | 5.4% | 5.7% | 8.4% | 6.2% | 6.4% | 6.2% | 8.3% | 7.9% | 6.3% | 8.1% | 6.3% | 6.1% |
| Polysorbate 80 | Report result (w/v%) | 0.053% | N/A | 0.054% | N/A | 0.052% | N/A | 0.050% | N/A | 0.037% | N/A | 0.052% | N/A |
| Protein concentration | Report result (mg/mL) | 103 | N/A | 224 | N/A | 222 | N/A | 213 | N/A | 207 | N/A | 217 | N/A |
| Viscosity[a] | Report result (cP) | 2.6 | N/A | 7.3 | N/A | 7.8 | N/A | 8.0 | N/A | 8.1 | N/A | 8.0 | N/A |
| | Protein conc. (mg/mL) | N/A | N/A | 204 | N/A | 210 | N/A | 203 | N/A | 200 | N/A | 199 | N/A |
| Osmolality | Report result (mOsm/kg) | 506 | N/A | 367 | N/A | 455 | N/A | 533 | N/A | 615 | N/A | 691 | N/A |

mM: mmol/L, CSYL: Clear slightly yellow liquid, Arg: Arginine, NS: Not scheduled.
a: Protein concentration was adjusted before viscosity measurement (F1-F5).

EP 4 121 007 B1

## Table 27 Results of Stability Study for BAN2401 Drug Product (F0–F7) Stored at 5±3 °C, Upright Position

| Test item | Acceptance Criterion | F0 Initial | F0 3M | F1 Initial | F1 3M | F2 Initial | F2 3M | F3 Initial | F3 3M | F4 Initial | F4 3M | F5 Initial | F5 3M | F6 Initial | F6 3M | F7 Initial | F7 3M |
|---|---|---|---|---|---|---|---|---|---|---|---|---|---|---|---|---|---|
| Appearance | Report result | CSYL | CSYL | CSYL | CSYL | CSYL | CSYL | CSYL | CSYL | CSYL | CSYL | CSYL | CSYL | CSYL | CSYL | CSYL | CSYL |
| pH | Report result | 5.00 | 4.95 | 4.89 | 4.85 | 4.90 | 4.85 | 4.90 | 4.85 | 4.90 | 4.85 | 4.93 | 4.86 | 4.88 | 4.82 | 4.85 | 4.81 |
| Size exclusion HPLC | Report result | | | | | | | | | | | | | | | | |
| | Monomer | 98.8% | 98.6% | 98.7% | 98.4% | 98.7% | 98.4% | 98.8% | 98.4% | 98.8% | 98.4% | 98.8% | 98.4% | 98.5% | 98.3% | 98.5% | 98.2% |
| | Aggregate | 0.9% | 1.0% | 1.0% | 1.2% | 1.0% | 1.2% | 1.0% | 1.2% | 1.0% | 1.2% | 1.0% | 1.2% | 1.1% | 1.3% | 1.2% | 1.3% |
| | Fragment | 0.3% | 0.4% | 0.3% | 0.4% | 0.3% | 0.4% | 0.3% | 0.4% | 0.3% | 0.4% | 0.2% | 0.4% | 0.4% | 0.4% | 0.4% | 0.4% |
| Ion exchange HPLC | Report result | | | | | | | | | | | | | | | | |
| | Main peak | 67.2% | 66.0% | 67.1% | 69.0% | 70.4% | 69.4% | 67.6% | 69.0% | 70.1% | 69.9% | 70.7% | 71.0% | 71.1% | 69.5% | 71.2% | 70.1% |
| | Acidic peak | 27.3% | 26.7% | 24.5% | 23.6% | 23.2% | 23.9% | 24.1% | 24.2% | 23.5% | 23.2% | 23.1% | 22.5% | 22.7% | 23.6% | 22.8% | 23.1% |
| | Basic peak | 5.4% | 7.2% | 8.4% | 7.4% | 6.4% | 6.7% | 8.3% | 6.9% | 6.3% | 6.8% | 6.3% | 6.5% | 6.2% | 6.8% | 6.0% | 6.8% |
| Polysorbate 80 | Report result (w/v%) | 0.053% | 0.056% | 0.054% | 0.057% | 0.052% | 0.055% | 0.050% | 0.054% | 0.037% | 0.055% | 0.052% | 0.052% | 0.056% | 0.055% | 0.051% | 0.052% |

CSYL: Clear slightly yellow liquid.

EP 4 121 007 B1

# Table 28 Results of Stability Study for BAN2401 Drug Product (F0–F7) Stored at 25±2 °C, 60%±5%RH, Upright Position

| Test item | Acceptance Criterion | F0 | | | F1 | | | F2 | | | F3 | | | F4 | | | F5 | | | F6 | | | F7 | | |
|---|---|---|---|---|---|---|---|---|---|---|---|---|---|---|---|---|---|---|---|---|---|---|---|---|---|
| | | Ini | 1M | 3M | Ini | 1M | 3M | Ini | 1M | 3M | Ini | 1M | 3M | Ini | 1M | 3M | Ini | 1M | 3M | Ini | 1M | 3M | Ini | 1M | 3M |
| Appearance | Report result | CSYL | CSYL | CSYL | CSYL | CSYL | CSYL | CSYL | CSYL | CSYL | CSYL | CSYL | CSYL | CSYL | CSYL | CSYL | CSYL | CSYL | CSYL | CSYL | CSYL | CSYL | CSYL | CSYL | CSYL |
| pH | Report result | 5.00 | 4.97 | 4.96 | 4.89 | 4.86 | 4.85 | 4.90 | 4.85 | 4.84 | 4.90 | 4.86 | 4.85 | 4.90 | 4.86 | 4.85 | 4.93 | 4.87 | 4.86 | 4.88 | 4.84 | 4.82 | 4.85 | 4.83 | 4.81 |
| SEC | Report result Monomer | 98.8% | 98.6% | 98.1% | 98.7% | 98.2% | 97.5% | 98.7% | 98.2% | 97.5% | 98.8% | 98.2% | 97.6% | 98.8% | 98.2% | 97.6% | 98.8% | 98.3% | 97.6% | 98.5% | 98.1% | 97.3% | 98.5% | 98.0% | 97.1% |
| | Aggregate | 0.9% | 0.9% | 1.0% | 1.0% | 1.4% | 1.7% | 1.0% | 1.4% | 1.7% | 1.0% | 1.3% | 1.6% | 1.0% | 1.3% | 1.6% | 1.0% | 1.3% | 1.6% | 1.1% | 1.4% | 1.9% | 1.2% | 1.5% | 2.0% |
| | Fragment | 0.3% | 0.5% | 0.8% | 0.3% | 0.5% | 0.8% | 0.3% | 0.5% | 0.8% | 0.3% | 0.5% | 0.8% | 0.3% | 0.5% | 0.8% | 0.2% | 0.5% | 0.8% | 0.4% | 0.5% | 0.8% | 0.4% | 0.5% | 0.9% |
| IEX | Report result Main | 67.2% | NS | 59.6% | 67.1% | NS | 63.0% | 70.4% | NS | 63.1% | 67.6% | NS | 63.1% | 70.1% | NS | 63.3% | 70.7% | NS | 63.9% | 71.1% | NS | 63.0% | 71.2% | NS | 63.1% |
| | Acidic | 27.3% | NS | 33.8% | 24.5% | NS | 29.4% | 23.2% | NS | 29.6% | 24.1% | NS | 29.1% | 23.5% | NS | 29.1% | 23.1% | NS | 28.3% | 22.7% | NS | 29.5% | 22.8% | NS | 28.8% |
| | Basic | 5.4% | NS | 6.6% | 8.4% | NS | 7.6% | 6.4% | NS | 7.4% | 8.3% | NS | 7.8% | 6.3% | NS | 7.5% | 6.3% | NS | 7.8% | 6.2% | NS | 7.5% | 6.0% | NS | 8.1% |
| PS80 | Report result (w/v%) | 0.053% | 0.053% | 0.052% | 0.054% | 0.052% | 0.052% | 0.052% | 0.050% | 0.050% | 0.050% | 0.050% | 0.049% | 0.037% | 0.051% | 0.052% | 0.052% | 0.049% | 0.049% | 0.056% | 0.053% | 0.053% | 0.051% | 0.050% | 0.049% |

CSYL: Clear slightly yellow liquid. Ini: Initial. SEC: Size exclusion HPLC. IEX: Ion exchange HPLC. PS80: Polysorbate 80. NS: Not scheduled.

EP 4 121 007 B1

30

**(b) Physical Properties**

As shown in Table 26, osmolality values increased as the arginine concentration increased. F1, F2 and F3 showed lower osmolality values as compared to F4 and F5. Therefore, arginine concentration was limited to 250 mmol/L or less. Viscosity values of F1-F5 were within a narrow range (7.3-8.1 cP) and were not correlated with arginine concentration within 150 to 350 mmol/L. The results of appearance, pH and concentration of PS80 and protein were almost at the target.

**(c) Freeze-Thaw Stability Study**

No significant changes were observed in all testing items after three cycles of freeze-thaw testing as shown in Table 26.

**(d) Long-Term Stability Study**

As shown in Table 27, no significant changes were observed in all testing items except for size exclusion HPLC (SEC) after three-month storage. Amounts of aggregate and fragment by SEC slightly increased in candidate formulations F1-F5. Each increase rate was similar to the formulation F0.

**(e) Accelerated Stability Study**

As shown in Table 28, no significant changes were observed in all testing items except for SEC and ion exclusion HPLC (IEX). Although the amount of aggregate by SEC increased in all the candidates (F1-F5, BAN2401 200 mg/mL) and the rate was faster than formulation F0 as expected, all the candidates were considered to be feasible considering the results in long-term condition and the stability of F0 with a long shelf-life. The higher arginine concentration resulted in slightly slower aggregate formation, consistent with the results described in Example 4. The amount of fragment by SEC also increased in all the candidates but the rate was similar to that in the formulation F0. As for IEX, the amount of acidic peak increased in all the candidates but the rate was similar to that in F0.

**(f) Arginine Concentration**

As shown in Table 26, F4 and F5 (at arginine concentrations of 300 and 350 mmol/L) were not considered feasible in the view of osmolality. Among feasible candidates F1, F2 and F3 (at arginine concentrations of 150, 200 and 250 mmol/L), F1 is the closest to isotonic. As shown in Table 28, formulations with higher arginine concentrations showed lower aggregate formation rate in accelerated stability studies but the difference in rate was not significant at the arginine concentration range evaluated. Taking both isotonicity and aggregate formation rate into consideration, 200 mmol/L was selected as the target arginine concentration based on the F2 formulation.

**(g) Selection of protein concentration**

To select the target protein concentration in the formulation, physical properties were evaluated and freeze-thaw, long-term, and accelerated stability studies were conducted for candidate formulations with different protein concentration (200 to 300 mg/mL, F2, F6, F7) and formulation F0. Physical properties and the results of freeze-thaw study are shown in Table 29. The results of long-term and accelerated stability studies were shown in Table 27 and Table 28.

**Table 29 Results of Physical Properties and Freeze-thaw Stability Study for BAN2401 Drug Product (F0, F6 and F7)**

| Test item | Acceptance Criterion | F0[a] | (Control) | F2[a] | BAN2401 200 mg/mL | F6 | BAN2401 250 mg/mL | F7 | BAN2401 300 mg/mL |
|---|---|---|---|---|---|---|---|---|---|
| | | Initial | Freeze-thaw (3 cycle) | Initial | Freeze-thaw (3 cycle) | Initial | Freeze-thaw (3 cycle) | Initial | Freeze-thaw (3 cycle) |
| Appearance | Report result | CSYL | NS | CSYL | NS | CSYL | CSYL | CSYL | CSYL |
| pH | Report result | 5.00 | 5.01 | 4.90 | 4.90 | 4.88 | 4.83 | 4.85 | 4.86 |
| Size exclusion HPLC | Report result | | | | | | | | |
| | Monomer | 98.8% | 98.8% | 98.7% | 98.7% | 98.5% | 98.5% | 98.5% | 98.4% |
| | Aggregate | 0.9% | 0.9% | 1.0% | 1.0% | 1.1% | 1.1% | 1.2% | 1.2% |
| | Fragment | 0.3% | 0.3% | 0.3% | 0.3% | 0.4% | 0.4% | 0.4% | 0.4% |
| Ion exchange HPLC | Report result | | | | | | | | |
| | Main peak | 67.2% | 67.2% | 70.4% | 70.7% | 71.1% | 71.2% | 71.2% | 71.4% |
| | Acidic peak | 27.3% | 27.1% | 23.2% | 23.1% | 22.7% | 22.6% | 22.8% | 22.5% |
| | Basic peak | 5.4% | 5.7% | 6.4% | 6.2% | 6.2% | 6.2% | 6.0% | 6.1% |
| Polysorbate 80 | Report result (w/v(%)) | 0.053% | NS | 0.052% | NS | 0.056% | NS | 0.051% | NS |
| Protein concentration | Report result (mg/mL) | 103 | NS | 222 | NS | 242 | NS | 299 | NS |
| Viscosity | Report result (cP) | 2.6 | NS | 7.8 (210 mg/mL) | NS | 21 | NS | 48 | NS |
| Osmolality | Report result (mOsm/kg) | 506 | NS | 455 | NS | 507 | NS | 554 | NS |

CSYL: Clear slightly yellow liquid, NS: Not scheduled.
a: Results are the same in Table 26

EP 4 121 007 B1

**(h) Physical Properties**

As shown in Table 29, F2 and F6 showed lower osmolality values as compared to F7. Viscosity values in F2, F6 and F7 were 7.8, 21 and 48 cP, respectively. High viscosity over 20 cP would cause difficulties in manufacturing DS by increasing the back-pressure of the pump and decreasing the transmembrane flux during ultrafiltration and diafiltration steps. In addition, higher concentration solution makes DP filling process more difficult by clogging of filling needles that lead to fill weight variation. Since the desired viscosity is reported to be not more than 20 cP[3], F2 was feasible. The results of appearance, pH and concentration of PS80 and protein were almost on target.

**(i) Freeze-Thaw Study**

No significant changes were observed in all the tested items after three cycles of freeze-thaw testing as shown in Table 29.

**(j) Long-Term Stability Study**

As shown in Table 27, no significant changes were observed other than size exclusion HPLC (SEC) after three-month storage. Amount of aggregate by SEC slightly increased in candidate formulations F2, F6 and F7 but increase rate was similar to that in F0.

**(k) Accelerated Stability Study**

As shown in Table 28, no significant changes were observed other than SEC and ion exclusion HPLC (IEX). The amount of aggregate by SEC increased in all the candidates (F2, F6 and F7) in similar rates at three-month timepoint (0.7-0.8% increase). Therefore, all the candidates were considered feasible. The amount of fragment by SEC also increased in all the candidates but the rate was similar to that in F0. As for IEX, the amount of acidic peak by IEX increased in all the candidates but the rate was similar to that in F0.

**(l) Protein Concentration**

Considering the results of osmolality and viscosity shown in Table 29, and the overall stability results shown in Table 27 and Table 28, protein concentration of 200 mg/mL was selected.

**(m) Selection of PS80 concentration**

To select the target PS80 concentration in the formulation, freeze-thaw and agitation studies were conducted for candidate formulations with different PS80 concentration [0 to 0.10 (w/v)%, F8-F12] and formulation F0. The results of freeze-thaw and agitation study are shown in Table 30 and Table 31, respectively.

**Table 30 Results of Freeze-thaw Study for BAN2401 Drug Product (F0, F8-F12)**

| Test item | Acceptance Criterion | F0 (Control) | | F8 PS80 0 w/v% | | F9 PS80 0.02 w/v % | | F10 PS80 0.05 w/v % | | F11 PS80 0.08 w/v % | | F12 PS80 0.10 w/v % | |
|---|---|---|---|---|---|---|---|---|---|---|---|---|---|
| | | Initial | Freeze-thaw (3 cycle) | Initial | Freeze-thaw (3 cycle) | Initial | Freeze-thaw (3 cycle) | Initial | Freeze-thaw (3 cycle) | Initial | Freeze-thaw (3 cycle) | Initial | Freeze-thaw (3 cycle) |
| Appearance | Report result | CSYL | CSYL | CSYL | CSYL | CSYL | CSYL | CSYL | CSYL | CSYL | CSYL | CSYL | CSYL |
| pH | Report result | 4.98 | 4.98 | 4.85 | 4.86 | 4.85 | 4.86 | 4.85 | 4.86 | 4.85 | 4.86 | 4.85 | 4.85 |
| Visible particles | Report result | FVP | FVP | FVP | FVP | FVP | FVP | FVP | FVP | FVP | FVP | FVP | FVP |
| Size exclusion HPLC | Report result | | | | | | | | | | | | |
| | Monomer | 98.3% | 98.4% | 98.0% | 98.0% | 98.0% | 97.9% | 98.0% | 98.0% | 98.0% | 98.0% | 98.0% | 98.0% |
| | Aggregate | 1.0% | 1.0% | 1.1% | 1.1% | 1.1% | 1.1% | 1.1% | 1.1% | 1.1% | 1.1% | 1.1% | 1.1% |
| | Fragment | 0.7% | 0.7% | 0.9% | 0.9% | 0.9% | 1.1% | 0.9% | 1.0% | 0.9% | 0.9% | 0.9% | 0.9% |
| Polysorbate 80 | Report result (w/v%) | 0.051% | 0.050% | <0.010% | <0.010% | 0.020% | 0.019% | 0.051% | 0.050% | 0.083% | 0.082% | 0.106% | 0.104% |
| Protein concentration | Report result (mg/mL) | 105 | 104 | 187 | 187 | 190 | 187 | 190 | 190 | 187 | 189 | 190 | 191 |
| MFI | Report result (unit/mL) | | | | | | | | | | | | |
| | ≧25 µm | 1 | 0 | 55 | 4 | 1 | 1 | 0 | 0 | 3 | 5 | 1 | 0 |
| | 10 – 25 µm | 4 | 11 | 1065 | 83 | 14 | 11 | 5 | 8 | 3 | 14 | 8 | 5 |
| | 5 – 10 µm | 27 | 73 | 1317 | 287 | 15 | 20 | 8 | 53 | 51 | 56 | 19 | 59 |
| | 2 – 5 µm | 150 | 308 | 2321 | 1172 | 43 | 80 | 50 | 339 | 174 | 304 | 47 | 358 |

CSYL: Clear slightly yellow liquid, FVP: Free from visible particles.

**Table 31 Results of Agitation Study for BAN2401 Drug Product (F0, F8-F12)**

| Test item | Acceptance Criterion | F0 (Control) | | F8 PS80 0 w/v% | | F9 PS80 0.02 w/v% | | F10 PS80 0.05 w/v% | | F11 PS80 0.08 w/v% | | F12 PS8 0.10 w/v% | |
|---|---|---|---|---|---|---|---|---|---|---|---|---|---|
| | | Initial | Agitation 250 rpm/3 D | Initial | Agitation 250 rpm/3 D | Initial | Agitation 250 rpm/3 D | Initial | Agitation 250 rpm/3 D | Initial | Agitation 250 rpm/3 D | Initial | Agitation 250 rpm/3 D |
| Appearance | Report result | CSYL | CSYL | CSYL | OWL | CSYL | CSYL | CSYL | CSYL | CSYL | CSYL | CSYL | CSYL |
| pH | Report result | 4.98 | 4.99 | 4.87 | 4.89 | 4.87 | 4.87 | 4.87 | 4.88 | 4.88 | 4.87 | 4.88 | 4.88 |
| Visible particles | Report result | FVP | FVP | FVP | FVP | FVP | FVP | FVP | FVP | FVP | FVP | FVP | FVP |
| Size exclusion HPLC | Report result | | | | | | | | | | | | |
| | Monomer | 98.3% | 98.3% | 97.8% | 95.3% | 97.9% | 97.9% | 98.0% | 98.0% | 98.0% | 98.0% | 98.1% | 98.0% |
| | Aggregate | 1.0% | 1.0% | 1.1% | 3.8% | 1.1% | 1.1% | 1.1% | 1.1% | 1.1% | 1.1% | 1.1% | 1.1% |
| | Fragment | 0.7% | 0.7% | 1.1% | 0.9% | 1.1% | 1.0% | 0.9% | 0.9% | 0.9% | 0.9% | 0.9% | 0.9% |
| Polysorbate 80 | Report result (w/v%) | 0.051% | 0.049% | <0.010% | <0.010% | 0.019% | 0.019% | 0.053% | 0.054% | 0.086% | 0.084% | 0.107% | 0.108% |
| Protein concentration | Report result (mg/mL) | 105 | 105 | 206 | 195 | 207 | 206 | 206 | 206 | 209 | 207 | 209 | 206 |
| MFI | Report result | | | | | | | | | | | | |
| | ≧25μm | 1 | 3 | 24 | NT[a] | 3 | 1 | 0 | 0 | 1 | 0 | 0 | 1 |
| | 10 – 25 μm | 4 | 10 | 555 | NT[a] | 4 | 5 | 3 | 5 | 3 | 6 | 3 | 6 |
| | 5 – 10 μm | 27 | 27 | 1242 | NT[a] | 25 | 22 | 18 | 45 | 67 | 34 | 28 | 27 |
| | 2 – 5 μm | 150 | 75 | 3209 | NT[a] | 62 | 50 | 67 | 176 | 145 | 335 | 182 | 250 |

CSYL: Clear slightly yellow liquid. OWL: Opalescent white liquid. FVP: Free from visible particles. NT: Not tested.

a: Results are not reported since particles could not be correctly measured by bubble contamination due to insufficient sample volume.

**(n) Freeze-Thaw Study**

As shown in Table 30, no significant changes were observed in all the tested items after three cycles of freeze-thaw testing. Particle counts by MFI were relatively high in F8 (without PS80).

**(o) Agitation Study**

As shown in Table 31, formulations containing 0.02(w/v)% to 0.10(w/v)% of PS80 (F9 to F12) were stable after agitation. Changes were observed in F8 (without PS80) for appearance, protein concentration and aggregate amount by SEC after agitation. Appearance was changed from clear slightly yellow liquid to opalescent white liquid. Protein concentration slightly decreased from 206 to 195 mg/mL. Aggregate amount increased from 1.1% to 3.8%. Therefore, F8 (without PS80) was not considered feasible.

**(p) PS80 Concentration**

Based on the results shown in Table 30 (freeze-thaw study) and Table 31 (agitation study), formulations containing 0.02(w/v)% to 0.10(w/v)% of PS80 were stable after three cycle of freeze-thaw and agitation up to three days. Therefore, PS80 concentration of 0.05 (w/v)% was selected as the target.

**(q) Conclusion**

In conclusion, the following formulation was selected for BAN2401 formulation for subsequent studies.

**Table 32 Formulation for Subsequent Studies**

| Formulation No. | BAN2401 | Buffer | | Stabilizer (Arginine) | | Polysorbate 80 | | pH |
|---|---|---|---|---|---|---|---|---|
| | mg/mL | mmol/L | mg/mL | mmol/L | mg/mL | (w/v)% | mg/mL | |
| F2 | 200 | Histidine 25 | 3.9 | 200 | 34.8 | 0.05 | 0.5 | 5.0 |

**Example 9: Stability Study for 200 mg/mL BAN2401 Formulations with Different pH**

A stability study was conducted to test the stability of BAN2401 formulations at 200 mg/mL with different pH. In addition, the impact of methionine addition on drug product (DP) stability were tested.

**A. Sample Preparation**

Each candidate formulation (Table 33) was prepared as follows. Formulations were equilibrated with corresponding formulation buffer by centrifugal filter units. Only for F20 preparation, formulation buffer at 3.5 of a pH was used until the pH of filtrated solution achieved to 4.0 since protons are repelled by concentrated charged protein near semipermeable membrane, known as Donnan effect. After concentration and equilibration, PS80 dissolved in the formulation buffer was added to achieve pre-determined concentration and then the protein concentration was adjusted to the final concentration. Each candidate formulation was filled into vials with fill volume of 0.5 mL. Formulation F0 (see, Table 33) was evaluated as a control.

## Table 33 Composition of Drug Substance and Candidate SC Formulations

| No. | BAN2401 | Buffer | | Stabilizer | | Polysorbate 80 | | pH[a] | Fill volume |
|---|---|---|---|---|---|---|---|---|---|
| | mg/mL | mmol/L | mg/mL | mmol/L | mg/mL | (w/v)% | mg/mL | | mL /Vial |
| F0 | 100 | Citrate 50 | 9.6 | Arg 350 | 61.0 | 0.05 | 0.5 | 5.0 | 5 |
| F13 | 200 | Histidine 25 | 3.9 | Arg 200 | 34.8 | 0.05 | 0.5 | 4.5 | 0.5 |
| F15 | 200 | Histidine 25 | 3.9 | Arg 200 | 34.8 | 0.05 | 0.5 | 5.0 | 0.5 |
| F17 | 200 | Histidine 25 | 3.9 | Arg 200 | 34.8 | 0.05 | 0.5 | 5.5 | 0.5 |
| F18 | 200 | Histidine 25 | 3.9 | Arg 200 Met 10 | 34.8 1.5 | 0.05 | 0.5 | 5.0 | 0.5 |
| F20 | 200 | Histidine 25 | 3.9 | Arg 200 | 34.8 | 0.05 | 0.5 | 4.0 | 0.5 |
| F21[b] | 200 | Histidine 25 | 3.9 | Arg 200 | 34.8 | 0.05 | 0.5 | 5.0 | 0.5 |
| F22 | 200 | Histidine 25 | 3.9 | Arg 200 | 34.8 | 0.05 | 0.5 | 6.0 | 0.5 |

Arg: Arginine, Met: Methionine

a: To adjust pH, arginine and arginine hydrochloride was combined for F0 and hydrochloride was added for F13 through F22.

b: F21 is the same formulation as F15

## B. Storage protocol

Storage protocols are shown in Table 34, Table 35, and Table 36. Once removed from storage, the vials were stored at 5 °C until the testing started. Storage conditions are as follows:

- Long-tenn condition: stored at 5 °C±3°C upright
- Accelerated condition: Stored at 25 °C±2 °C, 60%RH±5%RH upright
- Stressed condition (Freeze-thaw): Frozen at -30 °C and thawed at room temperature, upright

  1000 lx: 25 °C±2 °C, 60%RH±5%RH, lay down with light exposure of 1000 lx
  Dark: 25 °C±2 °C, 60%RH±5%RH, lay down without light exposure by covering with aluminum foil

To confirm aggregate level after three-month or longer storage, size exclusion HPLC was additionally evaluated as extended storage samples at nine-months for long-term and three-months for accelerated stability study. Latter samples were tested after stored in refrigerator for six months.

**Table 34 Sampling Schedule for Long-term Stability Study (5 °C±3°C Upright)**

| | F13, F17 | | | F0, F15, F18 | | | F20, F21, F22 | |
|---|---|---|---|---|---|---|---|---|
| | Initial | 5°C | | Initial | 5°C | | Initial | 5°C |
| | | 2M | 9Mª | | 2M | 9Mª | | 3M |
| Color | X | X | NT | X | X | NT | X | X |
| Clarity | X | X | NT | X | X | NT | X | X |
| Particles | X | X | NT | X | X | NT | X | X |
| pH | X | X | NT | X | X | NT | X | X |
| Protein conc | X | X | NT | X | X | NT | X | X |
| ELISA | X | X | NT | X | X | NT | NT | NT |
| FcR | X | X | NT | X | X | NT | NT | NT |
| cSDS-R | X | X | NT | X | X | NT | NT | NT |
| cSDS-NR | X | X | NT | X | X | NT | NT | NT |
| SEC | X | X | X | X | X | X | X | X |
| IEX | X | X | NT | X | X | NT | X | X |
| Viscosity | X | NT | NT | X | NT | NT | X | NT |
| Osmolality | X | NT | NT | X | NT | NT | X | NT |
| PS80 | X | X | NT | X | X | NT | X | X |
| MFI | X | X | NT | X | X | NT | X | NT |
| PMAP-R | NT | NT | NT | X | X | NT | NT | NT |

X: Conducted, NT: Not tested
Color: Degree of coloration of liquids, Clarity: Clarity and degree of opalescence, Protein conc: Protein concentration, ELISA: Antigen binding ELISA, FcR: Binding assay (CD32a), cSDS: Capillary SDS Gel Electrophoresis R: Reduced, NR: Non-reduced, SEC: Size exclusion HPLC, IEX: Ion exchange HPLC, PS80: Polysorbate 80 content, MFI: Micro-flow imaging, PMAP: Peptide map
a: Extended evaluation

**Table 35 Sampling Schedule for Accelerated Stability Study (25 °C±2 °C, 60%RH±5%RH Upright)**

| Test items | F13, F17 | | | F0, F15, F18 | | | | F20, F21, F22 | | |
|---|---|---|---|---|---|---|---|---|---|---|
| | Initial | 25°C | | Initial | 25°C | | | Initial | 25°C | |
| | | 1M, 2M | Ex | | 1M | 2M | Ex | | 1M | 3M |
| Color | X | X | NT | X | X | X | NT | X | X | X |
| Clarity | X | X | NT | X | X | X | NT | X | X | X |
| Particles | X | X | NT | X | X | X | NT | X | X | X |
| pH | X | X | NT | X | X | X | NT | X | X | X |
| Protein conc | X | X | NT | X | X | X | NT | X | X | X |
| ELISA | X | X | NT | X | X | X | NT | NT | NT | NT |
| FcR | X | X | NT | X | X | X | NT | NT | NT | NT |
| cSDS-R | X | X | NT | X | X | X | NT | NT | NT | NT |
| cSDS-NR | X | X | NT | X | X | X | NT | NT | NT | NT |
| SEC | X | X | X | X | X | X | X | X | X | X |
| IEX | X | X | NT | X | X | X | NT | X | X | X |
| Viscosity | X | NT | NT | X | NT | NT | NT | X | NT | NT |
| Osmolality | X | NT | NT | X | NT | NT | NT | X | NT | NT |
| PS80 | X | X | NT | X | X | X | NT | X | X | X |
| MFI | X | X | NT | X | X | X | NT | X | X | NT |
| PMAP-R | NT | NT | NT | X | NT | X | NT | NT | NT | NT |

Ex: Extended evaluation, stored for 3M at 25 °C and for 6M at 5 °C after sample pull

X: Conducted, NT: Not tested

Color: Degree of coloration of liquids, Clarity: Clarity and degree of opalescence, Protein conc: Protein concentration, ELISA: Antigen binding ELISA, FcR: Binding assay (CD32a), cSDS: Capillary SDS Gel Electrophoresis R: Reduced, NR: Non-reduced, SEC: Size exclusion HPLC, IEX: Ion exchange HPLC, PS80: Polysorbate 80 content, MFI: Micro-flow imaging, PMAP: Peptide map

## Table 36 Sampling Schedule for Freeze-thaw and Photo-stability study

| Test items | F15 | | | | F18 | | | |
|---|---|---|---|---|---|---|---|---|
| | Initial | FT | Dark | 1000lx | Initial | FT | Dark | 1000lx |
| | | 3cycle | 7D | 7D | | 3cycle | 7D | 7D |
| Color | X | X | X | X | X | X | X | X |
| Clarity | X | X | X | X | X | X | X | X |
| Particles | X | X | X | X | X | X | X | X |
| Pro conc | X | X | X | X | X | X | X | X |
| pH | X | X | X | X | X | X | X | X |
| PS80 | X | X | X | X | X | X | X | X |
| ELISA | X | X | X | X | X | X | X | X |
| FcR | X | X | X | X | X | X | X | X |
| cSDS-R | X | X | X | X | X | X | X | X |
| cSDS-NR | X | X | X | X | X | X | X | X |
| SEC | X | X | X | X | X | X | X | X |
| IEX | X | X | X | X | X | X | X | X |
| PMAP-R | X | NT | X | X | X | NT | X | X |

FT: Freeze-thaw (Frozen at −30 °C and thawed at room temperature, Upright), Dark: 25 °C±2 °C, 60%RH±5%RH, lay down without light exposure by covering with aluminum foil, 1000 lx: 25 °C±2 °C, 60%RH±5%RH, lay down with light exposure of 1000 lx by fluorescent light, X: Conducted,

Color: Degree of coloration of liquids, Clarity: Clarity and degree of opalescence, MFI: Micro-flow imaging, Pro conc: Protein concentration, PS80: Polysorbate 80 content, ELISA: Antigen binding ELISA, FcR: Binding assay (CD32a), cSDS: Capillary SDS Gel Electrophoresis, R: Reduced, NR: Non-reduced, SEC: Size exclusion HPLC, IEX: Ion exchange HPLC, PMAP: Peptide map

## C. Results and Discussion

Physical properties were evaluated and long-term, and accelerated studies were conducted for candidate formulations including pH variation (pH 4.0 to pH 6.0), and methionine addition. Freeze-thaw and photo-stability studies were also conducted to evaluate the efficiency of methionine addition.

## D. pH Variation

As shown in Table 37, Table 38A and 38B, Table 39, and Table 40, no significant differences among candidate formulations (F13, F15, F17 and F20 to F22) were observed for any testing items except for size exclusion HPLC (SEC). Formulation F20 (*i.e.*, a formulation with lower pH of 4.0), showed lower amount of aggregates by SEC at initial timepoint. In addition, slightly lower rate of aggregates formation and higher rate of fragments formation were observed in an accelerated stability study. Results of formulations with pH from 4.5 to 5.5 were confirmed to be comparable.

**Table 37 Results of Stability Study for Formulations of BAN2401 with pH 4.0 to 6.0, Stored at 5 °C±3°C, Upright Position**

| Test item | Acceptance Criterion | F0 (Control) Initial | F0 (Control) 5°C/2M | F13 pH4.5 Initial | F13 pH4.5 5°C/2M | F15 pH5.0 Initial | F15 pH5.0 5°C/2M | F17 pH5.5 Initial | F17 pH5.5 5°C/2M | F20 pH4.0 Initial | F20 pH4.0 5°C/3M | F21 pH5.0 Initial | F21 pH5.0 5°C/3M | F22 pH6.0 Initial | F22 pH6.0 5°C/3M |
|---|---|---|---|---|---|---|---|---|---|---|---|---|---|---|---|
| Color | Report result | BY5 | BY5 | BY5 | BY5 | BY5 | BY5 | BY5 | BY5 | BY5 | BY5 | BY5 | BY5 | BY5 | BY5 |
| Clarity | Report result | ≤ RS2 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 |
| Particles | Report result | FVP | FVP | FVP | FVP | FVP | FVP | FVP | FVP | FVP | FVP | FVP | FVP | FVP | FVP |
| pH | Report result | 4.95 | 4.98 | 4.63 | 4.64 | 4.94 | 4.96 | 5.68 | 5.73 | 4.03 | 4.05 | 4.91 | 4.92 | 5.90 | 5.91 |
| Protein conc | Report result(mg/mL) | 104 | 102 | 200 | 201 | 197 | 195 | 204 | 204 | 197 | 195 | 200 | 195 | 207 | 195 |
| ELISA | Report result | 101.3% | 102.6% | 109.2% | 104.5% | 102.1% | 123.1% | 107.0% | 125.7% | NT | NT | NT | NT | NT | NT |
| FcR | Report result | 91.7% | 93.8% | 101.0% | 100.4% | 108.8% | 99.0% | 116.6% | 103.4% | NT | NT | NT | NT | NT | NT |
| cSDS-R | Report result | 98.3% | 97.7% | 98.8% | 98.2% | 98.9% | 98.5% | 98.8% | 98.4% | NT | NT | NT | NT | NT | NT |
| cSDS-NR | Report result | 91.2% | 91.9% | 91.5% | 92.5% | 91.2% | 92.9% | 91.3% | 92.6% | NT | NT | NT | NT | NT | NT |
| SEC | Monomer ≥95.0% | 98.6% | 98.7% | 99.2% | 99.2% | 99.1% | 99.1% | 98.8% | 98.8% | 99.3% | 99.1% | 99.2% | 99.0% | 98.8% | 98.6% |
| | Aggregate ≤5.0% | 1.0% | 0.9% | 0.8% | 0.8% | 0.9% | 0.9% | 1.2% | 1.2% | 0.7% | 0.7% | 0.8% | 1.0% | 1.2% | 1.4% |
| | Report Fragment | 0.5% | 0.4% | <0.1% | <0.1% | <0.1% | <0.1% | <0.1% | <0.1% | 0.1% | 0.2% | <0.1% | <0.1% | <0.1% | <0.1% |
| IEX | Main 50%-90% | 71.3% | 70.8% | 77.2% | 73.1% | 76.7% | 72.9% | 76.8% | 72.8% | 76.8% | 74.7% | 76.7% | 75.2% | 76.6% | 74.8% |
| | Acidic | 24.4% | 25.2% | 19.1% | 19.5% | 19.4% | 19.7% | 19.4% | 19.9% | 18.8% | 21.6% | 18.9% | 21.1% | 19.1% | 21.3% |
| | Basic | 4.2% | 4.0% | 3.7% | 7.4% | 3.9% | 7.4% | 3.8% | 7.2% | 4.4% | 3.6% | 4.3% | 3.7% | 4.3% | 3.9% |
| Viscosity | Report result | 2.6 | NT | 8.1 | NT | 7.9 | NT | 10.0 | NT | 8.0 | NT | 9.4 | NT | 11.6 | NT |
| Osmolality | Report result | 512 | NT | 416 | NT | 407 | NT | 402 | NT | 420 | NT | 423 | NT | 396 | NT |
| Polysorbate 80 | Report result (w/v%) | 0.048% | 0.051% | 0.044% | 0.047% | 0.044% | 0.047% | 0.043% | 0.045% | 0.052% | 0.049% | 0.052% | 0.052% | 0.051% | 0.048% |
| MFI | Report result | | | | | | | | | | | | | | |
| | ≥25μm | 1 | 5 | 3 | 3 | 7 | 3 | 0 | 0 | 0 | NT | 0 | NT | 0 | NT |
| | 10-25μm | 4 | 81 | 99 | 5 | 26 | 26 | 17 | 10 | 10 | NT | 5 | NT | 14 | NT |
| | 5-10μm | 27 | 283 | 537 | 67 | 335 | 170 | 214 | 207 | 152 | NT | 5 | NT | 76 | NT |
| | 2-5μm | 150 | 1452 | 980 | 276 | 1246 | 977 | 1365 | 1273 | 698 | NT | 95 | NT | 322 | NT |

BY5: Equal to reference solution BY5. RS2: Reference suspension II, RS3: Reference suspension III. FVP: Free from visible particles, NT: Not tested.

## Table 38A Results of Stability Study for Formulations of BAN2401 with pH 4.0 to 6.0, Stored at 25 °C±2 °C, 60%RH±5%RH, Upright Position

| Test item | Acceptance Criterion | F0 (Control) | | | F13 pH4.5 | | | F15 pH5.0 | | | F17 pH5.5 | | | F20 pH4.0 | | | F21 pH5.0 | | | F22 pH6.0 | | |
|---|---|---|---|---|---|---|---|---|---|---|---|---|---|---|---|---|---|---|---|---|---|---|---|---|
| | | Initial | 25°C/ 1M | 25°C/ 2M | Initial | 25°C/ 1M | 25°C/ 2M | Initial | 25°C/ 1M | 25°C/ 2M | Initial | 25°C/ 1M | 25°C/ 2M | Initial | 25°C/ 1M | 25°C/ 3M | Initial | 25°C/ 1M | 25°C/ 3M | Initial | 25°C/ 1M | 25°C/ 3M |
| Color | Report | BY5 | BY5 | BY5 | BY5 | BY5 | BY5 | BY5 | BY5 | BY5 | BY5 | BY5 | BY5 | BY5 | BY5 | BY5 | BY5 | BY5 | BY5 | BY5 | BY5 | BY5 |
| Clarity | Report | ≤ RS2 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 | ≤ RS3 |
| Particles | Report | FVP | FVP | FVP | FVP | FVP | FVP | FVP | FVP | FVP | FVP | FVP | FVP | FVP | FVP | FVP | FVP | FVP | FVP | FVP | FVP | FVP |
| pH | Report | 4.95 | 4.94 | 4.99 | 4.63 | 4.63 | 4.66 | 4.94 | 4.96 | 4.98 | 5.68 | 5.68 | 5.72 | 4.03 | 4.02 | 4.10 | 4.91 | 4.91 | 4.94 | 5.90 | 5.91 | 5.91 |
| Protein conc | Report (mg/mL) | 104 | 103 | 101 | 200 | 201 | 199 | 197 | 194 | 192 | 204 | 207 | 203 | 197 | 198 | 193 | 200 | 198 | 194 | 207 | 204 | 194 |
| ELISA | Report | 101.3% | 102.7% | 91.8% | 109.2% | 104.5% | 107.0% | 102.1% | 103.6% | 96.9% | 107.0% | 107.4% | 101.6% | NT | NT | NT | NT | NT | NT | NT | NT | NT |
| FcR | Report | 91.7% | 95.5% | 93.5% | 101.0% | 99.4% | 101.5% | 108.8% | 98.3% | 102.2% | 116.6% | 103.8% | 104.9% | NT | NT | NT | NT | NT | NT | NT | NT | NT |
| cSDS-R | Report | 98.3% | 98.1% | 97.2% | 98.8% | 98.9% | 97.9% | 98.9% | 98.8% | 98.0% | 98.8% | 98.7% | 98.1% | NT | NT | NT | NT | NT | NT | NT | NT | NT |
| cSDS -NR | Report | 91.2% | 89.7% | 90.4% | 91.5% | 89.6% | 90.1% | 91.2% | 89.5% | 90.0% | 91.3% | 89.7% | 90.8% | NT | NT | NT | NT | NT | NT | NT | NT | NT |
| SEC | Monomer ≥95.0% | 98.6% | 98.4% | 98.4% | 99.2% | 98.6% | 98.4% | 99.1% | 98.4% | 98.2% | 98.8% | 98.2% | 98.2% | 99.3% | 98.4% | 97.4% | 99.2% | 98.6% | 98.4% | 98.8% | 98.2% | 98.1% |
| | Aggregate ≤5.0% | 1.0% | 1.0% | 0.9% | 0.8% | 1.1% | 1.2% | 0.9% | 1.4% | 1.5% | 1.2% | 1.8% | 1.7% | 0.7% | 1.0% | 0.9% | 0.8% | 1.3% | 1.3% | 1.2% | 1.7% | 1.7% |
| | Report Fragment | 0.5% | 0.5% | 0.7% | <0.1% | 0.2% | 0.4% | <0.1% | 0.1% | 0.3% | <0.1% | 0.1% | 0.1% | 0.1% | 0.6% | 1.7% | <0.1% | 0.1% | 0.4% | <0.1% | 0.1% | 0.2% |
| IEX | Main 50%-90% | 71.3% | 69.4% | 67.7% | 77.2% | 75.4% | 74.1% | 76.7% | 75.2% | 71.4% | 76.8% | 76.0% | 72.9% | 76.8% | 68.9% | 64.3% | 76.7% | 71.2% | 67.5% | 76.6% | 71.2% | 67.6% |
| | Acidic | 24.4% | 26.3% | 28.2% | 19.1% | 20.3% | 22.0% | 19.4% | 20.6% | 23.2% | 19.4% | 20.2% | 22.5% | 18.8% | 23.9% | 32.0% | 18.9% | 21.9% | 28.5% | 19.1% | 22.1% | 28.3% |
| | Basic | 4.2% | 4.4% | 4.1% | 3.7% | 4.3% | 3.8% | 3.9% | 4.2% | 5.4% | 3.8% | 3.8% | 4.6% | 4.4% | 7.2% | 3.7% | 4.3% | 7.0% | 4.0% | 4.3% | 6.7% | 4.0% |

BY5: Equal to reference solution BY₅. RS2: Reference suspension II, RS3: Reference suspension III, FVP: Free from visible particles. NT: Not tested. Report: Report result

## Table 38B

| Test item | Acceptance Criterion | F0 (Control) | | | F13 pH4.5 | | | F15 pH5.0 | | | F17 pH5.5 | | | F20 pH4.0 | | | F21 pH5.0 | | | F22 pH6.0 | | |
|---|---|---|---|---|---|---|---|---|---|---|---|---|---|---|---|---|---|---|---|---|---|---|---|
| | | Initial | 25°C/1M | 25°C/2M | Initial | 25°C/1M | 25°C/2M | Initial | 25°C/1M | 25°C/2M | Initial | 25°C/1M | 25°C/2M | Initial | 25°C/1M | 25°C/3M | Initial | 25°C/1M | 25°C/3M | Initial | 25°C/1M | 25°C/3M |
| Viscosity | Report (cP) | 2.6 | NT | NT | 8.1 | NT | NT | 7.9 | NT | NT | 10.0 | NT | NT | 8.0 | NT | NT | 9.4 | NT | NT | 11.6 | NT | NT |
| Osmolality | Report (mOsm/kg) | 512 | NT | NT | 416 | NT | NT | 407 | NT | NT | 402 | NT | NT | 420 | NT | NT | 423 | NT | NT | 396 | NT | NT |
| Polysorbate 80 | Report (w/v%) | 0.048% | 0.052% | 0.050% | 0.044% | 0.047% | 0.045% | 0.044% | 0.047% | 0.043% | 0.043% | 0.041% | 0.042% | 0.052% | 0.049% | 0.042% | 0.052% | 0.053% | 0.051% | 0.051% | 0.048% | 0.043% |
| MFI | $\geq 25\mu m$ | 1 | 5 | 0 | 3 | 0 | 3 | 7 | 0 | 0 | 0 | 10 | 5 | 0 | 0 | NT | 0 | 0 | NT | 0 | 0 | NT |
| | 10-25μm | 4 | 3 | 35 | 99 | 51 | 23 | 26 | 49 | 35 | 17 | 46 | 49 | 10 | 26 | NT | 5 | 3 | NT | 14 | 3 | NT |
| | 5-10μm | 27 | 81 | 292 | 537 | 769 | 163 | 335 | 959 | 714 | 214 | 597 | 553 | 152 | 246 | NT | 5 | 7 | NT | 76 | 35 | NT |
| | 2-5μm | 150 | 457 | 1830 | 980 | 3139 | 647 | 1246 | 2919 | 1963 | 1365 | 2243 | 1778 | 698 | 454 | NT | 95 | 69 | NT | 322 | 285 | NT |

NT: Not tested, Report: Report result

**Table 39 Results of Extended Stability Study for Formulations with pH 4.5 to 5.5, Stored at 5 °C±3°C, Upright Position**

| Test item | | F0 (Control) | | | F13 pH4.5 | | | F15 pH5.0 | | | F17 pH5.5 | | |
|---|---|---|---|---|---|---|---|---|---|---|---|---|---|
| | | Initial | 5°C/2M | 5°C/9M | Initial | 5°C/2M | 5°C/9M | Initial | 5°C/2M | 5°C/9M | Initial | 5°C/2M | 5°C/9M |
| Size exclusion HPLC | Monomer | 98.6% | 98.7% | 98.6% | 99.2% | 99.2% | 98.8% | 99.1% | 99.1% | 98.6% | 98.8% | 98.8% | 98.3% |
| | Aggregate | 1.0% | 0.9% | 1.0% | 0.8% | 0.8% | 1.1% | 0.9% | 0.9% | 1.4% | 1.2% | 1.2% | 1.7% |
| | Fragment | 0.5% | 0.4% | 0.5% | <0.1% | <0.1% | 0.1% | <0.1% | <0.1% | 0.1% | <0.1% | <0.1% | <0.1% |

**Table 40 Results of Extended Stability Study for Current and Lead Formulation with pH 4.5 to 5.5, Stored at 25 °C±2 °C, 60%RH±5%RH, Upright Position**

| Test item | | F0 (Control) | | | | F13 pH4.5 | | | | F15 pH5.0 | | | | F17 pH5.5 | | | |
|---|---|---|---|---|---|---|---|---|---|---|---|---|---|---|---|---|---|
| | | Initial | 25°C/ 1M | 25°C/ 2M | 25°C/ 3M +5°C/ 6M | Initial | 25°C/ 1M | 25°C/ 2M | 25°C/ 3M +5°C/ 6M | Initial | 25°C/ 1M | 25°C/ 2M | 25°C/ 3M +5°C/ 6M | Initial | 25°C/ 1M | 25°C/ 2M | 25°C/ 3M +5°C/ 6M |
| Size exclusion HPLC | Monomer | 98.6% | 98.4% | 98.4% | 98.0% | 99.2% | 98.6% | 98.4% | 97.7% | 99.1% | 98.4% | 98.2% | 97.7% | 98.8% | 98.2% | 98.2% | 97.7% |
| | Aggregate | 1.0% | 1.0% | 0.9% | 1.0% | 0.8% | 1.1% | 1.2% | 1.5% | 0.9% | 1.4% | 1.5% | 1.8% | 1.2% | 1.8% | 1.7% | 2.0% |
| | Fragment | 0.5% | 0.5% | 0.7% | 0.9% | <0.1% | 0.2% | 0.4% | 0.8% | <0.1% | 0.1% | 0.3% | 0.5% | <0.1% | 0.1% | 0.1% | 0.3% |

### E. Methionine Addition

As shown in Tables 41A and 41B, and Table 42 to Tables 46, no significant difference was observed between formulations with or without methionine (F18 and F15). Formulation with methionine (F18) showed slightly lower rate of aggregate formation in extended a long-term stability study, accelerated stability study, and photostability study. F18 also showed slightly lower oxidation of methionine residue (e.g., at position 259 in the heavy chains) in light exposure with 1000 lux up to seven days. Freeze-thaw study did not show any difference. The effect of 10 mmol/L. of methionine as a stabilizer was limited.

**Table 41A Results of Stability Study for Formulations with or without Met, Stored at 5 °C±3°C, Upright Position**

| | | F0 | (Control) | F15 | Met(-) | F18 | Met (+) |
|---|---|---|---|---|---|---|---|
| Test item | Acceptance Criterion | Initial | 5°C/2M | Initial | 5°C/2M | Initial | 5°C/2M |
| Color | Report result | BY5 | BY5 | BY5 | BY5 | BY5 | BY5 |
| Clarity | Report result | ≤ RS2 | ≤ RS3 | ≤ RS3 | ≤ RS3 | ≤ RS3 | ≤ RS3 |
| Particles | Report result | FVP | FVP | FVP | FVP | FVP | FVP |
| pH | Report result (mg/mL) | 4.95 | 4.98 | 4.94 | 4.96 | 4.95 | 4.98 |
| Protein conc | Report result | 104 | 102 | 197 | 195 | 191 | 189 |
| ELISA | Report result | 101.3% | 102.6% | 102.1% | 123.1% | 108.2% | 118.1% |
| FcR | Report result | 91.7% | 93.8% | 108.8% | 99.0% | 92.4% | 94.7% |
| cSDS-R | Report result | 98.3% | 97.7% | 98.9% | 98.5% | 98.9% | 98.5% |
| cSDS-NR | Report result | 91.2% | 91.9% | 91.2% | 92.9% | 91.5% | 92.7% |
| SEC | Monomer ≥95.0% | 98.6% | 98.7% | 99.1% | 99.1% | 99.2% | 99.1% |
| | Aggregate ≤5.0% | 1.0% | 0.9% | 0.9% | 0.9% | 0.8% | 0.9% |
| | Report Fragment | 0.5% | 0.4% | <0.1% | <0.1% | <0.1% | <0.1% |
| IEX | Main 50%-90% | 71.3% | 70.8% | 76.7% | 72.9% | 77.2% | 75.9% |
| | Acidic | 24.4% | 25.2% | 19.4% | 19.7% | 19.0% | 19.2% |
| | Basic | 4.2% | 4.0% | 3.9% | 7.4% | 3.8% | 4.9% |

Met: Methionine, BY5: Equal to reference solution BY₅, RS2: Reference suspension II, RS3: Reference suspension III, FVP: Free from visible particles

**Table 41B**

| Test item | Acceptance Criterion | F0 | (Control) | F15 | Met(-) | F18 | Met (+) |
|---|---|---|---|---|---|---|---|
| | | Initial | 5°C/2M | Initial | 5°C/2M | Initial | 5°C/2M |
| Viscosity | Report result (cP) | 2.6 | NT | 7.9 | NT | 7.3 | NT |
| Osmolality | Report result (mOsm/kg) | 512 | NT | 407 | NT | 416 | NT |
| Polysorbate 80 | Report result (w/v%) | 0.048% | 0.051% | 0.044% | 0.047% | 0.045% | 0.039% |
| MFI | ≥25μm | 1 | 5 | 7 | 3 | 0 | 0 |
| | 10-25μm | 4 | 81 | 26 | 26 | 3 | 0 |
| | 5-10μm | 27 | 283 | 335 | 170 | 131 | 159 |
| | 2-5μm | 150 | 1452 | 1246 | 977 | 691 | 163 |

Met: Methionine, NT: Not tested.

**Table 42 Results of Stability Study for Formulations with or without Met, Stored at 25 °C±2 °C, 60%RH±5%RH, Upright Position**

| Test item | Acceptance Criterion | F0 (Control) | | | F15 Met(-) | | | F18 Met(+) | | |
|---|---|---|---|---|---|---|---|---|---|---|
| | | Initial | 25°C/1M | 25°C/2M | Initial | 25°C/1M | 25°C/2M | Initial | 25°C/1M | 25°C/2M |
| Color | Report result | BY5 | BY5 | BY5 | BY5 | BY5 | BY5 | BY5 | BY5 | BY5 |
| Clarity | Report result | ≤ RS2 | ≤ RS3 | ≤ RS3 | ≤ RS3 | ≤ RS3 | ≤ RS3 | ≤ RS3 | ≤ RS3 | ≤ RS3 |
| Particles | Report result | FVP | FVP | FVP | FVP | FVP | FVP | FVP | FVP | FVP |
| pH | Report result | 4.95 | 4.94 | 4.99 | 4.94 | 4.96 | 4.98 | 4.95 | 4.94 | 4.97 |
| Protein conc | Report result | 104 | 103 | 101 | 197 | 194 | 192 | 191 | 192 | 189 |
| ELISA | Report result | 101.3% | 102.7% | 91.8% | 102.1% | 103.6% | 96.9% | 108.2% | 97.0% | 97.8% |
| FcR | Report result | 91.7% | 95.5% | 93.5% | 108.8% | 98.3% | 102.2% | 92.4% | 91.9% | 92.9% |
| cSDS-R | Report result | 98.3% | 98.1% | 97.2% | 98.9% | 98.8% | 98.0% | 98.9% | 98.8% | 98.1% |
| cSDS-NR | Report result | 91.2% | 89.7% | 90.4% | 91.2% | 89.5% | 90.0% | 91.5% | 89.8% | 90.6% |
| SEC | Monomer ≥95.0% | 98.6% | 98.4% | 98.4% | 99.1% | 98.4% | 98.2% | 99.2% | 98.6% | 98.5% |
| | Aggregate ≤5.0% | 1.0% | 1.0% | 0.9% | 0.9% | 1.4% | 1.5% | 0.8% | 1.2% | 1.2% |
| | Report Fragment | 0.5% | 0.5% | 0.7% | <0.1% | 0.1% | 0.3% | <0.1% | 0.1% | 0.3% |
| IEX | Main 50%-90% | 71.3% | 69.4% | 67.7% | 76.7% | 75.2% | 71.4% | 77.2% | 76.1% | 72.9% |
| | Acidic | 24.4% | 26.3% | 28.2% | 19.4% | 20.6% | 23.2% | 19.0% | 20.2% | 22.6% |
| | Basic | 4.2% | 4.4% | 4.1% | 3.9% | 4.2% | 5.4% | 3.8% | 3.7% | 4.4% |
| Viscosity | Report result (cP) | 2.6 | NT | NT | 7.9 | NT | NT | 7.3 | NT | NT |
| Osmolality | Report result (mOsm/kg) | 512 | NT | NT | 407 | NT | NT | 416 | NT | NT |
| Polysorbate 80 | Report result (w/v%) | 0.048% | 0.052% | 0.050% | 0.044% | 0.047% | 0.043% | 0.045% | 0.043% | 0.038% |
| MFI | ≧25μm | 1 | 5 | 0 | 7 | 0 | 0 | 0 | 0 | 3 |
| | 10-25μm | 4 | 3 | 35 | 26 | 49 | 35 | 3 | 7 | 65 |

| 5-10μm | 27 | 81 | 292 | 335 | 459 | 714 | 131 | 230 | 647 |
| 2-5μm | 150 | 457 | 1830 | 1246 | 2919 | 1963 | 691 | 792 | 2516 |

Meth: Methionine, NT: Not tested, BYS: Equal to reference solution BY₄, RS2: Reference suspension II, RS3: Reference suspension III, FVP: Free from visible particles, NT: Not tested.

**Table 43 Results of Extended Stability Study for Current and Lead Formulation with or without Met, from DS1 or DS2, Stored at 5 °C±3°C, Upright Position**

| Test item | | F0 | (Control) | | F15 | Met(-) | | F18 | Met (+) | |
|---|---|---|---|---|---|---|---|---|---|---|
| | | Initial | 5°C/ 2M | 5°C/ 9M | Initial | 5°C/ 2M | 5°C/ 9M | Initial | 5°C/ 2M | 5°C/ 9M |
| Size exclusion HPLC | Monomer | 98.6% | 98.7% | 98.6% | 99.1% | 99.1% | 98.6% | 99.2% | 99.1% | 98.7% |
| | Aggregate | 1.0% | 0.9% | 1.0% | 0.9% | 0.9% | 1.4% | 0.8% | 0.9% | 1.2% |
| | Fragment | 0.5% | 0.4% | 0.5% | <0.1% | <0.1% | 0.1% | <0.1% | <0.1% | 0.1% |

Met: Methionine

**Table 44 Results of Extended Stability Study for Formulations with or without Met, Stored at 25 °C±2 °C, 60%RH±5%RH, Upright Position**

| Test item | | F0 | (Control) | | | F15 | Met(-) | | | F18 | Met (+) | | |
|---|---|---|---|---|---|---|---|---|---|---|---|---|---|
| | | Initial | 25°C/ 1M | 25°C/ 2M | 25°C/ 3M +5°C/ 6M | Initial | 25°C/ 1M | 25°C/ 2M | 25°C/ 3M +5°C/ 6M | Initial | 25°C/ 1M | 25°C/ 2M | 25°C/ 3M +5°C/ 6M |
| Size exclusion HPLC | Monomer | 98.6% | 98.4% | 98.4% | 98.0% | 99.1% | 98.4% | 98.2% | 97.7% | 99.2% | 98.6% | 98.5% | 98.0% |
| | Aggregate | 1.0% | 1.0% | 0.9% | 1.0% | 0.9% | 1.4% | 1.5% | 1.8% | 0.8% | 1.2% | 1.2% | 1.5% |
| | Fragment | 0.5% | 0.5% | 0.7% | 0.9% | <0.1% | 0.1% | 0.3% | 0.5% | <0.1% | 0.1% | 0.3% | 0.5% |

Met: Methionine

## Table 45 Results of Stressed (Freeze-thaw and Photo-stability) Study for Formulations with or without Methionine

| Test item | Acceptance Criterion | F15 Met(-) | | | | F18 Met (+) | | | |
|---|---|---|---|---|---|---|---|---|---|
| | | Initial | FT 3cycle | 25°C Dark/7D | 25°C 1000lx/7D | Initial | FT 3cycle | 25°C Dark/7D | 25°C 1000lx/7D |
| vColor | Report result | BY5 | BY5 | BY5 | BY5 | BY5 | BY5 | BY5 | BY5 |
| Clarity | Report result | ≤ RS3 | ≤ RS3 | ≤ RS3 | ≤ RS3 | ≤ RS3 | ≤ RS3 | ≤ RS3 | ≤ RS3 |
| Particles | Report result | FVP | FVP | FVP | FVP | FVP | FVP | FVP | FVP |
| pH | Report result | 4.94 | 4.94 | 4.94 | 4.94 | 4.95 | 4.94 | 4.93 | 4.92 |
| Protein conc | Report result | 197 | 197 | 190 | 193 | 191 | 190 | 189 | 190 |
| ELISA | Report result | 102.1% | 107.7% | 107.0% | 100.9% | 108.2% | 105.8% | 104.7% | 109.1% |
| FcR | Report result | 108.8% | 107.5% | 104.0% | 117.6% | 106.0% | 105.6% | 104.8% | 109.4% |
| cSDS-R | Report result | 98.9% | 99.3% | 99.3% | 98.7% | 98.9% | 99.1% | 99.3% | 98.7% |
| cSDS-NR | Report result | 91.2% | 93.0% | 92.6% | 92.0% | 91.5% | 92.8% | 92.9% | 91.4% |
| Size exclusion HPLC | Monomer ≥95.0% | 99.1% | 99.0% | 98.9% | 97.5% | 99.2% | 99.1% | 98.9% | 97.9% |
| | Aggregate ≤5.0% | 0.9% | 1.0% | 1.0% | 2.4% | 0.8% | 0.9% | 1.0% | 2.0% |
| | Report Fragment | <0.1% | <0.1% | 0.1% | 0.1% | <0.1% | <0.1% | 0.1% | 0.1% |
| IEX | Main 50%-90% | 76.7% | 73.5% | 74.8% | 65.5% | 77.2% | 77.3% | 76.2% | 62.7% |
| | Acidic | 19.4% | 20.8% | 20.9% | 20.3% | 19.0% | 18.8% | 19.8% | 20.0% |
| | Basic | 3.9% | 5.8% | 4.4% | 14.2% | 3.8% | 3.9% | 3.9% | 17.3% |
| Viscosity | Report result | 7.9 | NT | NT | NT | 7.3 | NT | NT | NT |
| Osmolality | Report result | 407 | NT | NT | NT | 416 | NT | NT | NT |
| Polysorbate 80 | Report result (w/v%) | 0.044% | 0.047% | 0.046% | 0.046% | 0.045% | 0.047% | 0.042% | 0.046% |
| MFI | ≥25μm | 7 | 0 | NT | 44 | 0 | 5 | NT | 14 |
| | 10-25μm | 26 | 76 | NT | 278 | 3 | 44 | NT | 99 |
| | 5-10μm | 335 | 661 | NT | 1074 | 131 | 294 | NT | 698 |
| | 2-5μm | 1246 | 2704 | NT | 3293 | 691 | 1289 | NT | 2491 |

FT: Freeze-thaw, (Frozen at -30 °C and thawed at room temperature, Upright), 25 °C: 25 °C±2 °C, 60%RH±5%RH, 1000 lx: lay down with light exposure of 1000 lx, Dark: lay down without light exposure by covering with aluminum foil, Met: Methionine, BY5: Equal to reference solution BY5, RS3: Reference suspension III , FVP: Free from visible particles, NT: Not tested

## Table 46 Results of Reduced Peptide Map

| Formulation No. | | F0 | | | F15  Met (-) | | | | | F18  Met (+) | | | | |
|---|---|---|---|---|---|---|---|---|---|---|---|---|---|---|
| | | Initial | 5°C 2M | 25°C 2M | Initial | 5°C 2M | 25°C 2M | 25°C Dark,7D | 25°C 1000lux,7D | Initial | 5°C 2M | 25°C 2M | 25°C Dark,7D | 25°C 1000lux,7D |
| Sequence Coverage | HC | 100.0% | 100.0% | 100.0% | 100.0% | 100.0% | 100.0% | 100.0% | 100.0% | 100.0% | 100.0% | 100.0% | 100.0% | 100.0% |
| | LC | 99.5% | 100.0% | 100.0% | 100.0% | 98.6% | 100.0% | 100.0% | 98.6% | 98.6% | 100.0% | 100.0% | 98.6% | 98.6% |
| Deamidation | HC N332 | 8.7% | 8.4% | 8.3% | 8.2% | 7.6% | 7.6% | 7.6% | 7.8% | 7.7% | 7.2% | 7.2% | 7.7% | 7.6% |
| | LC N33 | 11.0% | 11.6% | 12.3% | 11.1% | 10.4% | 11.3% | 10.8% | 10.5% | 11.0% | 10.9% | 11.3% | 10.3% | 10.7% |
| Oxidation | HC M259 | <1% | 4.5% | 5.0% | 3.5% | 4.0% | 5.8% | 4.3% | 12.4% | 2.7% | 2.7% | 2.8% | 3.1% | 9.4% |
| | HC M356 | <1% | <1% | <1% | <1% | <1% | <1% | <1% | 1.9% | <1% | <1% | <1% | <1% | <1% |
| | HC M435 | 3.4% | 2.4% | 2.8% | 1.9% | 2.1% | 2.7% | 2.7% | 9.6% | 1.7% | 1.4% | 1.5% | 0.8% | 8.2% |
| | LC M4 | 1.5% | 1.5% | 1.5% | 1.6% | 1.8% | 1.7% | 1.8% | 2.2% | 1.6% | 1.5% | 1.4% | 1.7% | 1.7% |
| Lys loss | HC K454 | 95.6% | 96.0% | 95.8% | 98.7% | 98.6% | 98.6% | 98.6% | 98.7% | 98.5% | 98.7% | 98.7% | 98.6% | 98.6% |
| N-Glycan HC N304 | A2G0 | 3.1% | 3.0% | 2.8% | 2.3% | 2.3% | 2.2% | 2.2% | 2.2% | 2.3% | 2.3% | 2.3% | 2.3% | 2.3% |
| | A2G0F | 57.0% | 55.5% | 55.2% | 52.2% | 54.3% | 53.0% | 53.3% | 53.1% | 52.9% | 52.7% | 53.8% | 53.3% | 53.6% |
| | A2G1F | 34.8% | 34.1% | 34.6% | 37.8% | 38.1% | 39.1% | 38.7% | 38.1% | 39.1% | 39.4% | 38.4% | 39.1% | 38.8% |
| | A2G2F | 5.2% | 5.1% | 5.2% | 5.7% | 5.3% | 5.7% | 5.9% | 5.4% | 5.6% | 5.6% | 5.4% | 5.3% | 5.4% |
| | M5 | 0.0% | 1.2% | 1.1% | 1.0% | 0.0% | 0.0% | 0.0% | 0.0% | 0.0% | 0.0% | 0.0% | 0.0% | 0.0% |
| | M8 | 0.0% | 1.2% | 1.1% | 1.0% | 0.0% | 0.0% | 0.0% | 1.1% | 0.0% | 0.0% | 0.0% | 0.0% | 0.0% |

Met: Methionine. 5 °C: 5 °C±3°C, 25 °C: 25 °C±2 °C, 60%RH±5%RH, 1000 lx: lay down with light exposure of 1000 lx, Dark: lay down without light exposure by covering with aluminum foil. HC: heavy chain, LC: light chain

**F. Conclusion**

Quality attributes were comparable among formulations of BAN2401 with pH variation from pH 4.5 to pH 5.5. s at pH 4.0 showed lower aggregate formation rate and, on the contrary, faster fragment formation rate during storage. We saw no significant difference between formulations with and without methionine at a concentration of 10 mmol/L. based on evaluations of quality, stability studies including long-term, accelerated, freeze-thaw, and photo. Though slightly slower aggregate formation rate and minor suppression in oxidation of amino acid residue were observed, the differences were not significant. In conclusion, F15 and F21, which were composed of 200 mg/mL of BAN2401, 25 mmol/L. of L-histidine/histidine hydrochloride, 200 mmol/L of L-arginine, 0.05 (w/v)% of polysorbate 80 at a pH of 5.0 ± 0.5 were determined to be good drug substance candidates for further development.

**Example 10: Investigation on Effect of Arginine Concentration to the Stability of BAN2401 Formulation**

The effect of arginine concentration (0, 50, 100, and 200 mmol/L) to the stability of BAN2401 at 200mg/mL was evaluated. The samples of each arginine concentration level were stored at accelerated condition (25 °C/60%RH) and tested at 0, 1, and 2 months.

**A. Sample preparation**

Samples (F1-F4) were prepared by buffer exchange of the concentrated BAN2401 drug substance process intermediate by ultrafiltration. After aseptic filtration, each 0.4 mL sample was filled into vial. The sample information is shown in Table 47.

Table 47 Sample information

| Formulation No. | BAN2401 | Buffer | Stabilizer (Arginine) | Polysorbate 80 | pH |
|---|---|---|---|---|---|
| F1 | 200 mg/mL | Histidine 25 mmol/L | 0 mmol/L | 0.05 (w/v)% | 5.0 |
| F2 | 200 mg/mL | Histidine 25 mmol/L | 50 mmol/L | 0.05 (w/v)% | 5.0 |
| F3 | 200 mg/mL | Histidine 25 mmol/L | 100 mmol/L | 0.05 (w/v)% | 5.0 |
| F4 | 200 mg/mL | Histidine 25 mmol/L | 200 mmol/L | 0.05 (w/v)% | 5.0 |

**B. Results**

Results of this stability study for samples F1 to F4 are shown in Table 48 to 51. There was no difference in pH and protein concentration between samples. and the arginine concentration level were consistent with the target for initial samples.
Aggregates % for samples containing arginine (F2 to F4) were lower than that of the sample without arginine (F1).

**Table 48 Test results of sample F1 (Arginine 0 mmol/L targeted)**

| Test Items | | Initial | 1M | 2M |
|---|---|---|---|---|
| pH | | 5.1 | - | - |
| Protein concentration (mg/mL) | | 195 | - | - |
| SEC | Monomer area% | 98.3% | 98.0% | 97.8% |
| | Aggregates area% | 1.6% | 1.7% | 1.9% |
| | Fragments area% | 0.1% | 0.2% | 0.3% |
| Arginine concentration (mmol/L) | | 1 | - | - |

**Table 49 Test results of sample F2 (Arginine 50 mmol/L targeted)**

| Test Items | | Initial | 1M | 2M |
|---|---|---|---|---|
| pH | | 5.0 | - | - |
| Protein concentration (mg/mL) | | 192 | - | - |
| SEC | Monomer area% | 98.4% | 98.1% | 97.8% |
| | Aggregates area% | 1.5% | 1.7% | 1.8% |
| | Fragments area% | 0.1% | 0.2% | 0.4% |
| Arginine concentration (mmol/L) | | 47 | - | - |

**Table 50 Test results of sample F3 (Arginine 100 mmol/L targeted)**

| Test Items | | Initial | 1M | 2M |
|---|---|---|---|---|
| pH | | 5.0 | - | - |
| Protein concentration (mg/mL) | | 198 | - | - |
| SEC | Monomer area% | 98.4% | 98.1% | 97.8% |
| | Aggregates area% | 1.5% | 1.7% | 1.8% |
| | Fragments area% | 0.1% | 0.2% | 0.4% |
| Arginine concentration (mmol/L) | | 87 | - | - |

**Table 51 Test results of sample F4 (Arginine 200 mmol/L targeted)**

| Test Items | | Initial | 1M | 2M |
|---|---|---|---|---|
| pH | | 5.0 | - | - |
| Protein concentration (mg/mL) | | 191 | - | - |
| SEC | Monomer area% | 98.4% | 98.1% | 97.9% |
| | Aggregates area% | 1.5% | 1.6% | 1.7% |
| | Fragments area% | 0.1% | 0.2% | 0.4% |
| Arginine concentration (mmol/L) | | 173 | - | - |

## C. Discussion and Conclusion

Results of this study indicated that arginine concentrations of 50 to 200 mmol/L prevent increases of protein aggregates in BAN2401 200 mg/mL formulation. Variation of arginine concentration between batches may result in variation in aggregate % between batches. In addition, arginine concentration may affect increase trend of aggregates % in the stability study.

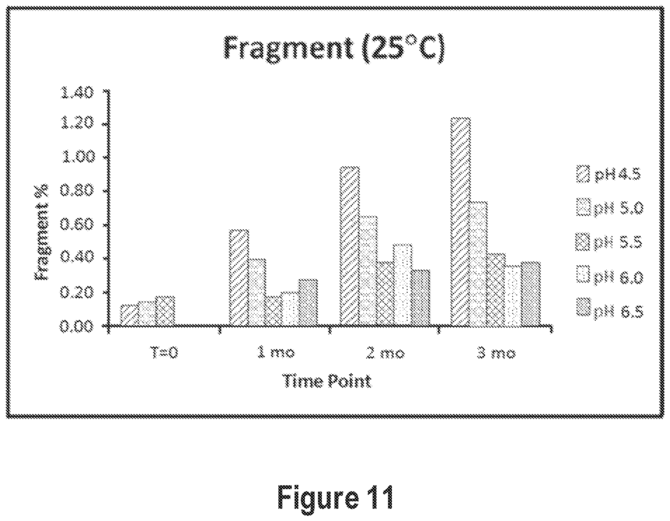

The formulation comprising histidine buffer appeared to show effect on reducing fragmentation of BAN2401 as compared the percentage of fragmentation in the sample F1 with the percentage of fragmentation shown in Figure 11 in Example 2, in which the samples comprising 200 mg/mL BAN2401, 50 mM sodium citrate and 100 mM sodium chloride were used.

## SEQUENCE LISTING

Table 52. Amino acid sequences of mAb variable regions

| mAb | IgG chain | SEQ ID NO | Amino acid sequence |
|---|---|---|---|
| BAN2401 | Heavy chain | 1 | EVQLVESGGGLVQPGGSLRLSCSASGF TFSSFGMHWVRQAPGKGLEWVAYISS GSSTIYYGDTVKGRFTISRDNAKNSLFL QMSSLRAEDTAVYYCAREGGYYYGRS YYTMDYWGQGTTVTVSS |
| BAN2401 | Light chain | 2 | DVVMTQSPLSLPVTPGAPASISCRSSQSI VHSNGNTYLEWYLQKPGQSPKLLIYKV SNRFSGVPDRFSGSGSGTDFTLRISRVE AEDVGIYYCFQGSHVPPTFGPGTKLEIK |

**Table 53. Amino acid sequences of mAb constant regions**

| mAb | IgG chain | Class | SEQ ID NO | Amino acid sequence |
|---|---|---|---|---|
| BAN2401 | Heavy chain | IgG1 | 3 | ASTKGPSVFPLAPSSKSTSGGT AALGCLVKDYFPEPVTVSWN SGALTSGVHTFPAVLQSSGLY SLSSVVTVPSSSLGTQTYICNV NHKPSNTKVDKRVEPKSCDK THTCPPCPAPELLGGPSVFLFP PKPKDTLMISRTPEVTCVVVD VSHEDPEVKFNWYVDGVEVH NAKTKPREEQYNSTYRVVSVL TVLHQDWLNGKEYKCKVSNK ALPAPIEKTISKAKGQPREPQV YTLPPSREEMTKNQVSLTCLV KGFYPSDIAVEWESNGQPENN YKTTPPVLDSDGSFFLYSKLT VDKSRWQQGNVFSCSVMHEA LHNHYTQKSLSLSPGK |
| BAN2401 | Light chain | kappa | 4 | RTVAAPSVFIFPPSDEQLKSGT ASVVCLLNNFYPREAKVQWK VDNALQSGNSQESVTEQDSK DSTYSLSSTLTLSKADYEKHK VYACEVTHQGLSSPVTKSFNR GEC |

**Table 54. Amino acid sequences of mAb CDRs**

| mAb | IgG chain | SEQ ID NO | Amino acid sequence |
|---|---|---|---|
| BAN2401 | HCDR1 | 5 | SFGMH |
| | HCDR2 | 6 | YISSGSSTIYYGDTVKG |
| | HCDR3 | 7 | EGGYYYGRSYYTMDY |
| BAN2401 | LCDR1 | 8 | RSSQSIVHSNGNTYLE |
| | LCDR2 | 9 | KVSNRFS |
| | LCDR3 | 10 | FQGSHVPPT |

**Heavy Chain (SEQ ID NO: 11):**

evqlvesgggglvqpggslrlscsasgftfssfgmhwvrqapgkglewvayissgsstiyygdtvkgrftisrdnaknslflqmsslr aedtavyycareggyyygrsyytmdywgqgttvtvssastkgpsvfplapsskstsggtaalgclvkdyfpepvtvswnsgalts gvhtfpavlqssglyslssvvtvpssslgtqtyicnvnhkpsntkvdkrvepkscdkthtcppcpapellggpsvflfppkpkdtl misrtpevtcvvvdvshedpevkfnwyvdgvevhnaktkpreeqynstyrvvsvltvlhqdwlngkeykckvsnkalpapie ktiskakgqprepqvytlppsreemtknqvsltclvkgfypsdiavewesngqpennykttppvldsdgsfflyskltvdksrwq qgnvfscsvmhealhnhytqkslslspgk

**Light Chain (SEQ ID NO: 12):**

# EP 4 121 007 B1

dvvmtqsplslpvtpgapasiscrssqsivhsngntylewylqkpgqspklliykvsnrfsgvpdrfsgsgsgtdftlrisrveaedv
giyycfqgshvpptfgpgtkleikrtvaapsvfifppsdeqlksgtasvvcllnnfypreakvqwkvdnalqsgnsqesvteqdsk
dstyslsstltlskadyekhkvyacevthqglsspvtksfnrgec

## Claims

1. An aqueous pharmaceutical formulation comprising:

   (a) an isolated anti-Aβ protofibril antibody that binds to human Aβ protofibrils, at a concentration of 80 mg/mL to 300 mg/mL,
   (b) 100 mM to 400 mM arginine,
   (c) 0.01% w/v to 0.1% w/v polysorbate 80, and
   (d) a pharmaceutically acceptable buffer,
   wherein the pharmaceutical formulation has a pH ranging from 4.5 to 5.5,
   wherein the isolated anti-Aβ protofibril antibody is lecanemab, and
   wherein the arginine is arginine, arginine hydrochloride, or a combination thereof.

2. The pharmaceutical formulation according to claim 1, wherein the isolated anti-Aβ protofibril antibody is present in a concentration of 100 mg/mL to 200 mg/mL.

3. The pharmaceutical formulation according to claim 1, wherein the isolated anti-Aβ protofibril antibody is present in a concentration of 100 mg/mL.

4. The pharmaceutical formulation according to claim 1, wherein the isolated anti-Aβ protofibril antibody is present in a concentration of 200 mg/mL.

5. The pharmaceutical formulation according to any one of claims 1-4, further comprising methionine.

6. The pharmaceutical formulation according to any one of claims 1-5, wherein the pharmaceutically acceptable buffer is citrate buffer or histidine buffer.

7. The pharmaceutical formulation according to any one of claims 1-6, comprising 10 to 100 mM citrate buffer or 10 to 100 mM histidine buffer.

8. The pharmaceutical formulation according to any one of claims 1-7, comprising 125 to 350 mM arginine.

9. The pharmaceutical formulation according to any one of claims 1-8, comprising 200 mM arginine, wherein the arginine is arginine hydrochloride.

10. The pharmaceutical formulation according to any one of claims 1-9, comprising 200 mM arginine, wherein the arginine is arginine hydrochloride, and 25 mM histidine buffer.

11. The aqueous pharmaceutical formulation according to claim 1 comprising:

    (a) 80 mg/mL to 240 mg/mL of the isolated anti-Aβ protofibril antibody ,
    (b) 140 mM to 260 mM arginine hydrochloride,
    (c) 0.02% w/v to 0.08% w/v polysorbate 80, and
    (d) 15 mM to 35 mM histidine buffer,

    wherein the pharmaceutical formulation has a pH ranging from 4.5 to 5.5.

12. The aqueous pharmaceutical formulation according to claim 1 comprising:

    (a) 80 mg/mL to 120 mg/mL of the isolated anti-Aβ protofibril antibody ,
    (b) 240 mM to 360 mM arginine,
    (c) 0.02% w/v to 0.08% w/v polysorbate 80, and

60

(d) 30 mM to 50 mM citrate buffer,
wherein the pharmaceutical formulation has a pH ranging from 4.5 to 5.5,
and wherein the arginine is arginine, arginine hydrochloride, or a combination thereof.

**13.** The aqueous pharmaceutical formulation according to claim 1, comprising:

(a) 100 mg/mL or 200 mg/mL of the isolated anti-Aβ protofibril antibody,
(b) 190 mM to 210 mM of arginine, arginine hydrochloride, or a combination thereof,
(c) 0.04% w/v to 0.06% w/v polysorbate 80, and
(d) 15 mM to 35 mM histidine buffer.

**14.** The aqueous pharmaceutical formulation according to claim 1, comprising:

(a) 100 mg/mL of the isolated anti-Aβ protofibril antibody,
(b) 200 mM arginine hydrochloride,
(c) 0.05% w/v polysorbate 80, and
(d) 25 mM histidine buffer.

**15.** The aqueous pharmaceutical formulation according to claim 1, comprising:

(a) 200 mg/mL of the isolated anti-Aβ protofibril antibody,
(b) 200 mM arginine hydrochloride,
(c) 0.05% w/v polysorbate 80, and
(d) 25 mM histidine buffer.

**16.** The aqueous pharmaceutical formulation according to any one of claims 13-15, wherein the histidine buffer is a combination of histidine and histidine hydrochloride monohydrate.

**17.** The aqueous pharmaceutical formulation according to any one of claims 13-16, wherein the pharmaceutical formulation has a pH of 5.0.

**Patentansprüche**

**1.** Wässrige pharmazeutische Formulierung, umfassend:

(a) einen isolierten Anti-Aβ-Protofibrillen-Antikörper, der an menschliche Aß-Protofibrillen bindet, in einer Konzentration von 80 mg/mL bis 300 mg/mL,
(b) 100 mM bis 400 mM Arginin,
(c) 0,01% w/v bis 0,1% w/v Polysorbat 80, und
(d) einen pharmazeutisch akzeptablen Puffer,
wobei die pharmazeutische Formulierung einen pH-Wert im Bereich von 4,5 bis 5,5 aufweist,
wobei der isolierte Anti-Aβ-Protofibrillen-Antikörper Lecanemab ist, und
wobei das Arginin Arginin, Argininhydrochlorid oder eine Kombination davon ist.

**2.** Pharmazeutische Formulierung nach Anspruch 1, wobei der isolierte Anti-Aβ-Protofibrillen-Antikörper in einer Konzentration von 100 mg/mL bis 200 mg/mL vorliegt.

**3.** Pharmazeutische Formulierung nach Anspruch 1, wobei der isolierte Anti-Aβ-Protofibrillen-Antikörper in einer Konzentration von 100 mg/mL vorliegt.

**4.** Pharmazeutische Formulierung nach Anspruch 1, wobei der isolierte Anti-Aβ-Protofibrillen-Antikörper in einer Konzentration von 200 mg/mL vorliegt.

**5.** Pharmazeutische Formulierung nach irgendeinem der Ansprüche 1-4, ferner umfassend Methionin.

**6.** Pharmazeutische Formulierung nach irgendeinem der Ansprüche 1-5, wobei der pharmazeutisch akzeptable Puffer Citratpuffer oder Histidinpuffer ist.

7. Pharmazeutische Formulierung nach irgendeinem der Ansprüche 1-6, ferner umfassend 10 bis 100 mM Citratpuffer oder 10 bis 100 mM Histidinpuffer.

8. Pharmazeutische Formulierung nach irgendeinem der Ansprüche 1-7, umfassend 125 bis 350 mM Arginin.

9. Pharmazeutische Formulierung nach irgendeinem der Ansprüche 1-8, umfassend 200 mM Arginin, wobei das Arginin Argininhydrochlorid ist.

10. Pharmazeutische Formulierung nach irgendeinem der Ansprüche 1-9, umfassend 200 mM Arginin, wobei das Arginin Argininhydrochlorid ist, und 25 mM Histidinbuffer.

11. Wässrige pharmazeutische Formulierung nach Anspruch 1, umfassend:

   (a) 80 mg/mL bis 240 mg/mL des isolierten Anti-Aß-Protofibrillen-Antikörpers,
   (b) 140 mM bis 260 mM Argininhydrochlorid,
   (c) 0,02% w/v bis 0,08% w/v Polysorbat 80, und
   (d) 15 mM bis 35 mM Histidinpuffer,

   wobei die pharmazeutische Formulierung einen pH-Wert im Bereich von 4,5 bis 5,5 aufweist.

12. Wässrige pharmazeutische Formulierung nach Anspruch 1, umfassend:

   (a) 80 mg/mL bis 120 mg/mL des isolierten Anti-Aß-Protofibrillen-Antikörpers,
   (b) 240 mM bis 360 mM Arginin,
   (c) 0,02% w/v bis 0,08% w/v Polysorbat 80, und
   (d) 30 mM bis 50 mM Citratpuffer,

   wobei die pharmazeutische Formulierung einen pH-Wert im Bereich von 4,5 bis 5,5 aufweist und wobei das Arginin Arginin, Argininhydrochlorid oder eine Kombination davon ist.

13. Wässrige pharmazeutische Formulierung nach Anspruch 1, umfassend:

   (a) 100 mg/mL oder 200 mg/mL des isolierten Anti-Aß-Protofibrillen-Antikörpers,
   (b) 190 mM bis 210 mM Arginin, Argininhydrochlorid oder einer Kombination davon,
   (c) 0,04% w/v bis 0,06% w/v Polysorbat 80, und
   (d) 15 mM bis 35 mM Histidinpuffer.

14. Wässrige pharmazeutische Formulierung nach Anspruch 1, umfassend:

   (a) 100 mg/mL des isolierten Anti-A$\beta$-Protofibrillen-Antikörpers,
   (b) 200 mM Argininhydrochlorid,
   (c) 0,05% w/v Polysorbat 80, und
   (d) 25 mM Histidinpuffer.

15. Wässrige pharmazeutische Formulierung nach Anspruch 1, umfassend:

   (a) 200 mg/mL des isolierten Anti-A$\beta$-Protofibrillen-Antikörpers,
   (b) 200 mM Argininhydrochlorid,
   (c) 0,05% w/v Polysorbat 80, und
   (d) 25 mM Histidinpuffer.

16. Wässrige pharmazeutische Formulierung nach irgendeinem der Ansprüche 13-15, wobei der Histidinpuffer eine Kombination aus Histidin und Histidinhydrochlorid-Monohydrat ist.

17. Wässrige pharmazeutische Formulierung nach irgendeinem der Ansprüche 13-16, wobei die pharmazeutische Formulierung einen pH-Wert von 5.0 hat.

**Revendications**

1. Formulation pharmaceutique aqueuse comprenant :

(a) un anticorps anti-protofibrile Aβ isolé qui se lie aux protofibriles Aβ humaines, à une teneur comprise entre 80 mg/mL et 300 mg/mL,
(b) 100 mM à 400 mM d'arginine,
(c) 0,01% p/v à 0,1% p/v de polysorbate 80, et
(d) un tampon pharmaceutiquement acceptable,
dans lequel la formulation pharmaceutique a un pH allant de 4,5 à 5,5,
dans lequel l'anticorps anti-protofibrile Aβ isolé est le lecanemab, et
dans lequel l'arginine est l'arginine, le chlorhydrate d'arginine ou une combinaison de ceux-ci.

2. Formulation pharmaceutique selon la revendication 1, dans laquelle l'anticorps anti-protofibrile Aβ isolé est présent à une teneur comprise entre 100 mg/mL et 200 mg/mL.

3. Formulation pharmaceutique selon la revendication 1, dans laquelle l'anticorps anti-protofibrile Aβ isolé est présent à une teneur de 100 mg/mL.

4. Formulation pharmaceutique selon la revendication 1, dans laquelle l'anticorps anti-protofibrile Aβ isolé est présent à une teneur de 200 mg/mL.

5. Formulation pharmaceutique selon l'une quelconque des revendications 1 à 4, comprenant en outre la méthionine.

6. Formulation pharmaceutique selon l'une quelconque des revendications 1 à 5, dans laquelle le tampon pharmaceutiquement acceptable est un tampon de citrate ou un tampon d'histidine.

7. Formulation pharmaceutique selon l'une quelconque des revendications 1 à 6, comprenant de 10 à 100 mM de tampon de citrate ou de 10 à 100 mM de tampon d'histidine.

8. Formulation pharmaceutique selon l'une quelconque des revendications 1 à 7, comprenant de 125 à 350 mM d'arginine.

9. Formulation pharmaceutique selon l'une quelconque des revendications 1 à 8, comprenant 200 mM d'arginine, dans laquelle l'arginine est du chlorhydrate d'arginine.

10. Formulation pharmaceutique selon l'une quelconque des revendications 1 à 9, comprenant 200 mM d'arginine, dans laquelle l'arginine est du chlorhydrate d'arginine et 25 mM d'un tampon d'histidine.

11. Formulation pharmaceutique aqueuse selon la revendication 1 comprenant :

(a) de 80 mg/mL à 240 mg/mL de l'anticorps anti-protofibrile Aβ isolé,
(b) de 140 mM à 260 mM de chlorhydrate d'arginine,
(c) de 0,02% p/v à 0.08% p/v de polysorbate 80, et
(d) de 15 mM à 35 mM de tampon d'histidine,

dans laquelle la formulation pharmaceutique a un pH compris entre 4,5 et 5,5.

12. Formulation pharmaceutique aqueuse selon la revendication 1 comprenant :

(a) de 80 mg/mL à 120 mg/mL de l'anticorps anti-protofibrile Aβ isolé,
(b) de 240 mM à 360 mM d'arginine,
(c) de 0,02% p/v à 0.08% p/v de polysorbate 80, et
(d) de 30 mM à 50 mM de tampon de citrate,
dans lequel la formulation pharmaceutique a un pH allant de 4,5 à 5,5,
et dans lequel l'arginine est l'arginine, le chlorhydrate d'arginine ou une combinaison de ceux-ci.

13. Formulation pharmaceutique aqueuse selon la revendication 1, comprenant :

(a) 100 mg/mL ou 200 mg/mL de l'anticorps anti-protofibrile Aβ isolé,
(b) de 190 mM à 210 mM d'arginine, de chlorhydrate d'arginine ou d'une combinaison de ceux-ci,
(c) 0,04% p/v à 0,06% p/v de polysorbate 80, et
(d) de 15 mM à 35 mM de tampon d'histidine.

14. Formulation pharmaceutique aqueuse selon la revendication 1, comprenant :

(a) 100 mg/mL de l'anticorps anti-protofibrile Aβ isolé,
(b) 200 mM de chlorhydrate d'arginine,
(c) 0,05% p/v de polysorbate 80, et
(d) 25 mM de tampon d'histidine.

15. Formulation pharmaceutique aqueuse selon la revendication 1, comprenant :

(a) 200 mg/mL de l'anticorps anti-protofibrile Aβ isolé,
(b) 200 mM de chlorhydrate d'arginine,
(c) 0,05% p/v de polysorbate 80, et
(d) 25 mM de tampon d'histidine.

16. Formulation pharmaceutique aqueuse selon l'une quelconque des revendications 13 à 15, dans laquelle le tampon d'histidine est une combinaison d'histidine et de chlorhydrate d'histidine monohydratée.

17. Formulation pharmaceutique aqueuse selon l'une quelconque des revendications 13 à 16, dans laquelle la formulation pharmaceutique a un pH de 5,0.

Figure 1

Figure 2

Figure 3

Figure 4

Figure 5

Figure 6

Figure 7

Figure 8

Figure 9

Figure 10

Figure 11

Figure 12

Figure 13

Figure 14

EP 4 121 007 B1

Figure 15

79

Figure 16

Figure 17

| Ingredient | Process | In-process Control |
|---|---|---|
| BAN2401 drug substance (Formulation B) | Bioburden reducing filtration (0.2 μm) | Bioburden pre- and post-filtration |
| | Sterile filtration (0.2 μm) | 0.2-μm filter integrity testing pre- and post-filtration |
| 5-mL glass vials 13-mm stoppers 13-mm aluminum seals | Aseptic filling and stoppering | Fill weight checks |
| | Crimp capping | |
| | Visual inspection | |

# Figure 18

Figure 19

Figure 20

Figure 21

Figure 22

**Figure 23**

Figure 24

Figure 25

**Figure 26**

Figure 27

**Figure 28**

Figure 29

Figure 30

Figure 31

Figure 32

Figure 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62992746
- US 63027263
- WO 2007108756 A
- WO 2002003911 A
- WO 2005123775 A
- WO 2011001366 A
- WO 2011104696 A
- WO 2016005466 A
- WO 2020023530 A
- US 62749614
- US 2019043067 W

**Non-patent literature cited in the description**

- **BROOKMEYER, R. et al.** Forecasting the global burden of Alzheimer's Disease. *Alzheimer Dement.*, 2007, vol. 3, 186-91
- *Alzheimer Dement.*, 2010, vol. 6, 158-94
- **HEBERT, L.E. et al.** Alzheimer disease in the U.S. population: prevalence estimates using the 2000 census. *Arch Neurol.*, 2003, vol. 60, 1119-1122
- **KABAT et al.** SEQUENCES OF PROTEINS OF IMMUNOLOGICAL INTEREST. U.S. Department of Health and Human Services, 1991
- *Journal of Alzheimer's Disease*, 2015, vol. 43, 575-588
- *J. Chromatography B*, 2010, vol. 878, 1865-1870